(19) Europäisches Patentamt
European Patent Office
Office européen des brevets

(11) EP 2 017 281 A1

(12) # EUROPEAN PATENT APPLICATION
published in accordance with Art. 153(4) EPC

(43) Date of publication:
**21.01.2009 Bulletin 2009/04**

(21) Application number: **07741584.2**

(22) Date of filing: **13.04.2007**

(51) Int Cl.:
***C07H 19/20*** (2006.01) ***A61K 31/7064*** (2006.01)
***A61P 31/00*** (2006.01) ***A61P 35/00*** (2006.01)
***A61P 37/04*** (2006.01) ***A61P 37/08*** (2006.01)
***A61P 43/00*** (2006.01) ***C07H 19/10*** (2006.01)
***C12N 1/20*** (2006.01) ***C12P 19/30*** (2006.01)

(86) International application number:
**PCT/JP2007/058148**

(87) International publication number:
**WO 2007/119815 (25.10.2007 Gazette 2007/43)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **14.04.2006 JP 2006111950**

(71) Applicant: **Kyowa Hakko Kirin Co., Ltd.**
**Tokyo 100-8185 (JP)**

(72) Inventors:
- **NAGATA, Hiroyuki**
**Asahi-machi 3-chome,Machida-shi, Tokyo 194-8533 (JP)**
- **ICHIMURA, Michio**
**Asahi-machi 3-chome,Machida-shi, Tokyo 194-8533 (JP)**
- **NAKATSU, Rieko**
**Asahi-machi 3-chome,Machida-shi, Tokyo 194-8533 (JP)**
- **IKEDA, Shun-ichi**
**TOKYO (JP)**
- **KAWABATA, Ayako**
**Asahi-machi 3-chome,Machida-shi, Tokyo 194-8533 (JP)**
- **OTA, Toshio**
**Nagaizumi-cho, Sunto-gun, SHIZUOKA 411-8731 (JP)**
- **ABE, Masayuki**
**Nagaizumi-cho, Sunto-gun SHIZUOKA 411-8731 (JP)**
- **TAKASHIMA, Michio**
**Nagaizumi-cho, Sunto-gun SHIZUOKA 411-8731 (JP)**
- **SUZUKI, Makoto**
**1-6-1 Ohtemachi, Chiyoda-ku TOKYO 100-8185 (JP)**

(74) Representative: **Vossius & Partner**
**Siebertstrasse 4**
**81675 München (DE)**

(54) **TOLL-LIKE RECEPTOR 9 AGONISTS**

(57) The present invention provides TLR9 agonists comprising, as an active ingredient, a compound represented by formula (I):

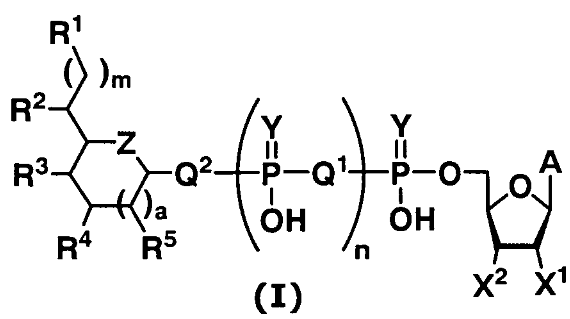

(I)



Printed by Jouve, 75001 PARIS (FR)

(Cont. next page)

(wherein a represents 0 or 1; n represents an integer of 0 to 2; m represents an integer of 0 to 5; $X^1$ and $X^2$ each independently represent a hydrogen atom or hydroxy; Y represents an oxygen atom or a sulfur atom; $-Q^1$-represents -O- or the like; $-Q^2$- represents -O- or the like; -Z- represents -O- or the like; $R^1$, $R^3$ and $R^4$ each independently represent hydroxy or the like; $R^2$ and $R^5$ each independently represent a hydrogen atom, hydroxy or the like; and A represents 6-aminopurin-9-yl or the like) or a pharmaceutically acceptable salt thereof, and the like.

## Description

### Technical Field

The present invention relates to immunostimulants, Toll-like receptor 9 (TLR9) agonists, and the like.

### Background Art

It has been revealed that CpG-oligodeoxynucleotides are recognized by Toll-like receptor 9 (TLR9), which is one of the Toll-like receptors [e.g., Nature, Vol. 408, p. 740 (2000)]. TLR9 is present in B cells, dendritic cells, etc. and is known to be activated by oligonucleotides having unmethylated CpG motifs which are present in DNA of bacteria and viruses. That is, TLR9 transmits signals into cells via adapter molecules such as MyD88, activates transcription factors such as NF-κB controlling expression of cytokine genes, and induces production of cytokines. Further, TLR9 enhances expression of surface molecules such as CD40, CD80 and CD86, which are co-stimulatory molecules important for the activation of lymphocytes.

It is also known that TLR9 agonists such as CpG-oligodeoxynucleotides have immunostimulatory activity and show anti-allergic effect, anti-tumor effect and anti-infection effect [e.g., EP0468520; WO96/02555; WO98/18810; WO98/37919; WO98/40100; WO99/51259; WO99/56755; Nature Reviews Drug Discovery, Vol. 1, p. 797 (2002); Nature Reviews Immunology, Vol. 4, p. 1 (2004); Clinical Cancer Research, Vol. 9, p. 2693 (2003); Clinical Cancer Research, Vol. 9, p. 3105 (2003); The Journal of Immunology, Vol. 160, p. 3627 (1998); Proceedings of the National Academy of Sciences of the United States of America, Vol. 96, p. 6970 (1999); Journal of Allergy & Clinical Immunology, Vol. 110, p. 867 (2002); American Journal of Respiratory and Critical Care Medicine, Vol. 170, p. 1153 (2004); American Society of Clinical Oncology: ASCO, Annual Meeting, Abstract, #7039 (2005); Journal of Allergy & Clinical Immunology, Vol. 113, p. 235 (2004); Nature, Vol. 408, p. 740 (2000); Blood, Vol. 89, p. 2635 (1997); Journal of Experimental Medicine, Vol. 184, p. 981 (1996); Leukemia Lymphoma, Vol. 19, p. 267 (1995); and Journal of Experimental Medicine, Vol. 178, p. 1057 (1993)].

As mononucleotide=5'-diphosphates, adenosine=5'-(L-glycero-β-D-mannopyranosyl)diphosphate, adenosine=5'-(D-glycero-β-D-mannoheptopyranosyl)diphosphate, etc. are known as intermediates in the biosynthesis of lipopolysaccharides (LPS) which are Gram-negative bacterial cell wall components (see non-patent document No. 1), and Compounds 18 to 23 described in the table given hereinbelow, etc. are commercially available. Also the known are methods for producing Compounds 9 to 17 described in the table given hereinbelow, etc. (see non-patent document No. 2).

Non-patent document No. 1:
Journal of Bacteriology, Vol. 184, p. 363 (2002)
Non-patent document No. 2:
Carbohydrate Research, Vol. 338, p. 2571 (2003)

### Disclosure of the Invention

### Problems to be Solved by the Invention

An object of the present invention is to provide compounds having TLR9 agonist activity which are useful, for example, as TLR9 agonists, immunostimulants, anti-allergic agents, anti-tumor agents and anti-infection agents, and the like.

### Means for Solving the Problems

The present invention relates to the following (1) to (49).

(1) A TLR9 agonist comprising, as an active ingredient,
a compound represented by formula (I):

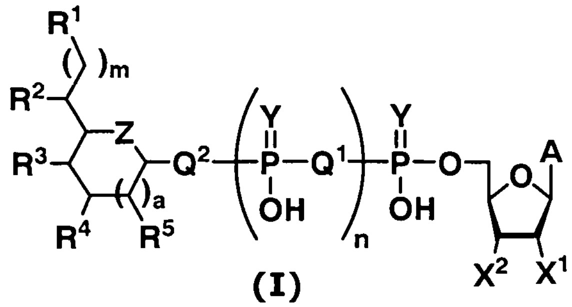

(I)

(wherein a represents 0 or 1;
n represents an integer of 0 to 2;
m represents an integer of 0 to 5;
$X^1$ and $X^2$ each independently represents a hydrogen atom or hydroxy;
Y represents an oxygen atom or a sulfur atom (when n is 1 or 2, two or three Ys may be the same or different);
$-Q^1-$ represents -O-, $-CH_2-$ or $-CF_2-$;
$-Q^2-$ represents a single bond, -O-, $-CH_2-O-$ or $-CH_2-$;
-Z- represents -O- or $-CH_2-$;
$R^1$, $R^3$ and $R^4$ each independently represents hydroxy, substituted or unsubstituted lower alkanoyloxy, substituted or unsubstituted lower alkoxy, substituted or unsubstituted lower alkenyloxy, substituted or unsubstituted aralkyloxy, or substituted or unsubstituted heterocyclic alkyloxy;
$R^2$ and $R^5$ each independently represents a hydrogen atom, hydroxy, substituted or unsubstituted lower alkanoyloxy, substituted or unsubstituted lower alkoxy, substituted or unsubstituted lower alkenyloxy, substituted or unsubstituted aralkyloxy, or substituted or unsubstituted heterocyclic alkyloxy; and
A represents a group selected from the following substituents (A)) or a pharmaceutically acceptable salt thereof

Substituents (A)

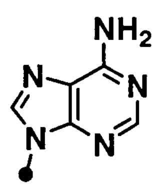
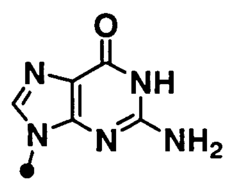

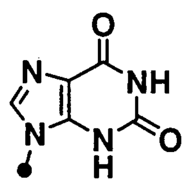
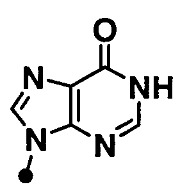

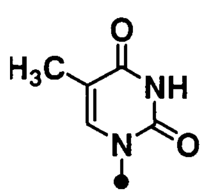
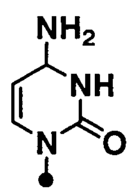
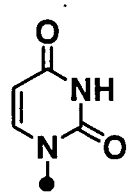

(2) The TLR9 agonist according to the above (1), wherein m is 1.
(3) The TLR9 agonist according to the above (1) or (2), wherein a is 1.
(4) The TLR9 agonist according to the above (1) or (2), wherein a is 0.
(5) The TLR9 agonist according to any of the above (1) to (4), wherein $R^2$ is a hydrogen atom.
(6) The TLR9 agonist according to any of the above (1) to (4), wherein $R^1$ and $R^2$ each independently represents

hydroxy or lower alkanoyloxy.

(7) The TLR9 agonist according to any of the above (1) to (6), wherein $X^1$ and $X^2$ are hydroxy.

(8) A compound rpresented by formula (IA):

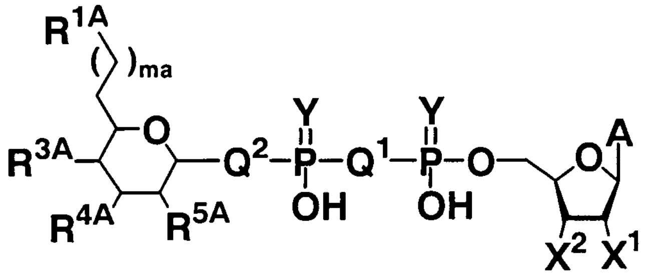

(IA)

(wherein ma represents an integer of 1 to 5;

$X^1$, $X^2$, Y, $Q^1$, $Q^2$ and A each have the same meanings as defined above;

$R^{1A}$, $R^{3A}$, $R^{4A}$ and $R^{5A}$ each independently represents hydroxy, substituted or unsubstituted lower alkanoyloxy, or substituted or unsubstituted lower alkoxy) or a pharmaceutically acceptable salt thereof.

(9) The compound or the pharmaceutically acceptable salt thereof according to the above (8), wherein ma is 1.

(10) The compound or the pharmaceutically acceptable salt thereof according to the above (8) or (9), wherein $X^1$ and $X^2$ are hydroxy.

(11) The compound or the pharmaceutically acceptable salt thereof according to any of the above (8) to (10), wherein $R^{1A}$ is hydroxy or lower alkanoyloxy.

(12) The compound or the pharmaceutically acceptable salt thereof according to any of the above (8) to (11), wherein A is

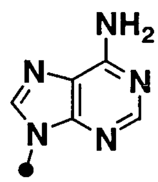

.

(13) A TLR9 agonist comprising, as an active ingredient, the compound or the pharmaceutically acceptable salt thereof described in any of the above (8) to (12).

(14) An anti-allergic agent comprising, as an active ingredient, the compound or the pharmaceutically acceptable salt thereof described in any of the above (8) to (12).

(15) An anti-tumor agent comprising, as an active ingredient, the compound or the pharmaceutically acceptable salt thereof described in any of the above (8) to (12).

(16) An anti-infection agent comprising, as an active ingredient, the compound or the pharmaceutically acceptable salt thereof described in any of the above (8) to (12).

(17) An immunostimulant comprising, as an active ingredient, the compound or the pharmaceutically acceptable salt thereof described in any of the above (8) to (12).

(18) A treating and/or preventing agent for a TLR9-associated disease comprising, as an active ingredient, the compound or the pharmaceutically acceptable salt thereof described in any of the above (8) to (12).

(19) An anti-allergic agent comprising, as an active ingredient, the compound or the pharmaceutically acceptable salt thereof described in any of the above (1) to (7).

(20) An anti-tumor agent comprising, as an active ingredient, the compound or the pharmaceutically acceptable salt thereof described in any of the above (1) to (7).

(21) An anti-infection agent comprising, as an active ingredient, the compound or the pharmaceutically acceptable salt thereof described in any of the above (1) to (7).

(22) An immunostimulant comprising, as an active ingredient, the compound or the pharmaceutically acceptable salt thereof described in any of the above (1) to (7).

(23) A treating and/or preventing agent for a TLR9-associated disease comprising, as an active ingredient, the compound or the pharmaceutically acceptable salt thereof described in any of the above (1) to (7).

(24) Massilia sp. KY 13101 strain (accession no.: NITE BP-348).
(25) A process for producing the compound described in any of the above (1) to (12) utilizing the microorganism described in the above (24).
(26) A method for activating TLR9 which comprises a step of administering an effective amount of the compound or the pharmaceutically acceptable salt thereof described in any of the above (1) to (7).
(27) A method for treating allergy which comprises a step of administering an effective amount of the compound or the pharmaceutically acceptable salt thereof described in any of the above (1) to (7).
(28) A method for treating tumor which comprises a step of administering an effective amount of the compound or the pharmaceutically acceptable salt thereof described in any of the above (1) to (7).
(29) A method for treating an infective disease which comprises a step of administering an effective amount of the compound or the pharmaceutically acceptable salt thereof described in any of the above (1) to (7).
(30) An immunostimulatory method which comprises a step of administering an effective amount of the compound or the pharmaceutically acceptable salt thereof described in any of the above (1) to (7).
(31) A method for treating and/or preventing a TLR9-associated disease which comprises a step of administering an effective amount of the compound or the pharmaceutically acceptable salt thereof described in any of the above (1) to (7).
(32) A method for activating TLR9 which comprises a step of administering an effective amount of the compound or the pharmaceutically acceptable salt thereof described in any of the above (8) to (12).
(33) A method for treating allergy which comprises a step of administering an effective amount of the compound or the pharmaceutically acceptable salt thereof described in any of the above (8) to (12).
(34) A method for treating tumor which comprises a step of administering an effective amount of the compound or the pharmaceutically acceptable salt thereof described in any of the above (8) to (12).
(35) A method for treating an infective disease which comprises a step of administering an effective amount of the compound or the pharmaceutically acceptable salt thereof described in any of the above (8) to (12).
(36) An immunostimulatory method which comprises a step of administering an effective amount of the compound or the pharmaceutically acceptable salt thereof described in any of the above (8) to (12).
(37) A method for treating and/or preventing a TLR9-associated disease which comprises a step of administering an effective amount of the compound or the pharmaceutically acceptable salt thereof described in any of the above (8) to (12).
(38) Use of the compound or the pharmaceutically acceptable salt thereof described in any of the above (1) to (7) for the manufacture of a TLR9 agonist.
(39) Use of the compound or the pharmaceutically acceptable salt thereof described in any of the above (1) to (7) for the manufacture of an anti-allergic agent.
(40) Use of the compound or the pharmaceutically acceptable salt thereof described in any of the above (1) to (7) for the manufacture of an anti-tumor agent.
(41) Use of the compound or the pharmaceutically acceptable salt thereof described in any of the above (1) to (7) for the manufacture of an anti-infection agent.
(42) Use of the compound or the pharmaceutically acceptable salt thereof described in any of the above (1) to (7) for the manufacture of an immunostimulant.
(43) Use of the compound or the pharmaceutically acceptable salt thereof described in any of the above (1) to (7) for the manufacture of a treating and/or preventing agent for a TLR9-associated disease.
(44) Use of the compound or the pharmaceutically acceptable salt thereof described in any of the above (8) to (12) for the manufacture of a TLR9 agonist.
(45) Use of the compound or the pharmaceutically acceptable salt thereof described in any of the above (8) to (12) for the manufacture of an anti-allergic agent.
(46) Use of the compound or the pharmaceutically acceptable salt thereof described in any of the above (8) to (12) for the manufacture of an anti-tumor agent.
(47) Use of the compound or the pharmaceutically acceptable salt thereof described in any of the above (8) to (12) for the manufacture of an anti-infection agent.
(48) Use of the compound or the pharmaceutically acceptable salt thereof described in any of the above (8) to (12) for the manufacture of an immunostimulant.
(49) Use of the compound or the pharmaceutically acceptable salt thereof described in any of the above (8) to (12) for the manufacture of a treating and/or preventing agent for a TLR9-associated disease.

## Effect of the Invention

The present invention provides compounds having TLR9 agonist activity which are useful, for example, as TLR9 agonists, immunostimulants, anti-allergic agents, anti-tumor agents and anti-infection agents, and the like.

Brief Description of the Drawings

[Fig. 1]
Fig. 1 is a figure illustrating the result of a phylogenetic analysis using a 16S rDNA partial sequence of the Gram-negative bacillus employed for the production of a compound used in the present invention.
[Fig. 2]
Fig. 2 shows the activating effect of representative Compounds (I) on NF-κB. The results are shown in terms of luciferase activity (RLU) at each concentration of each compound. The ordinate indicates RLU and the abscissa indicates the concentration of test compounds (mol/L). -filled square-: Compound 1, -open square-: Compound 2, -open rhombus-: Compound 10, -X-: Compound 12, -+-: Compound 14, -open triangle-: Compound 16, -filled circle-: Compound 23.
[Fig. 3]
Fig. 3 shows the activating effect of Compound 30 on NF-κB. For comparison, the result on Compound 16 is also shown. The results are shown in terms of luciferase activity (RLU) at each concentration of each compound.
The ordinate indicates RLU and the abscissa indicates the concentration of test compounds (mol/L).
-open triangle-: Compound 16, -filled reverse triangle-: Compound 30.

Best Modes for Carrying Out the Invention

Hereinafter, the compounds represented by formula (I) are referred to as Compounds (I). This applies to compounds of other formula numbers.

In the definitions of the groups in formula (I) and formula (IA): the lower alkyl moiety of the lower alkoxy includes straight-chain or branched alkyl groups having 1 to 8 carbon atoms, such as methyl, ethyl, propyl, isopropyl, butyl, sec-butyl, tert-butyl, pentyl, isopentyl, neopentyl, hexyl, heptyl and octyl.

The lower alkenyl moiety of the lower alkenyloxy includes straight-chain or branched alkenyl groups having 2 to 8 carbon atoms, such as vinyl, allyl, 1-propenyl, methacryl, crotyl, 1-butenyl, 3-butenyl, 2-pentenyl, 4-pentenyl, 2-hexenyl, 5-hexenyl, 2-heptenyl and 2-octenyl.

The lower alkanoyl moiety of the lower alkanoyloxy includes straight-chain or branched alkanoyl groups having 1 to 7 carbon atoms, such as formyl, acetyl, propionyl, butyryl, isobutyryl, valeryl, isovaleryl, pivaloyl, hexanoyl and heptanoyl.

The alkylene moiety of the aralkyloxy and the heterocyclic alkyloxy includes groups in which one hydrogen atom is removed from the above lower alkyl.

The aryl moiety of the aralkyloxy includes monocyclic, bicyclic or tricyclic aryl groups having 6 to 14 carbon atoms, such as phenyl, indenyl, naphthyl and anthryl.

The heterocyclic group moiety of the heterocyclic alkyloxy includes aromatic heterocyclic groups and alicyclic heterocyclic groups. Examples of the aromatic heterocyclic groups include 5- or 6-membered monocyclic aromatic heterocyclic groups containing at least one atom selected from a nitrogen atom, an oxygen atom and a sulfur atom, and bicyclic or tricyclic fused aromatic heterocyclic groups containing at least one atom selected from a nitrogen atom, an oxygen atom and a sulfur atom in which 3- to 8-membered rings are condensed, such as pyridyl, pyrazinyl, pyrimidinyl, pyridazinyl, quinolyl, isoquinolyl, phthalazinyl, quinazolyl, quinoxalyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzopyranyl, benzothienyl, benzofuranyl, benzimidazolyl, benzotriazolyl, benzothiazolyl, benzopyrazolyl, benzodioxanyl, benzoxazolyl, purinyl and benzodioxolanyl. Examples of the alicyclic heterocyclic groups include 5- or 6-membered monocyclic alicyclic heterocyclic groups containing at least one atom selected from a nitrogen atom, an oxygen atom and a sulfur atom, and bicyclic or tricyclic fused alicyclic heterocyclic groups containing at least one atom selected from a nitrogen atom, an oxygen atom and a sulfur atom in which 3- to 8-membered rings are condensed, such as pyrrolidinyl, piperidino, piperazinyl, morpholino, morpholinyl, thiomorpholino, thiomorpholinyl, homopiperidino, homopiperazinyl, tetrahydropyridinyl, tetrahydroquinolinyl, tetrahydroisoquinolinyl, tetrahydrofuranyl, tetrahydropyranyl, dihydrobenzofuranyl, oxopiperazinyl and 2-oxopyrrolidinyl.

The substituted lower alkanoyloxy, the substituted lower alkoxy and the substituted lower alkenyloxy each have one to a substitutable number, preferably 1 to 5, more preferably 1 to 3 substituents (a) which are the same or different. Examples of substituents (a) include hydroxy, halogen, substituted or unsubstituted lower alkoxy, lower alkanoyl, lower alkanoyloxy, carboxy, lower, alkoxycarbonyl, carbamoyl, mono-lower alkylaminocarbonyl, di-lower alkylaminocarbonyl, cyano, amino, mono-lower alkylamino and di-lower alkylamino.

The substituted aralkyloxy and the substituted heterocyclic alkyloxy each have one to a substitutable number, preferably 1 to 5, more preferably 1 to 3 substituents (b) which are the same or different. Examples of substituents (b)

include examples of substituents (a) mentioned above and also substituted or unsubstituted lower alkyl.

The substituted lower alkyl and the substituted lower alkoxy mentioned as examples of substituents (a) and substituents (b) each have one to a substitutable number, preferably 1 to 3 substituents (i) which are the same or different. Examples of substituents (i) include halogen.

In the exemplification of substituents (a), substituents (b) and substituents (i), the lower alkyl moiety of the lower alkyl, the lower alkoxy, the lower alkoxycarbonyl, the mono-lower alkylaminocarbonyl, the di-lower alkylaminocarbonyl, the mono-lower alkylamino and the di-lower alkylamino has the same meaning as the above-mentioned lower alkyl moiety of the lower alkoxy; the lower alkanoyl moiety of the lower alkanoyl and the lower alkanoyloxy has the same meaning as the above-mentioned lower alkanoyl moiety of the lower alkanoyloxy; and the halogen means iodine, bromine, chlorine and fluorine atoms. The two lower alkyl moieties of the di-lower alkylamino and the di-lower alkylaminocarbonyl may be the same or different.

The pharmaceutically acceptable salts of Compounds (I) include pharmaceutically acceptable acid addition salts, metal salts, ammonium salts, organic amine addition salts and amino acid addition salts.

Examples of the pharmaceutically acceptable acid addition salts of Compounds (I) include inorganic acid addition salts such as hydrochloride, sulfate, nitrate and phosphate, and organic acid addition salts such as acetate, maleate, fumarate and citrate. Examples of the pharmaceutically acceptable metal salts of Compounds (I) include alkali metal salts such as sodium salt and potassium salt, alkaline earth metal salts such as magnesium salt and calcium salt, aluminum salt and zinc salt. Examples of the pharmaceutically acceptable ammonium salts of Compounds (I) include ammonium salt and tetramethylammonium salt. Examples of the pharmaceutically acceptable organic amine addition salts of Compounds (I) include addition salts such as morpholine and piperidine. Examples of the pharmaceutically acceptable amino acid addition salts of Compounds (I) include addition salts such as glycine, phenylalanine, lysine, aspartic acid and glutamic acid.

For some of Compounds (I) used in the present invention, there may exist various kinds of stereoisomers, positional isomers, tautomeric isomers and the like. All possible isomers including them and mixtures thereof in arbitrary mixture ratios may be used in the present invention.

The processes for producing Compounds (I) are described below.

In the processes shown below, when the defined groups undergo changes under the reaction conditions or are not suitable to carry out the processes, production can be easily performed by applying means usually used in synthetic organic chemistry, such as protection of functional groups, removal of protecting groups, etc. [e.g., T.W. Greene, Protective Groups in Organic Synthesis, third edition, John Wiley & Sons Inc. (1999)]. If necessary, the order of reaction steps such as introduction of a substituent may be changed.

Compounds (I) can be obtained, for example, according to Production Processes 1 to 4 shown below, though some of them can also be obtained as commercial products.

Production Process 1:

Compound (Ia), i.e. Compound (I) in which n is 1, -$Q^1$- is -O-, Y and Z are oxygen atoms, and $X^2$ is hydroxy can be produced by the method of P. Kosma, et al. [Carbohydrate Research, Vol. 338, No. 23, p. 2571-2589 (2003)] or methods similar thereto.

Specifically, Compound (Ia) can be produced according to the processes described below and the like.

Step 1-1:

Compound (Ia) can be produced according to the following steps.

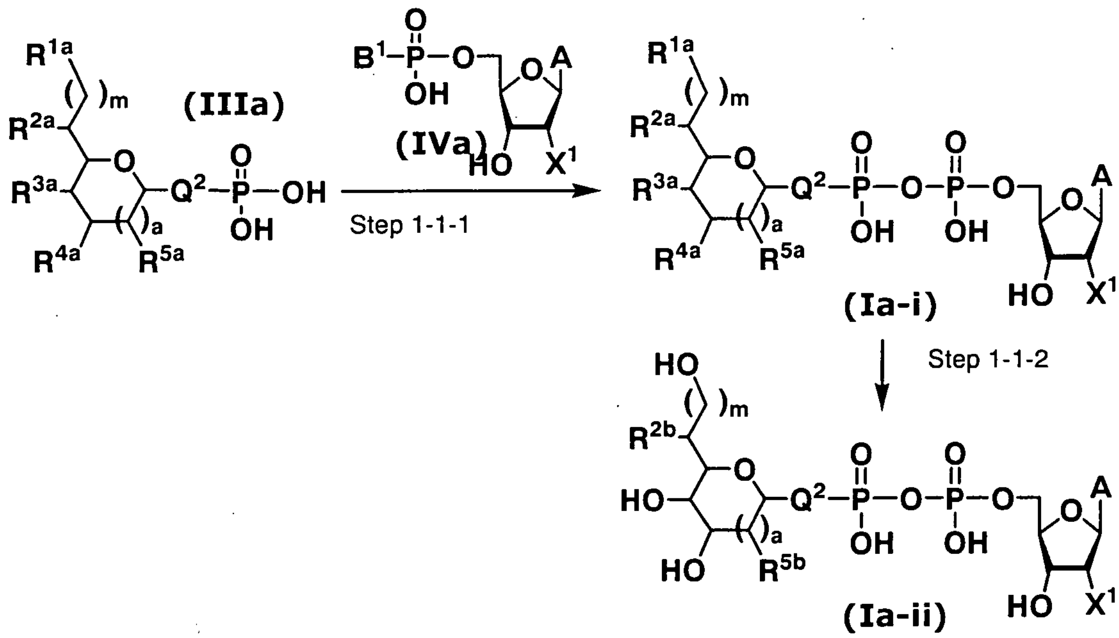


[wherein a, m, $X^1$, $Q^2$ and A each have the same meanings as defined above; $R^{1a}$, $R^{3a}$ and $R^{4a}$ each independently represents substituted or unsubstituted lower alkanoyloxy, substituted or unsubstituted lower alkoxy, substituted or unsubstituted lower alkenyloxy, substituted or unsubstituted aralkyloxy, or substituted or unsubstituted heterocyclic alkyloxy; $R^{2a}$ and $R^{5a}$ each independently represents a hydrogen atom, substituted or unsubstituted lower alkanoyloxy, substituted or unsubstituted lower alkoxy, substituted or unsubstituted lower alkenyloxy, substituted or unsubstituted aralkyloxy, or substituted or unsubstituted heterocyclic alkyloxy; $R^{2b}$ and $R^{5b}$ each independently represents a hydrogen atom or hydroxy; and $B^1$ represents mono-lower alkylamino, di-lower alkylamino, morpholino, piperidino or -OPO $(OR^6)_2$ (wherein $R^6$ represents a hydrogen atom, lower alkyl or aryl).]

Step 1-1-1

Among Compounds (Ia), Compound (Ia-i) can be obtained by reacting Compound (IIIa) with 1 to 3 equivalents of Compound (IVa) in a solvent, if necessary in the presence of a base or an acid, at a temperature usually between -20°C and 250°C, preferably between 0°C and 40°C for 1 to 72 hours. Examples of the solvent include pyridine, dimethylformamide (DMF), dimethylacetamide (DMA), dimethyl sulfoxide (DMSO), sulfolane and N-methylpyrrolidone (NMP). Examples of the base include potassium carbonate, sodium carbonate, potassium hydroxide, sodium hydroxide, sodium hydrogencarbonate and triazole, and examples of the acid include tetrazole. The base or acid is usually used in an amount of 0.1 to 3 equivalents based on Compound (IIIa).

Step 1-1-2

Compound (Ia-ii), i.e. Compound (Ia) in which $R^1$, $R^3$ and $R^4$ are hydroxy, and $R^2$ and $R^5$ each independently represents a hydrogen atom or hydroxy can be obtained by: (1) subjecting Compound (Ia-i) to hydrolysis reaction in a solvent in the presence of a base; or (2) subjecting Compound (Ia-i) to hydrogenolysis with a catalyst such as palladium, platinum oxide, rhodium or Raney nickel in a solvent in a hydrogen atmosphere or in the presence of an appropriate hydrogen source, if necessary in the presence of preferably 1 to 5 equivalents of an additive, at a temperature between -20°C and the boiling point of the solvent used for 5 minutes to 72 hours. These processes can be appropriately selected according to the structure of Compound (Ia-i), substituents, etc. and be carried out.

In (1), an aqueous solvent is used as the solvent. For example, a solvent prepared by adding water to methanol, ethanol, pyridine, DMF, DMA, DMSO, sulfolane, NMP or a mixture thereof is used.

As the base, ammonia, triethylamine, sodium methoxide, sodium ethoxide, potassium carbonate, sodium carbonate, potassium hydroxide, sodium hydroxide, sodium hydrogencarbonate or the like is used usually in an amount of 0.3 to 100 equivalents based on Compound (Ia-i).

In (2), methanol, ethanol, ethyl acetate, water or the like is used as the solvent.

The catalyst is used preferably in an amount of 0.001 to 10 equivalents, more preferably 0.01 to 1 equivalent based on Compound (Ia-i).

As the additive, a base or an acid is used preferably in an amount of 0.01 to 2 equivalents based on Compound (Ia-i). Examples of the base include ammonia and diethylamine, and examples of the acid include acetic acid.
As the hydrogen source, formic acid, ammonium formate, sodium formate, cyclohexenone or the like is used preferably in an amount of 2 equivalents to a large excess.

Step 1-2:

Compound (IVa) can be obtained as a commercial product or by the following process.

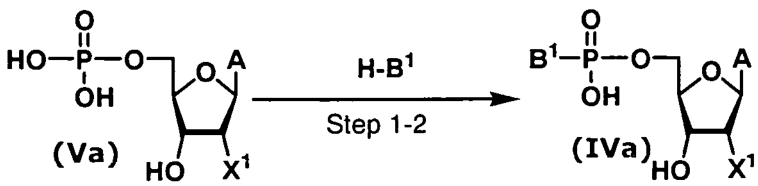


(wherein $X^1$, A and $B^1$ each have the same meanings as defined above.)
Compound (IVa) can be obtained by: (1) treating Compound (Va) with preferably 1 equivalent to a large excess amount of a chlorinating agent or a brominating agent in a solvent or without a solvent, if necessary in the presence of preferably 0.1 to 10 equivalents of an appropriate additive, at a temperature between -20˚C and 150˚C for 5 minutes to 72 hours; and (2) reacting the obtained compound with preferably 1 to 10 equivalents of H-$B^1$ (wherein $B^1$ has the same meaning as defined above) in a solvent or without a solvent, if necessary in the presence of preferably 1 to 10 equivalents of a base, at a temperature between -20˚C and 150˚C for 5 minutes to 72 hours.
In (1), thionyl chloride, oxalyl chloride, phosphorus oxychloride or the like is used as the chlorinating agent, and thionyl bromide, phosphorus oxybromide or the like is used as the brominating agent.
As the additive, DMF, pyridine or the like is used.
Examples of the solvent include dichloromethane, chloroform, 1,2-dichloroethane, toluene, ethyl acetate, acetonitrile, diethyl ether, tetrahydrofuran (THF), 1,2-dimethoxyethane (DME), dioxane, DMF, DMA, NMP and pyridine, which are used alone or as a mixture.
In (2), potassium carbonate, potassium hydroxide, sodium hydroxide, potassium tert-butoxide, triethylamide, diisopropylethylamine, N-methylmorpholine, pyridine, 1,8-diazabicyclo[5.4.0]-7-undecene (DBU), 4-dimethylaminopyridine (DMAP) or the like is used as the base.
Examples of the solvent include methanol, ethanol, dichloromethane, chloroform, 1,2-dichloroethane, toluene, ethyl acetate, acetonitrile, diethyl ether, THF, DME, dioxane, DMF, DMA, NMP, pyridine and water, which are used alone or as a mixture.
Alternatively, Compound (IVa) can also be obtained by reacting Compound (Va) with preferably 1 to 5 equivalents of H-$B^1$ (wherein $B^1$ has the same meaning as defined above) in a solvent in the presence of preferably 1 to 5 equivalents of a condensing agent, if necessary in the presence of preferably 1 to 5 equivalents of an additive, at a temperature between -20˚C and the boiling point of the solvent used for 5 minutes to 72 hours.
Examples of the condensing agent include 1,3-dicyclohexanecarbodiimide (DCC), 1-ethyl-3-(3-dimethylaminopropyl)carbodiimide hydrochloride (EDC) and carbonyldiimidazole (CDI).
Examples of the additive include 1-hydroxybenzotriazole monohydrate (HOBt).
Examples of the solvent include methanol, ethanol, dichloromethane, chloroform, 1,2-dichloroethane, toluene, ethyl acetate, acetonitrile, diethyl ether, THF, DME, dioxane, DMF, DMA, NMP, pyridine and water, which are used alone or as a mixture.

Step 1-3:

Compound (IIIa) can be obtained as a commercial product or by the following process.

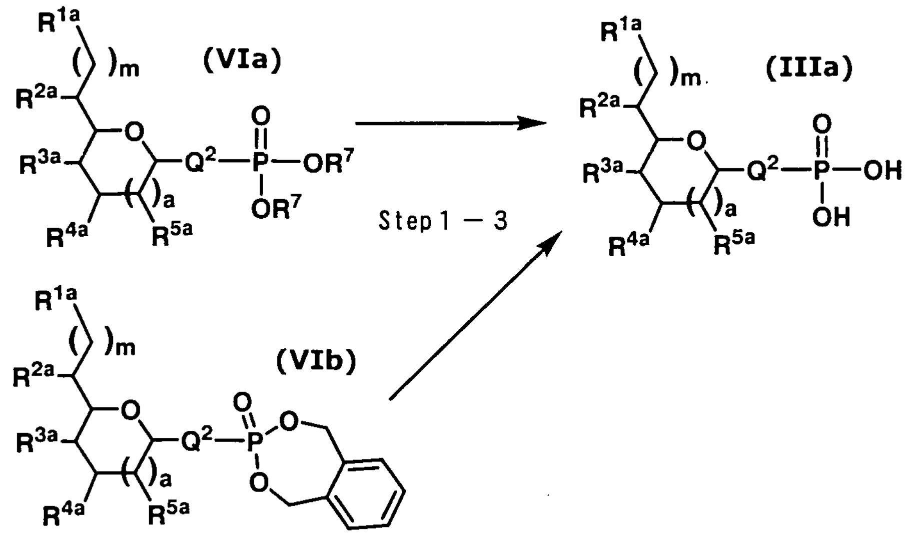


(wherein a, m, $R^{1a}$, $R^{2a}$, $R^{3a}$ , $R^{4a}$, $R^{5a}$ and $Q^2$ each have the same meanings as defined above; and $R^7$ represents lower alkyl, substituted or unsubstituted phenyl, or benzyl.)

Compound (IIIa) can be produced by treating Compound (VIa) or Compound (VIb) in a solvent in the presence of preferably 2 equivalents to a large excess amount of an additive at a temperature between 0˚C and the boiling point of the solvent used for 5 minutes to 72 hours.

Examples of the additive include acids such as hydrochloric acid, bases such as potassium carbonate, lithium hydroxide, potassium hydroxide, sodium hydroxide and sodium methoxide, trimethylsilyl bromide and trimethylsilyl iodide. These additives can be appropriately selected according to the structure of the compound, substituents, etc. and be carried out.

Examples of the solvent include methanol, ethanol, dichloromethane, chloroform, 1,2-dichloroethane, toluene, ethyl acetate, acetonitrile, diethyl ether, THF, DME, dioxane, DMF, DMA, NMP and pyridine, which are used alone or used with water.

Alternatively, Compound (IIIa) can also be obtained by subjecting Compound (VIa) or Compound (VIb) to hydrogenolysis with a catalyst such as palladium, platinum oxide, rhodium or Raney nickel in a solvent in a hydrogen atmosphere or in the presence of an appropriate hydrogen source, if necessary in the presence of preferably 1 to 5 equivalents of an additive, at a temperature between -20˚C and the boiling point of the solvent used for 5 minutes to 72 hours. These processes can be appropriately selected according to the structure of the compound, substituents, etc. and be carried out. The catalyst is used preferably in an amount of 0.001 to 10 equivalents, more preferably 0.01 to 1 equivalent based on Compound (VIa) or Compound (VIb).

As the hydrogen source, formic acid, ammonium formate, sodium formate, cyclohexenone or the like is used preferably in an amount of 2 equivalents to a large excess.

Examples of the solvent include methanol, ethanol, dichloromethane, chloroform, 1,2-dichloroethane, toluene, ethyl acetate, acetonitrile, diethyl ether, THF, DME, dioxane, DMF, DMA, NMP and water, which are used alone or as a mixture.

Examples of the additive include bases such as ammonia and diethylamine, and acids such as acetic acid.

Step 1-4:

Compound (VIa) or Compound (VIb) can be obtained as a commercial product or by the processes described below.

For example, Compound (VIa-i) or Compound (VIb-i), i.e. Compound (VIa) or Compound (VIb) in which $-Q^2-$ is -O-or $-CH_2-O-$ can be obtained by the following process.

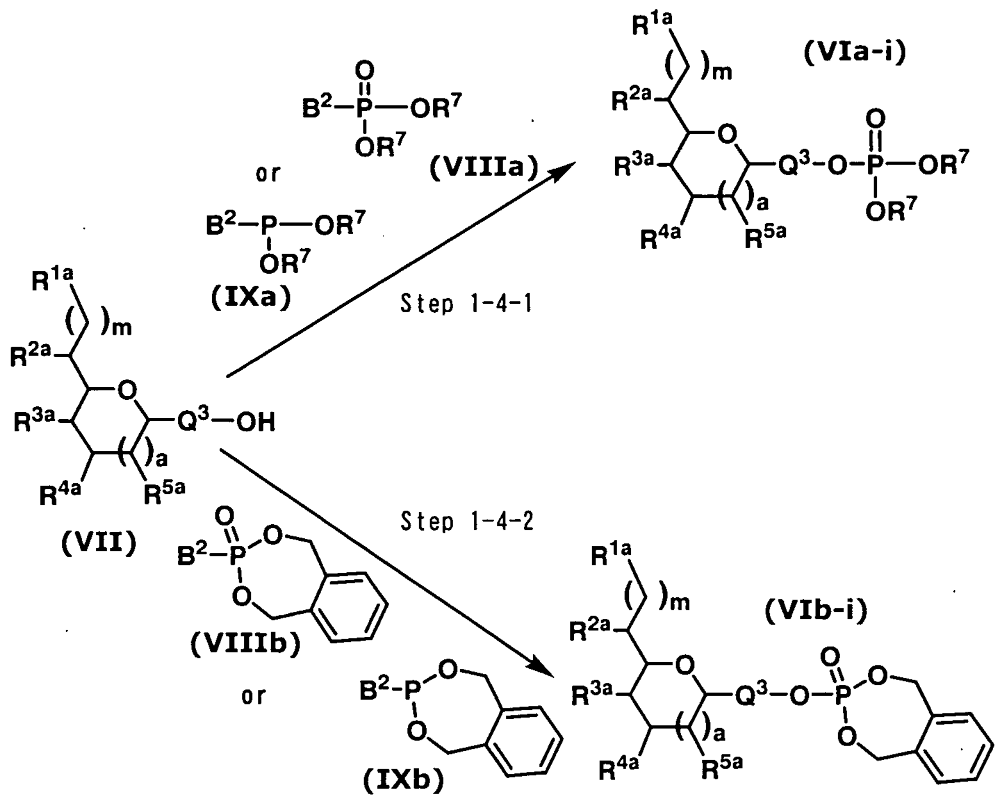


(VIIIa) or (IXa); (VII); (VIa-i); (VIIIb) or (IXb); (VIb-i)

[wherein $Q^3$ represents $-CH_2-$ or a single bond; a, m, $R^{1a}$, $R^{2a}$, $R^{3a}$, $R^{4a}$, $R^{5a}$ and $R^7$ each have the same meanings as defined above; and $B^2$ represents halogen, mono-lower alkylamino, di-lower alkylamino, morpholino, piperidino or $-OPO(OR^6)_2$ (wherein $R^6$ has the same meaning as defined above).]

Compound (VIa-i) or Compound (VIb-i) can be obtained by reacting Compound (VII) with usually 1 to 10 equivalents, preferably 1 to 3 equivalents of Compound (VIIIa) or Compound (VIIIb), respectively, in a solvent in the presence of a base or an acid. The reaction is carried out at a temperature usually between -20˚C and 250˚C, preferably between 20˚C and 40˚C for 1 to 120 hours. Examples of the solvent include dichloromethane, ethyl acetate and DMF. Examples of the base include triethylamine, pyridine, DMAP and triazole, and examples of the acid include tetrazole. The base or acid is usually used in an amount of 0.1 to 3 equivalents based on Compound (VII).

Compound (VIa-i) or Compound (VIb-i) can also be obtained by reacting Compound (VII) with usually 1 to 10 equivalents, preferably 1 to 3 equivalents of Compound (IXa) or Compound (IXb), respectively, in a solvent in the presence of an additive, and then, treating the obtained compound with usually 1 to 10 equivalents, preferably 1 to 3 equivalents of an oxidizing agent such as aqueous hydrogen peroxide. The reaction of Compound (VII) with Compound (IXa) or Compound (IXb) is carried out at a temperature usually between -20˚C and 250˚C, preferably between 20˚C and 40˚C for 1 to 120 hours, and the treatment with an oxidizing agent is carried out at a temperature usually between -20˚C and 50˚C, preferably between 0˚C and 40˚C for 1 to 120 hours.

These processes can be appropriately selected according to the structure of Compound (VII), substituents, etc. and be carried out.

Examples of the solvent include dichloromethane, ethyl acetate and DMF.

As the additive, triethylamine, pyridine, DMAP, triazole, tetrazole or the like is used usually in an amount of 0.1 to 3 equivalents based on Compound (VII).

Compound (VIIIa) or Compound (VIIIb) and Compound (IXa) or Compound (IXb) can be obtained as commercial products or by the method of C. C. Tang, et al. [Synthetic Communication, Vol. 28, No. 15, p. 2769 (1998)] or methods similar thereto.

Step 1-5:

Compound (VIa-ii), i.e. Compound (VIa) in which $-Q^2-$is $-CH_2-$ can be obtained by the method of R. A. Field, et al. [Tetrahedron Letters, Vol. 42, p. 2231-2234 (2001)] or methods similar thereto.

Specifically, Compound (VIa-ii) can be obtained by the following process.

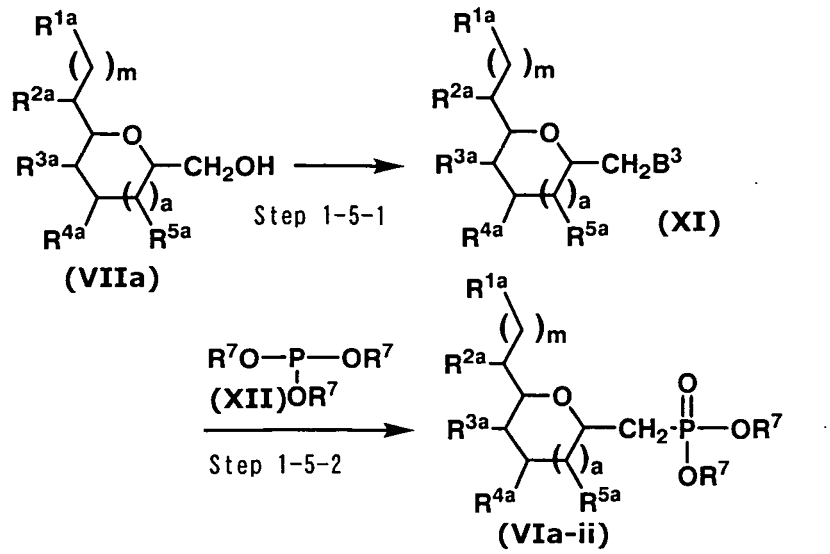


[wherein a, m, $R^{1a}$, $R^{2a}$, $R^{3a}$, $R^{4a}$, $R^{5a}$ and $R^7$ each have the same meanings as defined above; and $B^3$ represents a leaving group such as halogen (e.g. iodine or bromine), trifluoromethanesulfonyloxy, methanesulfonyloxy or p-toluenesulfonyloxy.]

Compound (VIa-ii) can be obtained by: (1) treating Compound (VIIa) with 1 equivalent to a large excess amount of a halogenating agent or 1 to 10 equivalents of a sulfonylating agent in a solvent, if necessary in the presence of a catalytic amount to 10 equivalents of an additive, at a temperature between -20°C and 150°C for 5 minutes to 72 hours to obtain Compound (XI); and (2) treating Compound (XI) with a solvent amount of Compound (XII) at a temperature between -20°C and 150°C for 5 minutes to 72 hours to perform Arbuzov reaction.

In (1), bromine, iodine, hydrobromic acid or the like is used as the halogenating agent, and anhydrous trifluoromethanesulfonic acid, anhydrous methanesulfonic acid, methanesulfonyl chloride, p-toluenesulfonyl chloride or the like is used as the sulfonylating agent.

Examples of the additive include bases such as triethylamine, diisopropylethylamine and pyridine, and triphenylphosphine.

Examples of the solvent include dichloromethane, chloroform, 1,2-dichloroethane, toluene, ethyl acetate, acetonitrile, diethyl ether, THF, DME, dioxane, DMF, DMA, NMP and pyridine, which are used alone or as a mixture.

Compound (XII) can be obtained as a commercial product.

Compound (VIIa-i), i.e. Compound (VIIa) in which m is 0 can be synthesized by the method of M. J. Hadd, et al. [Journal of Organic Chemistry, Vol. 62, p. 6961-6967 (1997)], and Compound (VIIa-ii), i.e. Compound (VIIa) in which m is 1 can be synthesized by the method of A. Murai, et al. [Tetrahedron, Vol. 54, p. 21-44 (1998)].

Step 1-6:

Compound (VIa-iii), i.e. Compound (VIa) in which $-Q^2-$ is a single bond can be obtained by the method of A. Vasella, et al. [Helvetica Chimica Acta, Vol. 69, p. 25-34 (1986)] or methods similar thereto.

Specifically, Compound (VIa-iii) can be obtained by the following process.

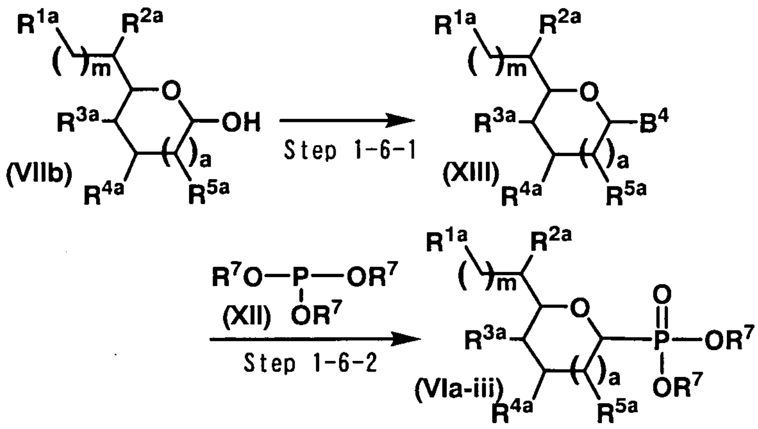


(wherein a, m, $R^{1a}$, $R^{2a}$, $R^{3a}$, $R^{4a}$, $R^{5a}$ and $R^7$ each have the same meanings as defined above; and $B^4$ represents lower alkanoyloxy.)

Compound (Via-iii) can be obtained by: (1) treating Compound (VIIb) with preferably 1 to 10 equivalents of an acylating agent in a solvent, if necessary in the presence of preferably a catalytic amount to 10 equivalents of a base, at a temperature between -20˚C and 150˚C for 5 minutes to 72 hours to obtain Compound (XIII); and (2) treating Compound (XIII) with 0.1 to 10 equivalents of Compound (XII) in a solvent, if necessary in the presence of preferably a catalytic amount to 10 equivalents of an additive, at a temperature between -20˚C and 150˚C for 5 minutes to 72 hours.

In (1), acetyl chloride, acetic anhydride or the like is used as the acylating agent, and triethylamine, diisopropylethylamine, pyridine or the like is used as the base.

Examples of the solvents used in (1) and (2) include dichloromethane, chloroform, 1,2-dichloroethane, toluene, ethyl acetate, acetonitrile, diethyl ether, THF, DME, dioxane, DMF, DMA, NMP and pyridine, which are used alone or as a mixture.

In (2), trifluoromethanesulfonic acid trimethylsilyl ether or the like is used as the additive usually in an amount of 0.1 to 3 equivalents based on Compound (XIII).

Compound (VIIb) can be obtained as a commercial product. Compound (VIIb-i), i.e. Compound (VIIb) in which m and a are 1 can be obtained by the method of R. K. Sood, et al. [Canadian Journal of Chemistry, Vol. 72, p. 247-251 (1994)], the method of S. Bertil, et al. [Carbohydrate Research, Vol. 67, p. 263-236 (1978)], the method of P. Manitto, et al. [Tetrahedron Letters, Vol. 28, No. 42, p. 5047-5048 (1987)], the method of Akepilotti, et al. [Acta Chemica Scandinavica, Vol. 26, p. 4143-4146 (1972)] or methods similar thereto. Compound (VIIb-ii), i.e.
Compound (VIIb) in which m is 2 and a is 1 can be obtained by the method of Kishi, et al. [Journal of American Chemical Society, Vol. 118, No. 34, p. 7946-7968 (1996)], the method of J. H. van Boom, et al. [Organic Letters, Vol. 2, No. 9, p. 1275-1277 (2000)] or methods similar thereto.

Production Process 2:

Compound (Ib), i.e. Compound (I) in which n is 1, $-Q^1-$ is $-CH_2-$, and $Q^2$, Y and Z are oxygen atoms can be produced, for example, by the process described below.

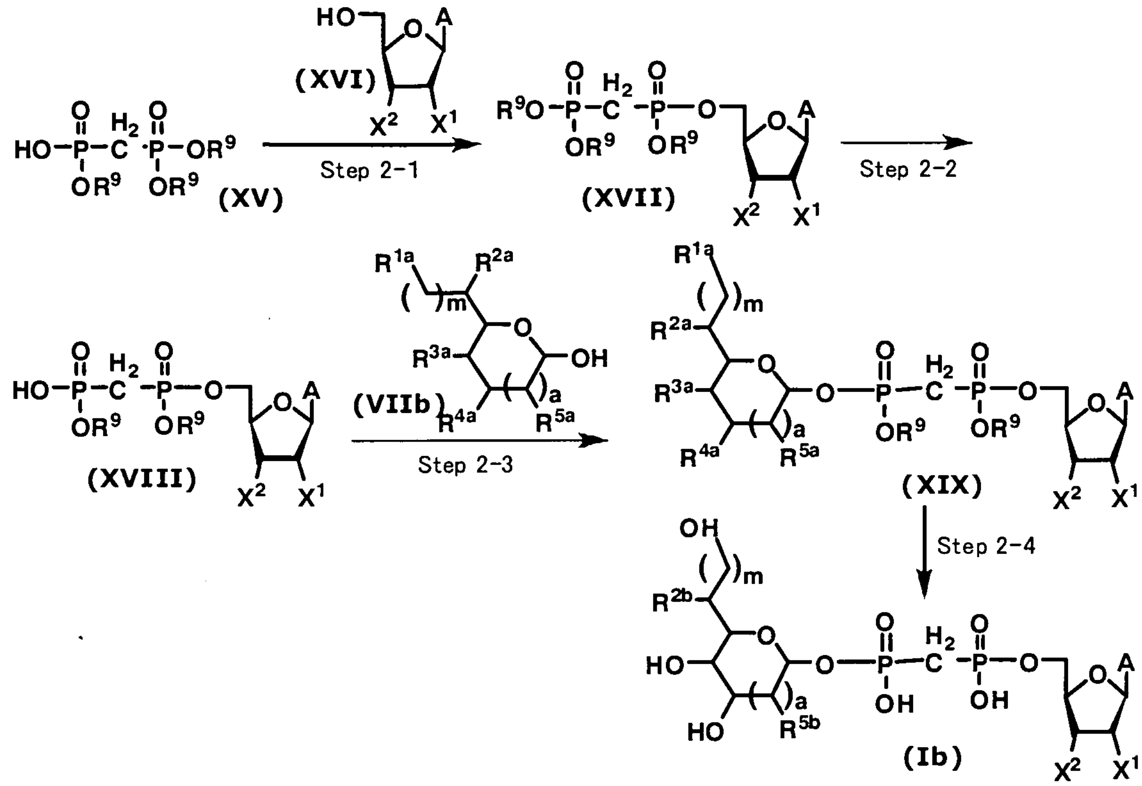

(XV) (XVI) (XVII) (XVIII) (VIIb) (XIX) (Ib)

(wherein a, m, $R^{1a}$, $R^{2a}$, $R^{3a}$, $R^{4a}$, $R^{5a}$, $X^1$, $X^2$ and A each have the same meanings as defined above; and $R^9$ represents substituted or unsubstituted benzyl.)

Step 2-1:

Compound (XVII) can be obtained by the method of C. Mioskowski, et al. [Journal of Organic Chemistry, Vol. 67, No. 1, p. 146-153 (2002)] or methods similar thereto. Specifically, Compound (XVII) can be obtained, for example, by subjecting Compound (XV) to Mitsunobu reaction with Compound (XVI) in a solvent in the presence of preferably 1 to 10 equivalents of a phosphine compound and preferably 1 to 10 equivalents of an azo compound, or in the presence of 1 to 10 equivalents of a phosphorane compound, at a temperature between -78°C and the boiling point of the solvent used for 5 minutes to 72 hours. These processes can be appropriately selected according to the structure of Compound (XV), substituents, etc. and be carried out.

Examples of the solvent include dichloromethane, chloroform, 1,2-dichloroethane, toluene, ethyl acetate, acetonitrile, diethyl ether, THF, DME, dioxane, DMF, DMA, NMP and pyridine, which are used alone or as a mixture.

As the azo compound, diethyl azodicarboxylate (DEAD), diisopropyl azodicarboxylate (DIAD), N,N,N',N'-tetramethyl azadicarboxamide, 1,1'-(azadicarbonyl)dipiperazine, N,N,N',N'-tetraisopropyl azadicarboxamide or the like is used usually in an amount of 1 to 10 equivalents based on Compound (XV).

As the phosphine compound, triphenylphosphine, tributylphosphine or the like is used usually in an amount of 1 to 10 equivalents based on Compound (XV).

As the phosphorane compound, a Tsunoda reagent such as cyanomethylene tributylphosphorane or cyanomethylene trimethylphosphorane or the like is used usually in an amount of 1 to 10 equivalents based on Compound (XV).

Compound (XV-i), i.e. Compound (XV) in which $R^9$ is benzyl can be obtained by the method of C. Mioskowski, et al. [Journal of Organic Chemistry, Vol. 60, p. 2946-2947 (1995)] or methods similar thereto.

Step 2-2:

Compound (XVIII) can be obtained by the method of C. Mioskowski, et al. [Journal of Organic Chemistry, Vol. 60, p. 2946-2947 (1995)] or methods similar thereto.

Specifically, Compound (XVIII) can be obtained by: (1) treating Compound (XVII) with a tertiary amine in a solvent; or (2) subjecting Compound (XVII) to hydrogenolysis with a catalyst such as palladium, platinum oxide, rhodium or Raney nickel in a solvent in a hydrogen atmosphere or in the presence of an appropriate hydrogen source, if necessary in the presence of preferably 1 to 5 equivalents of an additive, at a temperature between -20°C and the boiling point of the solvent used for 5 minutes to 72 hours. These processes can be appropriately selected according to the structure of the compound, substituents, etc. and carried out.

In (1), the reaction is carried out at a temperature usually between -20°C and 250° C, preferably between 70°C and 120°C for 1 to 24 hours. Examples of the solvent include toluene, DMF and dichloroethane. As the tertiary amine, quinuclidine, diazabicyclo[2,2,2]octane, N-methylmorpholine or the like is used usually in an amount of 1 equivalent based on Compound (XVII).

In (2), methanol, ethanol, ethyl acetate, water or the like is used as the solvent. As the hydrogen source, formic acid, ammonium formate, sodium formate, cyclohexenone or the like is used preferably in an amount of 2 equivalents to a large excess. The catalyst is used preferably in an amount of 0.001 to 10 equivalents, more preferably 0.01 to 1 equivalent based on Compound (XVII). As the additive, ammonia, diethylamine, acetic acid or the like is used preferably in an amount of 0.01 to 2 equivalents based on Compound (XVII).

Step 2-3:

Compound (XIX) can be obtained by reacting Compound (XVIII) with Compound (VIIb) by the method of Mitsunobu, et al. [Synthesis, p. 1 (1981)] or under similar conditions as in Step 2-1.

Step 2-4:

Compound (Ib) can be obtained by treating Compound (XIX) under similar conditions as in Step 1-1-2.

<u>Production Process 3:</u>

Compound (XIX) can also be obtained, for example, by the following process.

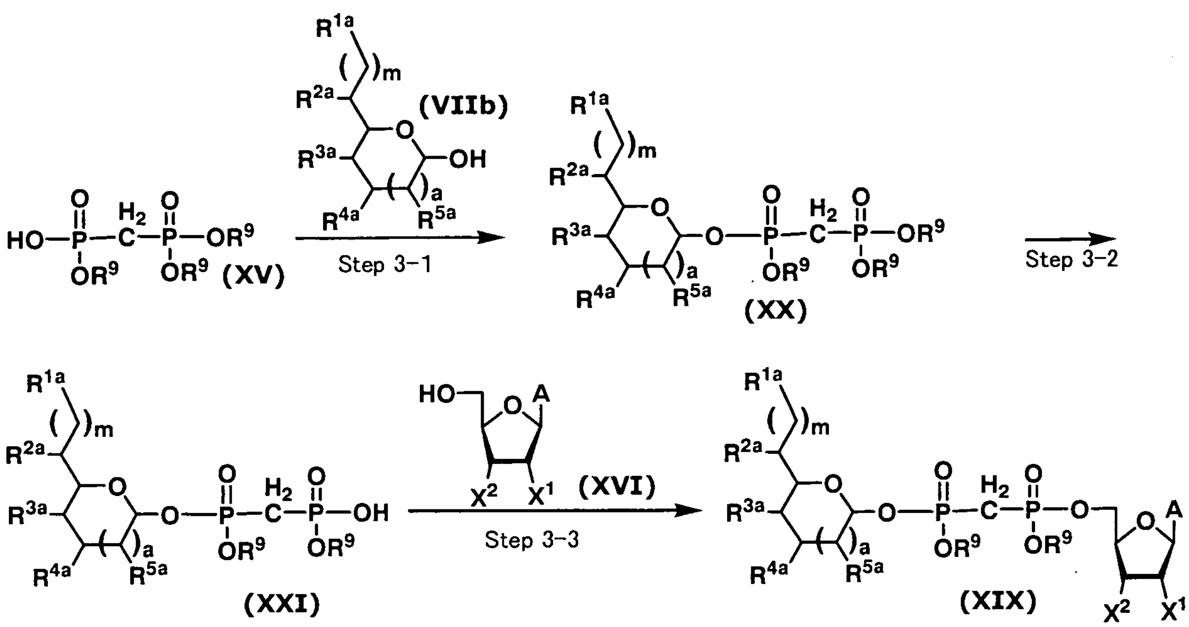


(wherein a, m, $R^{1a}$, $R^{2a}$, $R^{3a}$, $R^{4a}$, $R^{5a}$, $R^9$, $X^1$, $X^2$ and A each have the same meanings as defined above.)

Step 3-1:

Compound (XX) can be obtained by reacting Compound (XV) with Compound (VIIb) under similar conditions as in Step 2-1.

Step 3-2:

Compound (XXI) can be obtained by subjecting Compound (XX) to reaction under similar conditions as in Step 2-2.

Step 3-3:

Compound (XIX) can be obtained by reacting Compound (XXI) with Compound (XVI) under similar conditions as in Step 2-1.

Compounds (I), particularly Compound 16 [adenosine=5'-(D-glycero-β-D-mannoheptopyranosyl)diphosphate: ADP-D-glycero-β-D-mannoheptose], etc. can also be produced by the following culturing method.

Production Process 4:

Compounds (I), particularly Compound 16 can be produced by culturing a microorganism having the ability to produce ADP-D-glycero-β-D-mannoheptose in a medium, allowing the compound to form and accumulate in the culture, and recovering the compound from the culture. As the microorganism having the ability to produce ADP-D-glycero-β-D-mannoheptose, any microorganism can be employed so long as it is a microorganism belonging to Gram-negative bacteria and having the ability to produce ADP-D-glycero-β-D-mannoheptose. Further, mutants obtained by subjecting such microorganism to artificial mutation techniques such as ultraviolet irradiation, X-ray irradiation and treatment with a mutagenic agent, and spontaneous mutants can also be used in the present invention, so long as they have the ability to produce ADP-D-glycero-β-D-mannoheptose.

Examples of the microorganism having the ability to produce ADP-D-glycero-β-D-mannoheptose include Massilia sp. KY 13101, a Gram-negative bacillus belonging to the genus Massilia which has been newly isolated from the soil by the present inventors.

For the culturing of the strains producing and accumulating ADP-D-glycero-β-D-mannoheptose of the present invention, ordinary culturing methods for microorganisms can be applied. As the medium, any of synthetic media and natural media can be used insofar as it is a medium appropriately containing carbon sources, nitrogen sources and inorganic substances which can be assimilated by the microorganism, necessary substances promoting growth and production and the like.

As the carbon sources, glucose, starch, dextrin, mannose, fructose, sucrose, lactose, xylose, arabinose, mannitol, molasses and the like can be used alone or in combination. Further, hydrocarbons, alcohols, organic acids and the like can be used depending upon the assimilability of the strain.

As the nitrogen sources, ammonium chloride, ammonium nitrate, ammonium sulfate, sodium nitrate, urea, peptone, meat extract, yeast extract, dry yeast, corn steep liquor, soybean flour, Casamino acid and the like can be used alone or in combination.

Additionally, inorganic salts such as sodium chloride, potassium chloride, magnesium sulfate, calcium carbonate, potassium dihydrogenphosphate, ferrous sulfate, calcium chloride, manganese sulfate, zinc sulfate and copper sulfate can be added according to need.

Further, trace components promoting the growth of the strain used or the production and accumulation of ADP-D-glycero-β-D-mannoheptose can be appropriately added.

As the culturing method, liquid culture, especially, submerged spinner culture is suitable. Culturing is carried out at a temperature of 16 to 37˚C, preferably 25 to 32˚C, at pH 4 to 10, preferably pH 6 to 8, and is usually completed in 1 to 7 days, whereby ADP-D-glycero-β-D-mannoheptose is formed and accumulated in the culture liquor and microbial cells. The pH adjustment of the medium is carried out by using aqueous ammonia, an ammonium carbonate solution or the like. The culturing is terminated when the amount of the product formed in the culture reached the maximum.

For isolating and purifying the formed ADP-D-glycero-β-D-mannoheptose from the culture, the methods conventionally used for the isolation and purification of ordinary microbial metabolites from the culture are applied. For example, the culture is extracted with a solvent such as ethanol or 2-propanol, and then subjected successively to polystyrene adsorbent, ODS column chromatography, gel filtration chromatography, ion exchange chromatography, reversed-phase high performance liquid chromatography and the like, whereby ADP-D-glycero-β-D-mannoheptose can be obtained.

Isolation and purification of the formed ADP-D-glycero-β-D-mannoheptose from the culture is carried out according to the methods conventionally used for the isolation and purification of ordinary microbial metabolites from the culture. For example, the culture is separated into a culture filtrate and microbial cells by filtration, and cell components are extracted from the cells with a solvent such as chloroform, acetone, methanol and ethanol. Subsequently, the extract and the culture filtrate are combined and passed through a column of a polystyrene adsorbent, for example, Diaion HP-20 (Mitsubishi Chemical Corporation) to adsorb active components, followed by elution with an aqueous solution of

acetic acid, an aqueous solution of ammonium acetate, methanol, ethanol, acetone or the like. The eluate is concentrated and subjected to octadecyl group-bound silica gel (ODS) column chromatography, ordinary-phase or reversed-phase high performance liquid chromatography, silica gel column chromatography and the like to obtain ADP-D-glycero-β-D-mannoheptose.

Shown below are the microbiological characteristics and the basis for identification of Massilia sp. KY 13101, a Gram-negative bacillus belonging to the class Proteobacteria, the beta subclass, the Oxalobacter group, the genus Massilia which has been newly isolated from the soil by the present inventors as a microorganism having the ability to produce ADP-D-glycero-β-D-mannoheptose.

(A) Morphological properties

The morphological characteristics of the strain KY 13101 grown on nutrient agar medium and nutrient broth are shown below.

(1) Shape of cells; rod
Size; 0.8 to 1.3 μm x 1.4 to 3.1 μm
(2) Polymorphism of cells; none
(3) Motility; motile
(4) Spore; not observed

(B) Cultural properties

(1) Bouillon agar plate culture

1. Growth; good growth, a viscous colony is formed, colony diameter reaches ca. 8 mm by culturing at 30°C for 3 days
2. Color; cream
3. Diffusible pigment; not produced

(2) Bouillon liquid culture

1. Growth on surface; none
2. Turbidity; turbid
3. Aggregation of cells; observed

(3) Litmus milk

1. Reaction; alkalified
2. Coagulation; not observed
3. Liquefaction; observed

(C) Physiological properties

(1) Gram staining; negative
(2) Reduction of nitrate; reduced to nitrite
(3) MR test; negative
(4) VP test; negative
(5) Formation of indole; negative
(6) Formation of hydrogen sulfide; negative
(7) Hydrolysis of starch; positive
(8) Utilization of citric acid; weakly positive
(9) Utilization of inorganic nitrogen source;

1. Nitrate; positive

2. Ammonia; positive

(10) Formation of pigment; no water-soluble pigment produced
(11) Urease; negative
(12) Oxidase; positive
(13) Catalase; positive
(14) β-Galactosidase; negative
(15) Growth range

1. pH; 6 to 8
2. Temperature; 13 to 42°C

(16) Attitude to oxygen; aerobic
(17) O-F test; oxidation
(18) Acid formation from sugars

1. Acid formation is observed from the following sugars; D-arabinose, L-arabinose, D-xylose, glucose, fructose, mannose, amygdalin, cellobiose, maltose, lactose, melibiose, salicin, trehalose, starch, glycogen
2. No acid formation is observed from the following sugars; glycerol, erythritol, ribose, L-xylose, adonitol, β-methyl-D-xyloside, galactose, sorbose, dulcitol, inositol, mannitol, sorbitol, α-methyl-D-mannoside, α-methyl-D-glucoside, N-acetyl-glucosamine, arbutin, salicin, inulin, melezitose, raffinose, xylitol, D-turanose, D-lyxose, D-tagatose, L-fucose, D-arabitol, L-arabitol

(19) Decomposition of esculin; positive
(20) Decomposition of arginine; negative
(21) Resistance to sodium chloride; no resistance to 4% NaCl
(22) Decarboxylation reaction of lysine; negative
(23) Decarboxylation reaction of ornithine; negative

(D) Chemical properties

(1) Isoprenoid quinone; ubiquinone-8
(2) G+C content of DNA; 65.4 mol%
(3) Cellular lipids

1. Phospholipids;
Main component: phosphatidylethanolamine
Sub-components: phosphatidylglycerol,
phosphatidylcholine, cardiolipin
2. Fatty acids;
Main components: C16:1 36%, C16:0 33%, C18:1 8%

(E) Other properties

A partial nucleotide sequence (ca. 1.5 kb) of the 16S ribosomal DNA (16S rDNA) of the strain KY 13101 was amplified by PCR to determine its sequence. The 16S rRNA or 16S rDNA sequences of known bacteria were subjected to multiple alignment using the sequence alignment program Clustal W, and a molecular phylogenetic analysis was performed by the neighbor-joining method using Phylip version 3.6 progaram. As a result, it was demonstrated that the strain KY 13101 belongs to the class Proteobacteria, the beta subclass, the Oxalobacter group, and is included in the genus Massilia. The result of the phylogenetic analysis using the 16S rDNA partial sequence is shown in Fig. 1.

This bacterial strain is a Gram-negative, aerobic, motile bacillus, and was found, as a result of the molecular phylogenetic analysis of 16S ribosomal DNA, to belong to the class Proteobacteria, the beta subclass, the Oxalobacter group, and to be located in the genus Massilia. The strain was most closely related to Massilia timonae among the species of the genus Massilia. Massilia timonae is a motile aerobic bacillus isolated from a clinical specimen. The 16S rDNA sequence similarity between the strain KY 13101 and the type strain of Massilia timonae was 96% (1361/1405 bp), which is not high enough to classify them as the same species. Further, Massilia timonae is a clinical isolate,

whereas the strain KY 13101 is a soil-isolate. The two strains, as judged from the difference of habitat area, the low similarity of 16S rDNA nucleotide sequences and the like, are classified as different species.

From the above results, the strain KY 13101 was identified as Massilia sp, a novel Gram-negative bacillus belonging to the class Proteobacteria, the beta subclass, the Oxalobacter group, the genus Massilia. The strain KY 13101 was deposited with National Institute of Technology and Evaluation, Patent Microorganisms Depositary (2-5-8, Kazusakamatari, Kisarazu-shi, Chiba, Japan) on March 30, 2007 under accession No. NITE BP-348.

The conversion of A, $X^1$ or $X^2$ in Compounds (I) or the functional groups contained in A can also be carried out according to known methods [e.g. R.C. Larock, Comprehensive Organic Transformations, 2nd edition, Vch Verlagsgesellschaft Mbh (1999)] or methods similar thereto.

The intermediates and the desired compounds in the above-described production processes can be isolated and purified by purification methods conventionally used in synthetic organic chemistry, for example, filtration, extraction, washing, drying, concentration, recrystallization, and various kinds of chromatography. The intermediates can also be subjected to the subsequent reactions without purification.

When it is desired to obtain a salt of Compound (I), in the case where Compound (I) is produced in the form of the salt, it can be purified as such, and where it is produced in the free form, it can be converted into a salt by an ordinary method, that is, by dissolving or suspending it in an appropriate solvent and then adding a desired acid or base thereto, followed by isolation and purification.

Further, Compounds (I) and pharmaceutically acceptable salts thereof may exist in the form of adducts with water or various solvents, and these adducts are also included in the present invention.

Specific examples of Compounds (I) used for the TLR9 agonist, immunostimulant, etc. of the present invention are shown in Tables 1 to 4 below, but the compounds of the present invention are not limited thereto. In the tables, Ac represents acetyl, and Me represents methyl.

Table 1

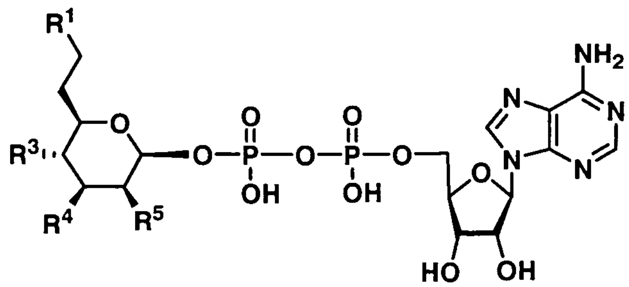

(I)

| Compd. No. | $R^1$ | $R^3$ | $R^4$ | $R^5$ |
|---|---|---|---|---|
| 1 | OAc | OAc | OAc | OAc |
| 2 | OH | OH | OH | OH |
| 3 | OAc | OMe | OMe | OMe |
| 4 | OH | OMe | OMe | OMe |

Table 2

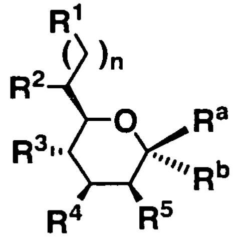

(I)

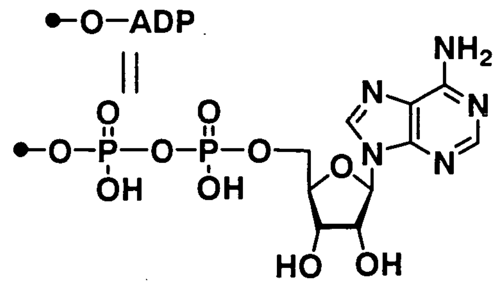

| Compd. No. | $R^a$ | $R^b$ | $R^3$ | $R^4$ | $R^5$ | 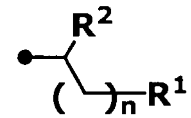 |
|---|---|---|---|---|---|---|
| 5 | H | O-ADP | OAc | OAc | OAc | •-$CH_2OAc$ |
| 6 | H | O-ADP | OH | OH | OH | •-$CH_2OH$ |
| 7 | O-ADP | H | OAc | OAc | OAc | •-$CH_2OAc$ |
| 8 | O-ADP | H | OH | OH | OH | •-$CH_2OH$ |
| 9 | H | O-ADP | OAc | OAc | OAc | 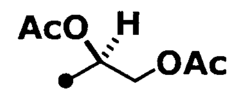 |
| 10 | O-ADP | H | OAc | OAc | OAc | 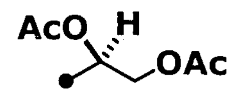 |
| 11 | H | O-ADP | OH | OH | OH | 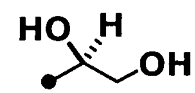 |
| 12 | O-ADP | H | OH | OH | OH | 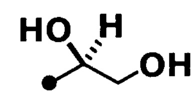 |
| 13 | H | O-ADP | OAc | OAc | OAc | 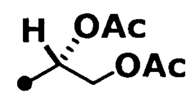 |

(continued)

| Compd. No. | $R^a$ | $R^b$ | $R^3$ | $R^4$ | $R^5$ | 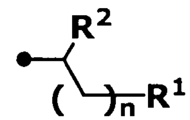 |
|---|---|---|---|---|---|---|
| 14 | O-ADP | H | OAc | OAc | OAc | 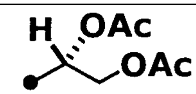 |
| 15 | H | O-ADP | OH | OH | OH | 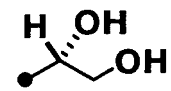 |
| 16 | O-ADP | H | OH | OH | OH | 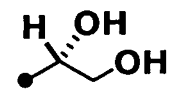 |

Table 3

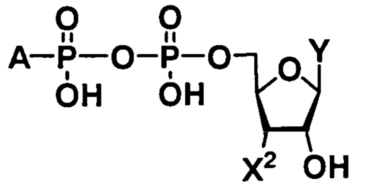 (I)

| Compd.No. | A | $X^2$ | Y |
|---|---|---|---|
| 17 | 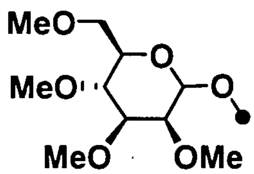 | OH | 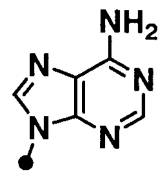 |
| 18 | 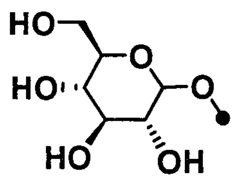 | OH | 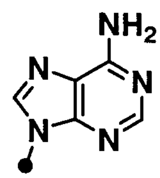 |
| 19 | 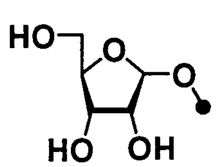 | OH | 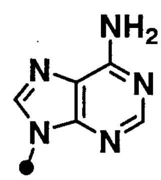 |
| 20 | 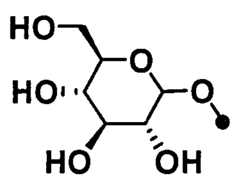 | OH | 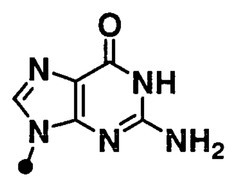 |
| 21 | 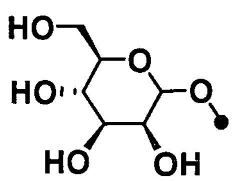 | OH | 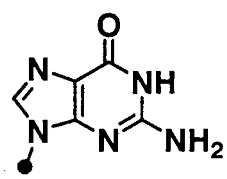 |
| 22 | 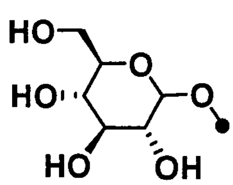 | OH | 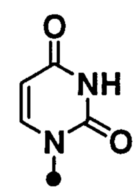 |
| 23 | 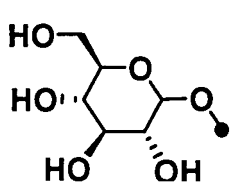 | H | 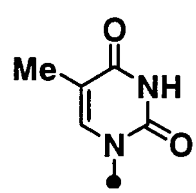 |

Table 4

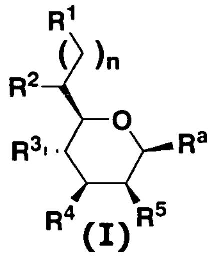

(I)

•—ADP =

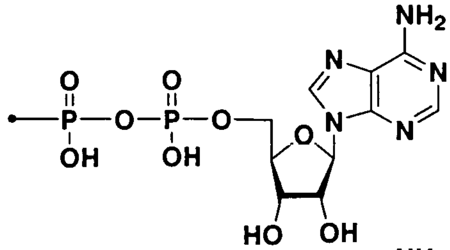

•—meADP =

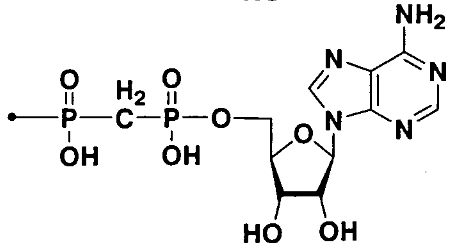

| Compd. No. | $R^a$ | $R^3$ | $R^4$ | $R^5$ | 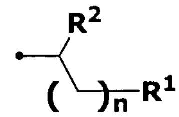 |
|---|---|---|---|---|---|
| 24 | $CH_2O$-ADP | OAc | OAc | OAc | •-$CH_2OAc$ |
| 25 | $CH_2O$-ADP | OH | OH | OH | •-$CH_2OH$ |
| 26 | $CH_2O$-ADP | OAc | OAc | OAc | •-$CH_2CH_2OAc$ |
| 27 | $CH_2O$-ADP | OH | OH | OH | •-$CH_2CH_2OH$ |
| 28 | $CH_2$-ADP | OH | OH | OH | •-$CH_2CH_2OH$ |
| 29 | ADP | OH | OH | OH | •-$CH_2CH_2OH$ |
| 30 | O-meADP | OH | OH | OH | •-$CH_2CH_2OH$ |
| **31** | O-ADP | OAc | OAc | OAc | •-$CH_2CH_2CH_2OAc$ |

The immunostimulatory activity of representative Compounds (I) is specifically described below by referring to test examples.

Test Example 1: Promoting Effect on NF-κB Promoter-dependent Transcriptional Activity

For the purpose of constructing an evaluation system for determination of NF-KB activity, the following stable transformant cell line was used. NF-κB luciferase reporter plasmid pIF-luc (containing a hygromycin resistant gene expression unit) introduced into cells was prepared by the method described in Japanese Published Unexamined Patent Application No. 169479/00. The plasmid was introduced into a human B-cell line, Namalwa (KJM-1) [Cytotechnology, Vol. 1, p. 151 (1988)], followed by culturing with addition of 0.3 mg/mL hygromycin B to select hygromycin-resistant cells. Among the selected hygromycin-resistant cells, a cell line with which enhancement of luciferase activity by TNF-α(10 ng/mL) stimulation was confirmed (hereinafter referred to as BGERKB5 cells) was used in the following test.

A test on promotion of NF-κB activity utilizing BGERKB5 cells can be carried out in the following manner. However, the following method is an example and does not limit the method for carrying out the test.

The BGERKB5 cells were suspended in RPMI1640·ITPSG medium having the following composition at a concentration of 1 x $10^6$ cells/mL (hereinafter referred to as the BGERKB5 cell suspension). A test compound was dissolved in phosphate buffer (pH 5) and diluted to a desired concentration with RPMI1640·ITPSG medium to prepare an aqueous solution. The BGERKB5 cell suspension (90 μL) was put into wells of a 96-well plate containing an aqueous solution of a test compound at each test concentration (10 μL), followed by incubation in a $CO_2$ incubator at 37°C for 5 hours.

<Composition of RPMI1640·ITPSG medium>

To RPMI1640 medium (Nissui Pharmaceutical Co., Ltd.) were added the following substances at the following concentration or ratio based on the medium (the resulting medium is hereinafter referred to as RPMI1640·ITPSG medium).

7.5% Sodium hydrogencarbonate (Invitrogen Corp.): 1/40 volume
200 mmol/L L-Glutamine solution (Nacalai Tesque, Inc.): 3%
penicillin·streptomycin solution (Invitrogen Corp., 5000 units/mL penicillin, 5000 μg/mL streptomycin): 0.5%
N-2-Hydroxyethylpiperazine-N'-2-ethanesulfonic acid (HEPES, Invitrogen Corp.): 10 mmol/L
Insulin (Sigma): 3 μg/mL
Transferrin (Sigma): 5 μg/mL
Sodium pyruvate (Nacalai Tesque, Inc.): 0.55 mg/mL
Selenious acid (Nacalai Tesque, Inc.): 17 ng/mL
Galactose (Nacalai Tesque, Inc.): 1 mg/mL

Steady-Glo (Promega Corp.) (100 μL) was added to the 96-well plate and the luciferase activity (RLU) in each well was measured using Multilabel Counter ARVO (Perkin-Elmer Corp.). The results are shown in Figs. 2 and 3.

According to Figs. 2 and 3, enhancement of luciferase activity was confirmed with Compounds 1, 2, 10, 12, 14, 16, 23, 30, etc. That is, it was revealed that Compounds (I) promote activation of NF-κB and possess evident immunostimulatory activity.

Test Example 2: Enhancing Effect on Expression Level of CD40, CD80 and CD86

Flow cytometry analysis of CD40, CD80, CD86, etc., which are co-stimulating molecules important for the activation of lymphocytes, can be carried out in the following manner. However, the following method is an example and does not limit the method for carrying out the flow cytometry.

The BGERKB5 cell suspension having a concentration of 1.1 x $10^6$ cells/mL obtained in Test Example 1 (90 μL) was put into wells of a 96-well plate containing an aqueous solution of a test compound prepared according to the method described in Test Example 1 (10 μL) (final concentration: 50 nmol/L), followed by incubation in a $CO_2$ incubator at 37°C for 12 hours (test compound group). As a control test, a control group without addition of a test compound was also prepared.

The following experiments were carried out at a temperature not more than 4°C. The cells were washed twice with 200 μL of phosphate buffer (PBS) containing 1% bovine serum albumin (Fraction V, Sigma) and 0.05% sodium azide, followed by replacement with the same buffer (100 μL). A phycoerythrin-labeled mouse anti-human CD40 monoclonal antibody solution (BD Biosciences), a phycoerythrin-labeled mouse anti-human CD80 monoclonal antibody solution (BD Biosciences) or a phycoerythrin-labeled mouse anti-human CD86 monoclonal antibody solution (BD Biosciences) (2 μL) was added to the cells, and after stirring, the cells were allowed to stand in the dark for 50 minutes.

The cells were washed twice with 200 μL of phosphate buffer (PBS) containing 1% bovine serum albumin (Fraction V, Sigma) and 0.05% sodium azide, followed by replacement with the same buffer (500 μL).

The cell count was determined using EPICS XL-MCL (Beckman Coulter, Inc., excitation wavelength 488 nm, argon laser) as a flow cytometer and a positive cell ratio was calculated. As a measurement software, System 2 version 3.0 (Beckman Coulter, Inc.) was used. The results of positive cell ratio are shown in Table 5.

Table 5

| | Positive cell ratio (%) | |
|---|---|---|
| | Control | Compound 16 |
| CD40 | 8.64 | 93.6 |
| CD80 | 0.5 | 45.7 |
| CD86 | 53.3 | 78.4 |

According to Table 5, increase in positive cell ratio was observed in all of CD40, CD80 and CD86 with, for example, Compound 16. That is, it was revealed that Compounds (I) enhance expression of CD40, CD80 and CD86 and possess evident immunostimulatory activity.

Test Example 3: IP-10 Production-inducing Effect

Enzyme-linked immunosorbent assay (ELISA) of IP-10 can be carried out in the following manner. However, the following method is an example and does not limit the method for carrying out the ELISA.

A test compound to give a final concentration of 50 nmol/L was added to the BGERKB5 cell suspension (3 mL) having a concentration of 1.1 x $10^6$ cells/mL obtained in Test Example 1, followed by incubation in a $CO_2$ incubator at 37°C for 48 hours (test compound group). As a control test, a control group without addition of a test compound was also prepared.

Measurement of the amount of IP-10 produced by the BGERKB5 cells was carried out using an immunoassay kit (BioSource International, Inc.). A calibration curve was prepared using the preparation attached to the kit, and the amount of IP-10 in the culture supernatant was measured.

As a result, the amount of IP-10 produced in the control group was below the detection limit, whereas the values obtained with Compound 16 and Compound 30 were 1441 pg/mL and 821 pg/mL, respectively.

That is, it was revealed that Compounds (I) induce production of IP-10 and evidently possess immunostimulatory activity.

Test Example 4: Suppression of Immunostimulatory Activity by Knockdown of TLR9 Using siRNA

A knockdown test of TLR9 using siRNA in BGERKB5 cells can be carried out in the following manner. However, the following method is an example and does not limit the method for carrying out the test.

A BGERKB5 cell suspension (0.5 mL) having a concentration of 4 x $10^5$ cells/mL obtained according to the method described in Test Example 1 was seeded into 6 wells of a 24-well plate (total cell number: 1.2 x $10^6$ cells), followed by incubation in a $CO_2$ incubator at 37˚C for 24 hours.

Then, siRNA against TLR9 (Ambion, Inc., pre-designed siRNA, ID#6055, Cat. 16704, Lot. 047951si) was introduced in an amount of 100 pmol per well using lipofectamine 2000 (Invitrogen Corp.).

Twenty-four hours after the siRNA introduction, the medium was replaced with a fresh one, followed by further incubation for 48 hours. The obtained cells were collected in a sterile tube and suspended in RPMI1640·ITPSG medium at a concentration of 1 x $10^6$ cells/mL (TLR9 kd group). "Mock group" containing no siRNA was prepared in the same manner as in the preparation of "TLR9 kd group".

An aqueous solution of a test compound prepared according to the method described in Test Example 1 (final concentration: 50 nmol/L) (test compound group) was added to each of the suspensions of the TLR9 kd group and the Mock group (3 mL). As a control test, a solvent control group without addition of a test compound was prepared for each group.

A 400-μL portion from each of the cell suspensions of the four groups (3 mL) was seeded in quadruplicate 100-μL aliquots into a 96-well plate, followed by incubation for 5 hours. Steady-Glo (Promega Corp.) (100 μL) was added and the luciferase activity (RLU) in each well was measured using Multilabel Counter ARVO (Perkin-Elmer Corp.). The results are shown in Table 6. In the table, Mock refers to the cells to which siRNA was not added, and TLR9 kd refers to the cells to which siRNA targeted against TLR9 was added.

Table 6

| | Luciferase activity (RLU) (mean ± standard error) | |
|---|---|---|
| | Solvent control | Compound 16 |
| Mock | 114.6 ± 7.5 | 7196.7 ± 88.8 |
| TLR9 kd | 112.5 ± 3.4 | 4918.3 ± 86.4 |

According to Table 6, the NF-κB activity of, for example, Compound 16 was reduced by the knockdown of TLR9. That is, it was revealed that the NF-κB activity of Compounds (I) is mediated by TLR9 and that the immunostimulatory activity of Compounds (I) is evidently mediated by TLR9.

Test Example 5: Confirmation of Variation in Expression of TLR-related Genes

The remaining portion (2.6 mL) of each of the cell suspensions of the four groups used in Test Example 4 was incubated in a 15-mL sterile tube for 5 hours. The cells were collected, and the expression levels of genes of glyceraldehyde-3-phosphoate dehydrogenase (GAPDH), luciferase (NF-κB promoter-derived) (NF-κB-LUC), interleukin 12B (IL12B) and CD80 antigen (CD80) were measured by the quantitative RT-PCR method using SYBR-Green [see Bio Techniques, Vol. 22, p. 130-131, p.134-138 (1997)].

That is, the cell suspension of each group remaining in the above test was subjected to the following procedure.

The cell suspension (1 x $10^6$ cells/mL) of each group (2.6 mL: total cell number 2.6 x $10^6$ cells) was centrifuged using himac CF702 centrifuge (Hitachi Koki Co., Ltd.) at 4˚C at 800 rpm for 10 minutes, and after the supernatant was removed using Pipetman, PBS (2 mL) was added. The obtained cell suspension was further centrifuged using himac CF702 centrifuge (Hitachi Koki Co., Ltd.) at 4˚C at 800 rpm for 10 minutes, and the supernatant was removed using Pipetman to obtain cells.

Total RNA was extracted from the obtained cells using RNeasy Mini Kit (registered trademark: Qiagen, Inc.) according to the manual attached to the kit. By using the obtained total RNA as a template, cDNA was synthesized in

the following manner using Super Script First-strand Synthesis System for RT-PCR (Invitrogen Corp.) according to the manual attached to the kit.

Ten mmol/L dNTPs mixture solution (1.0 μL) and 0.5 μg/μL oligo(dT)$_{12-18}$ primer (1.0 μL) were added to the total DNA (5 μg), and the mixture was diluted with an aqueous solution of diethyl pyrocarbonate (DEPC) to make a total volume of 10.0 μL. The obtained solution was heated at 65°C for 5 minutes, then rapidly cooled on ice, and allowed to stand for more than one minute for denaturation. Ten x RT buffer (2.0 μL), 25 mmol/L magnesium chloride (4.0 μL), 0.1 mol/L DTT (2.0 μL) and RNaseOUT (1.0 μL) were added to the obtained mRNA solution, and the mixture was made up to a total volume of 19.0 μL and then incubated at 42°C for 2 minutes. SuperScript II Reverse Transcriptase (Invitrogen Corp.) (1.0 μL, 50 U) was added thereto, and the mixture was subjected to reverse transcription reaction at 42°C for 50 minutes, followed by heating at 70°C for 15 minutes to inactivate the enzyme. RNaseH (1.0 μL) was added to the obtained mixture, and after the mixture was subjected to reaction at 37°C for 20 minutes, TE buffer (pH 8.0) (Ambion, Inc.) was added to make a total volume of 200 μL. The resulting solution was diluted 5-fold for use in real-time PCR.

To the cDNA prepared above (10.0 μL: corresponding to 50 ng of total RNA) were added sets of forward primers (FW) and reverse primers (RV) consisting of the following sequences (i.e. sets of SEQ ID NOS: 1 and 2, SEQ ID NOS: 3 and 4, SEQ ID NOS: 5 and 6, and SEQ ID NOS: 7 and 8: all produced by Profrigo s.r.l.) to give a final concentration of 300 nmol/L respectively.

Gene name: glyceraldehyde-3-phosphate dehydrogenase (GAPDH)

FW: 5'- ACCAGGGCTGCTTTTAACTCT -3' (SEQ ID NO: 1)

RV: 5'-TCATGAGTCCTTCCACGATAC-3' (SEQ ID NO: 2)

Gene name: luciferase (NF-κB promoter-derived) (NF-κB-LUC)

FW : 5'-CCTATGATTATGTCCGGTTATG-3' (SEQ ID NO: 3)

RV : 5' -GGATCTCTCTGATTTTTCTTGC-3' (SEQ ID NO: 4)

Gene name: interleukin 12B (IL12B)

FW : 5'- ATAAAGCAATTTAGGGCCACTTAC -3' (SEQ ID NO: 5)

RV : 5'- TGACAATTTCATGTCCTTAGCC -3' (SEQ ID NO: 6)

Gene name: CD80 antigen (CD80)

FW : 5'- TAGAAGGATAATTTGCTCAACCTC -3' (SEQ ID NO: 7)

RV : 5'-GCTACCTTCAGATCTTTTCAGC-3' (SEQ ID NO: 8)

To the obtained solution were added 10 x R-PCR buffer $Mg^{2+}$ free (2.0 μL: Takara Bio Inc.), 250 mmol/L $Mg^{2+}$ solution (0.2 μL), 10 mmol/L dNTPs (0.6 μL), ExTaq-PCR (0.2 μL: Takara Bio Inc.), SYBR Green I (1.0 μL: BMA Corp., 2500-fold dilution of the original solution) and sterile water to make a total volume of 20.0 μL. After the obtained solution was heated at 94°C for 5 minutes, reaction carried out for 45 cycles of 94°C for 30 seconds, 65°C for 30 seconds and 72°C for 30 seconds. The fluorescence intensity emitted from SYBR Green I intercalated in the amplification product was measured using ABI PRISM7700 (PE Applied Biosystems) and data analysis was conducted with the software

attached to the instrument, Sequence detector ver. 1.7a.

Variation in the expression level of mRNA of each gene when a test compound was added was expressed as the ratio between a sample of the test compound group and a sample of the solvent control group. That is, the cycle number at which the signal value measured with ABI PRISM7700 reached 500 was designated as Ct, and the difference in Ct value (ΔCt) between the test compound group and the solvent control group was calculated. The variation value was calculated as "relative expression level" according to the following equation, regarding a difference of one cycle as a 2-fold difference. The judgment as to whether the signal obtained by the above reaction is the desired amplified fragment was made by subjecting, after the completion of reaction, the resulting solution to agarose gel electrophoresis and observing the size of the main amplified fragment.

$$\text{Relative expression level in each test area} = 2^{Ct(C)-Ct(S)}$$

(calculated by regarding the amplification rate per cycle as 2-fold)
Ct(C): Ct value in solvent control group
Ct(S): Ct value in test compound group

After the mRNA expression level of each gene in the test compound group was corrected with GAPDH (glyceraldehyde-3-phosphate dehydrogenase) as an internal standard, that is, regarding GAPDH as 1, the ratio of "mRNA expression level of each gene in the test compound group" to "mRNA expression level of each gene in the solvent control group" was calculated (that is, "mRNA expression level of each gene in the solvent control group" was regarded as 1). The results of calculation when Compound 16 was used as a test compound are shown in Table 7.

Table 7

| Gene | siRNA | |
|---|---|---|
| | Mock | TLR9 kd |
| Glyceraldehyde-3-phosphate dehydrogenase (GAPDH) | 1.0 | 1.0 |
| Luciferase (NF-κB promoter-derived) (NF-κB-LUC) | 59.7 | 25.7 |
| Interleukin 12B (IL12B) | 1.4 | 0.6 |
| CD80 antigen (CD80) | 127 | 73.0 |

As shown in Table 7, the expression level of each of the NF-κB-LUC, IL12B and CD80 genes in BGERKB5 cells was enhanced by addition of Compound 16. The expression level of these genes enhanced by Compound 16 was reduced by knockdown of TLR9.

That is, it was revealed that Compounds (I) enhance expression of the NF-κB-LUC, IL12B and CD80 genes and this enhancement of transcription is mediated by TLR9.

As immunostimulants, OK432 (picibanil), PSK (krestin), lentinan, etc. are known and are clinically used as antitumor drugs.

Bacteria-derived DNA is also known to have immunostimulatory activity. CpG dinucleotide sequences in bacterial DNA differ from human Cpg dinucleotide sequences in that the 5-position of a cytosine base is not methylated. Further, in human DNA, the CpG dinucleotide sequence frequency is low. This unmethylated CpG motif is considered to be important for immunostimulatory activity. Synthetic oligo DNA comprising this motif (CpG-oligodeoxynucleotide) has a potent immunostimulatory activity, similarly to bacteria-derived DNA.

Helper T cells are divided into Th1 and Th2 cells having different cytokine-producing ability. Th1 cells regulate cellular immunity and they are involved in bacterial or viral infection, etc. and exhibit strong cytotoxicity (Th1 immunity). Th2 cells regulate humoral immunity and are involved in allergy, etc. (Th2 immunity). CpG-oligodeoxynucleotides induce strong Th1 immunity, exhibit anti-tumor activity and anti-bacterial or antiviral activity, and suppress allergy-promoting Th2 immunity. Medical uses of these CpG-oligodeoxynucleotides as anti-tumor agents, anti-infection agents, anti-allergic agents, etc. are known and their usefulness has been confirmed in animal models and clinical tests [Nature Reviews Drug Discovery, Vol. 1, p. 797 (2002); Nature Reviews Immunology, Vol. 4, p. 1 (2004); EP0468520; WO96/02555; WO98/18810; WO98/37919; WO9/40100; WO99/51259; WO99/56755, etc.].

For example, in a mouse model of cervical cancer, CpG-oligodeoxynucleotide showed anti-tumor effect and life-prolonging effect [Clinical Cancer Research, Vol. 9, p. 2693 (2003)]. Further, in a mouse model of rhabdomyosarcoma, the life-prolonging rate by surgical excision and chemotherapy (cyclophosphamide or topotecan) was raised by combination use of CpG-oligodeoxynucleotide [Clinical Cancer Research, Vol. 9, p. 3105 (2003)].

For example, in a mouse model of leishmaniasis, CpG-oligodeoxynucleotide was effective [The Journal of Immunology, Vol. 160, p. 3627 (1998); Proceedings of the National Academy of Sciences of the United States of America, Vol. 96, p. 6970 (1999)].

For example, in an OVA-sensitized mouse model of chronic asthma, CpG-oligodeoxynucleotide suppressed airway inflammation, airway hypersensitivity and airway remodeling [Journal of Allergy & Clinical Immunology, Vol. 110, p. 867 (2002)]. Further, in a rhesus monkey model of asthma sensitized by house dust mite allergen, CpG-oligodeoxynucleotide suppressed airway hypersensitivity and airway remodeling [American Journal of Respiratory and Critical Care Medicine, Vol. 170, p. 1153 (2004)].

For example, in a clinical test, CpG-oligodeoxynucleotide was effective against non-small cell lung cancer when used in combination with taxan and platinum medicine [American Society of Clinical Oncology: ASCO, Annual Meeting, Abstract, #7039 (2005)]. Further, in a clinical test, a conjugate of ragweed pollen antigen and CpG-oligodeoxynucleotide enhanced Th1 immunity and suppressed Th2 immunity, thereby improving the symptoms of allergic rhinitis [Journal of Allergy & Clinical Immunology, Vol. 113, p. 235 (2004)].

It has been revealed that CpG-oligodeoxynucleotides are recognized by Toll-like receptor 9 (TLR9), which is one of the Toll-like receptors [Nature, Vol. 408, p. 740 (2000)]. TLR9 is present in B cells, dendritic cells, etc. TLR9 transmits signals into cells via adapter molecules such as MyD88, activates transcriptional factors such as NF-κB controlling expression of cytokine genes, and induces production of cytokines. Further, TLR9 enhances expression of surface molecules such as CD40, CD80 and CD86, which are co-stimulatory molecules important for the activation of lymphocytes.

Chemokine IP-10 (interferon-gamma inducible protein 10 kDa) (CXCL10) is known to have potent angiogenesis-inhibiting effect and to show anti-tumor activity [Blood, Vol. 89, p. 2635 (1997); Journal of Experimental Medicine, Vol. 184, p. 981 (1996); Leukemia Lymphoma, Vol. 19, p. 267 (1995); and Journal of Experimental Medicine, Vol. 178, p. 1057 (1993)]. IP-10 also has natural killer cell-activating ability and T cell migratory activity.

From these facts and the results of the above Test Examples 1 to 5, Compounds (I) having TLR9 agonist activity and/or immunostimulatory activity are considered to be useful as TLR9 agonists, immunostimulants, anti-allergic agents, anti-tumor agents, anti-infection agents, and the like.

Compounds (I) or pharmaceutically acceptable salts thereof can be used as such or in various pharmaceutical forms according to the pharmacological activity and the purpose of administration. The pharmaceutical compositions of the present invention can be prepared by uniformly mixing an effective amount of Compound (I) or a pharmaceutically acceptable salt thereof, as an active ingredient, with a pharmaceutically acceptable carrier. The carrier can take a wide variety of forms according to the pharmaceutical preparation form desirable for administration. These pharmaceutical compositions are preferably in a unit dose form suitable for oral administration or non-oral administration in the form of ointment, injection, or the like.

Tablets can be prepared using excipients such as lactose, glucose, sucrose, mannitol and methyl cellulose, disintegrating agents such as starch, sodium alginate, calcium carboxymethyl cellulose and crystalline cellulose, lubricants such as magnesium stearate and talc, binders such as gelatin, polyvinyl alcohol, polyvinylpyrrolidone, hydroxypropyl cellulose and methyl cellulose, surfactants such as sucrose fatty acid ester and sorbitol fatty acid ester, and the like in a conventional manner. It is preferred that each tablet contains 1 to 300 mg of the active ingredient.

Granules can be prepared using excipients such as lactose and sucrose, disintegrating agents such as starch, binders such as gelatin, and the like in a conventional manner. Powders can be prepared using excipients such as lactose and mannitol, and the like in a conventional manner. Capsules can be prepared using gelatin, water, sucrose, gum arabic, sorbitol, glycerin, crystalline cellulose, magnesium stearate, talc, and the like in a conventional manner. It is preferred that each capsule contains 1 to 300 mg of the active ingredient.

Syrup can be prepared using sugars such as sucrose, water, ethanol, and the like in a conventional manner.

Ointment can be prepared using ointment bases such as vaseline, liquid paraffin, lanolin and macrogol, emulsifiers such as sodium lauryl lactate, benzalkonium chloride, sorbitan mono-fatty acid ester, sodium carboxymethyl cellulose and gum arabic, and the like in a conventional manner.

Injections can be prepared using solvents such as water, physiological saline, vegetable oils (e.g., olive oil and peanut oil), ethyl oleate and propylene glycol, solubilizing agents such as sodium benzoate, sodium salicylate and urethane, isotonicity agents such as sodium chloride and glucose, preservatives such as phenol, cresol, p-hydroxybenzoic acid ester and chlorobutanol, antioxidants such as ascorbic acid and sodium pyrosulfite, and the like in a conventional manner.

Compounds (I) or pharmaceutically acceptable salts thereof can be administered by oral administration, inhalation administration or non-oral administration as an ointment, injection, or the like. The effective dose and the administration schedule of Compound (I) or a pharmaceutically acceptable salt thereof vary depending on the mode of administration, the patient's age, body weight and symptoms, etc. However, it is generally preferred to administer Compound (I) or a pharmaceutically acceptable salt thereof in a dose of 0.0001 to 20 mg/kg 1 to 4 times a day.

The present invention is more specifically described below by referring to examples and reference examples.

These examples are not to be construed as limiting the scope of the present invention. The proton nuclear magnetic resonance spectra ($^1H$-NMR) in the examples and reference examples were measured at 300 MHz, 400 MHz or 500 MHz, and in some cases, exchangeable protons were not clearly observed according to the compound and measurement conditions. Multiplicity of signals is expressed in conventional terms, wherein "br" indicates an apparently broad signal.

The structures of synthetic intermediates used in the following examples and reference examples are shown in Tables 8-1 to 8-4. In the tables, Me represents methyl, Ac represents acetyl, Bn represents benzyl, and Ph represents phenyl.

Table 8-1

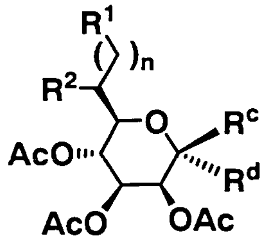

| Compd.No. | $R^c$ | $R^d$ | 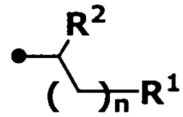 |
|---|---|---|---|
| A | H | OH | •-$CH_2OAc$ |
| B | H | O-PO$(OH)_2$ | •-$CH_2OAc$ |
| C | O-PO$(OPh)_2$ | H | •-$CH_2OAc$ |
| D | O-PO$(OH)_2$ | H | •--$CH_2OAc$ |
| E | H | OH | 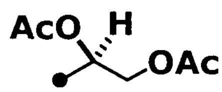 |
| Fa | H | O-PO$(OPh)_2$ | 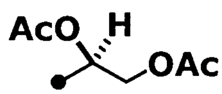 |
| Fb | O-PO$(OPh)_2$ | **H** | 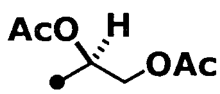 |
| Ga | H | O-PO$(OH)_2$ | 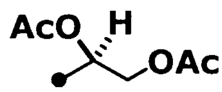 |
| Gb | O-PO$(OH)_2$ | H | 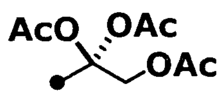 |
| H | H | OH | 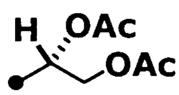 |
| Ia | H | O-PO$(OPh)_2$ | 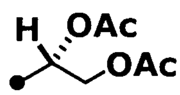 |
| Ib | O-PO$(OPh)_2$ | H | 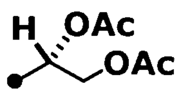 |

Table 8-2

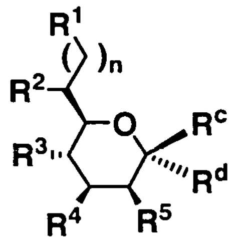

| Compd. No. | $R^c$ | $R^d$ | $R^3$,$R^4$,$R^5$ | 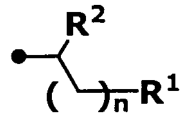 |
|---|---|---|---|---|
| Ja | H | O-PO(OH)$_2$ | OAc | 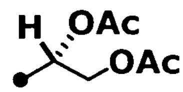 |
| Jb | O-PO(OH)$_2$ | H | OAc | 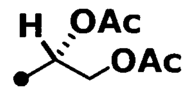 |
| K | H | OH | OMe | •-CH$_2$OMe |
| M | H | OMe | OH | •-CH$_2$OC(Ph)$_3$ |
| N | H | OMe | OBn | •-CH$_2$CH$_2$OH |
| O | H | OH | OAc | •-CH$_2$CH$_2$OAc |
| P | O-PO(OH)$_2$ | H | OAc | •-CH$_2$CH$_2$OAc |
| Q | H | OMe | OMe | •-CH$_2$CH$_2$OH |

Table 8-3

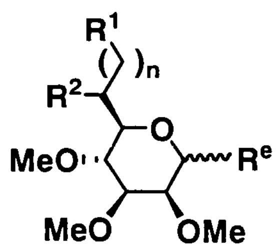

| Compd. No. | $R^e$ | 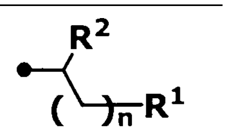 |
|---|---|---|
| L | O-PO(OH)$_2$ | •-CH$_2$OMe |
| R | O-PO(OH)$_2$ | •-CH$_2$CH$_2$OAc |

Table 8-4

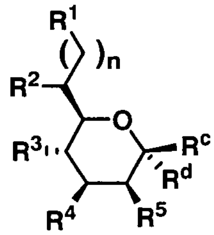

(continued)

| Compd. No. | $R^c$ | $R^d$ | $R^3$,$R^4$,$R^5$ | 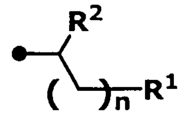 |
|---|---|---|---|---|
| S | $CH_2OH$ | H | OBn | 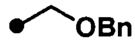 |
| T | $CH_2OH$ | H | OBn | 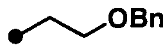 |
| U | OH | H | OAc | 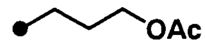 |

The processes for synthesizing the intermediates used for synthesis of Compounds (I) are described in Reference Examples 1 to 20 and 34 to 36.

Reference Example 1: Synthesis of 2,3,4,6-Tetra-O-acetyl-α-D-mannopyranose (Compound A)

[Step 1]

To an acetic anhydride solution (24 mL) of D-mannose (5.0 g, 28 mmol) was added trimethylsilyl chloride (0.80 mL, 7.0 mmol), and the mixture was stirred at 80°C for 2 hours and then concentrated under reduced pressure. The residue was purified by silica gel column chromatography (hexane/ethyl acetate = 2/1) to obtain a penta-O-acetyl form (5.5 g, yield 51%).
FAB-MS m/z: 391 $(M+H)^+$.

[Step 2]

To a DMF solution (50 mL) of the penta-O-acetyl form obtained in Step 1 (5.5 g, 14 mmol) was added hydrazine acetate (2.0 g, 22 mmol), followed by stirring at room temperature for one hour. To the reaction mixture was added water, and the mixture was extracted with ethyl acetate. The organic layer was washed with a saturated aqueous solution of sodium chloride, dried over anhydrous sodium sulfate, and then concentrated under reduced pressure. The residue was purified by silica gel column chromatography (hexane/ethyl acetate = 2/1) to obtain Compound A (3.5 g, yield 36%).
FAB-MS m/z: 349 $(M+H)^+$.

Reference Example 2: Synthesis of 2,3,4,6-Tetra-O-acetyl-α-D-mannopyranosyl Phosphate (Compound B)

[Step 1]

To a dichloromethane solution (20 mL) of Compound A (1.8 g, 5.2 mmol) and triazole (0.18 g, 2.6 mmol) was added bis(benzyloxy)(diisopropylamino)phosphine (1.8 g, 5.2 mmol), followed by stirring at room temperature for 12 hours. A nonane solution (5.5 mol/L, 1.5 mL) of t-butylhydroperoxide was poured into the reaction mixture, water was added thereto, and the mixture was extracted with ethyl acetate. The organic layer was washed successively with an aqueous solution of sodium thiosulfate and a saturated aqueous solution of sodium chloride, dried over anhydrous sodium sulfate, and then concentrated under reduced pressure. The residue was purified by silica gel column chromatography (hexane/ethyl acetate = 2/1) to obtain a dibenzylphosphate (2.2 g, yield 70%).
$^1$H-NMR (300 MHz, $CDCl_3$)δ(ppm): 7.36-7.27 (m, 10H), 5.61 (d, J = 6.6 Hz, 1H), 5.29 (d, J = 6.2 Hz, 2H), 5.22 (br s, 1H), 5.11-5.07 (m, 4H), 4.21-4.05 (m, 2H), 3.93 (d, J = 12.0 Hz, 1H), 2.04 (s, 3H), 2.03 (s, 3H), 2.00 (s, 3H), 1.99 (s, 3H).
FAB-MS m/z: 609 $(M+H)^+$.

[Step 2]

The dibenzylphosphate obtained in Step 1 (1.1 g, 1.8 mmol) was dissolved in a mixed solvent of methanol and triethylamine (20/1, 21 mL) and 10% palladium carbon (0.80 mL, 7.0 mmol) was added thereto, followed by stirring at room temperature for 2 days in an atmosphere of hydrogen. The catalyst was removed from the reaction mixture by filtration, and the filtrate was concentrated to obtain a triethylamine salt of Compound B (0.88 g, yield 78%).
$^1$H-NMR (300 MHz, $CDCl_3$)δ(ppm): 5.61 (s, J = 6.6 Hz, 1H), 5.30-5.22 (m, 2H), 4.47-4.37 (m, 2H), 4.38 (d, J = 10.3 Hz,

1H), 4.14 (d, J = 12.9 Hz, 1H), 3.15 (q, J = 7.2 Hz, 6H), 2.17 (s, 3H), 2.10 (s, 3H), 2.07 (s, 3H), 2.05 (s, 3H), 1.24 (t, J = 7.2 Hz, 9H).
FAB-MS m/z: 427 $(M-H)^-$.

Reference Example 3: Synthesis of Diphenyl(2,3,4,6-tetra-o-acetyl-β-D-mannopyranosyl) Phosphate (Compound C)

To a dichloromethane solution (20 mL) of Compound A (0.75 g, 2.2 mmol) was added DMAP (0.60 g, 4.9 mmol), and a dichloromethane solution (10 mL) of diphenyl chlorophosphate (1.0 mL, 4.9 mmol) was added dropwise thereto over a period of one hour at room temperature, followed by stirring at room temperature for one hour. To the reaction mixture was added water, and the mixture was extracted with ethyl acetate. The organic layer was washed with a saturated aqueous solution of sodium chloride and dried over sodium sulfate, and the solvent was distilled off under reduced pressure. The residue was purified by silica gel column chromatography (hexane/ethyl acetate = 2/1) to obtain Compound C (0.72 g, yield 56%).
$^1$H-NMR (300 MHz, $CDCl_3$)δ(ppm): 7.37-7.11 (m, 10H), 5.60 (d, J = 7.1 Hz, 1H), 5.48 (m, 1H), 5.26 (t, J = 9.6 Hz, 1H), 5.07 (dd, J = 9.6, 3.3 Hz, 1H), 4.27 (dd, J = 12.3, 5.5 Hz, 1H), 4.10 (m, 1H), 3.70 (m, 1H), 2.06 (s, 3H), 2.052 (s, 3H), 2.049 (s, 3H), 1.99 (s, 3H).
FAB-MS m/z: 581 $(M+H)^+$.

Reference Example 4: Synthesis of 2,3,4,6-Tetra-O-acetyl-β-D-mannopyranosyl Phosphate (Compound D)

Compound C (0.029 g, 0.05 mmol) was dissolved in a mixed solvent of ethyl acetate and ethanol (1/1, 5.0 mL) and platinum oxide (0.029 g) was added thereto, followed by stirring at room temperature for 2 days in an atmosphere of hydrogen. The catalyst was removed from the reaction mixture by filtration, and the filtrate was concentrated to obtain Compound D (0.017 g, yield 78%).
FAB-MS m/z: 427 $(M-H)^-$.

Reference Example 5: Synthesis of 2,3,4,6,7-Penta-O-acetyl-L-glycero-α-D-mannoheptopyranose (Compound E)

[Step 1]

From an acetic anhydride solution (3.1 mL, 3.3 mmol) of L-glycero-D-mannoheptopyranose (0.7 g, 3.3 mmol), a hexa-O-acetyl form (1.0 g, yield 65%) was obtained in a manner similar to Step 1 of Reference Example 1 using trimethylsilyl chloride (0.11 mL, 0.83 mmol).
FAB-MS m/z: 461 $(M-H)^-$.

[Step 2]

In a manner similar to Step 2 of Reference Example 1, Compound E (0.60 g, yield 38%) was obtained from the hexa-O-acetyl form obtained in Step 1 (1.5 g, 32 mmol) using hydrazine acetate (2.0 g, 22 mmol).
$^1$H-NMR (300 MHz, $CDCl_3$)δ(ppm): 5.9 (dd, J = 10.0, 3.5 Hz, 1H), 5.33-5.22 (m, 4H), 4.41 (dd, J = 11.4, 5.9 Hz, 1H), 4.25 (dd, J = 9.9, 1.9 Hz, 1H), 4.14 (dd, J = 11.4, 7.0 Hz, 1H), 2.18 (s, 3H), 2.18 (s, 3H), 2.15 (s, 3H), 2.07 (s, 3H), 2.03 (s, 3H).
FAB-MS m/z 421 $(M+H)^+$.

Reference Example 6: Synthesis of Diphenyl(2,3,4,6,7-penta-O-acetyl-L-glycero-α-D-mannoheptopyranosyl) Phosphate (Compound Fa) and Diphenyl(2,3,4,6,7-penta-O-acetyl-L-glycero-β-D-mannoheptopyranosyl) Phosphate

(Compound Fb)

In a manner similar to Reference Example 3, Compound Fa (0.18 g, yield 28%) and Compound Fb (0.21 g, yield 32%) were obtained from Compound E (0.42 g, 1.0 mmol) using DMAP (0.20 g, 1.6 mmol) and diphenyl chlorophosphate (0.33 mL, 1.6 mmol).

Compound Fa:

$^1$H-NMR (300 MHz, $CDCl_3$)δ(ppm): 7.40-7.35 (m, 4H), 7.26-7.20 (m, 6H), 5.88 (d, J = 6.6 Hz, 1H), 5.37-5.28 (m, 4H), 4.26 (d, J = 8.4 Hz, 1H), 4.15-4.13 (m, 2H), 2.18 (s, 3H), 2.12 (s, 3H), 2.03 (s, 3H), 2.00 (s, 3H), 1.92 (s, 3H).
FAB-MS m/z 653 $(M+H)^+$.

Compound Fb:

$^1$H-NMR (300 MHz, $CDCl_3$)δ(ppm): 7.39-7.13 (m, 10H), 5.56 (d, J = 7.0 Hz, 1H), 5.51 (d, J = 3.3 Hz, 1H), 5.35-5.26 (m, 2H), 5.05 (dd, J = 9.9, 3.3 Hz, 1H), 4.26 (dd, J = 11.3, 5.1 Hz, 1H), 4.15-4.06 (m, 2H), 2.18 (s, 3H), 2.06 (s, 3H), 2.052 (s, 3H), 2.049 (s, 3H), 1.99 (s, 3H).
FAB-MS m/z 653 $(M+H)^+$.

Reference Example 7: Synthesis of 2,3,4,6,7-Penta-O-acetyl-L-glycero-α-D-mannoheptopyranosyl Diphosphate

(Compound Ga)

In a manner similar to Reference Example 4, Compound Ga (0.16 g, quantitative) was obtained from Compound Fa (0.18 g, 0.28 mmol) using platinum oxide (0.090 g).
FAB-MS m/z 499 $(M-H)^-$.

Reference Example 8: Synthesis of 2,3,4,6,7-Penta-O-acetyl-L-glycero-β-D-mannoheptopyranosyl Diphosphate

(Compound Gb)

In a manner similar to Reference Example 4, Compound Gb (0.18 g, quantitative) was obtained from Compound Fb (0.21 g, 0.32 mmol) using platinum oxide (0.029 g).
FAB-MS m/z 499 $(M-H)^-$.

Reference Example 9: Synthesis of 2,3,4,6,7-Penta-O-acetyl-D-glycero-α-D-mannoheptopyranose (Compound H)

[Step 1]

From an acetic anhydride solution (0.44 mL, 0.27 mmol) of D-glycero-D-mannoheptopyranose (0.1 g, 0.47 mmol), a hexa-O-acetyl form (0.17 g, yield 78%) was obtained in a manner similar to Step 1 of Reference Example 1 using triethylsilane (0.016 mL, 0.12 mmol).
FAB-MS m/z 461 $(M-H)^-$.

[Step 2]

In a manner similar to Step 2 of Reference Example 1, Compound H (0.086 g, yield 54%) was obtained from the hexa-O-acetyl form obtained in Step 1 (0.17 g, 0.38 mmol) using hydrazine acetate (0.22 g, 2.4 mmol).
FAB-MS m/z 421 $(M+H)^+$.

Reference Example 10: Synthesis of Diphenyl(2,3,4,6,7-penta-O-acetyl-D-glycero-α-D-mannoheptopyranosyl)

Phosphate (Compound Ia) and Diphenyl(2,3,4,6,7-penta-O-acetyl-D-glycero-β-D-mannoheptopyranosyl) Phosphate

(Compound Ib)

In a manner similar to Reference Example 3, Compound Ia (0.15 g, 23%), Compound Ib (0.12 g, 18%) and a mixture of Compound Ia and Compound Ib (0.30 g, 46%) were obtained from Compound H (0.42 g, 1.0 mmol) using DMAP (0.22 g, 1.5 mmol) and diphenyl chlorophosphate (0.31 mL, 1.5 mmol). Compound Ia:
$^1$H-NMR (500 MHz, $CDCl_3$)δ(ppm): 7.39-7.30 (m, 4H), 7.26-7.18 (m, 6H), 5.85 (dd, J = 6.5, 2.1 Hz, 1H), 5.38 (dd, J = 9.1, 3.2 Hz, 1H), 5.35 (dd, J = 9.7, 9.1 Hz, 1H), 5.29 (dd, J = 3.2, 2.1 Hz, 1H), 5.15 (ddd, J = 7.6, 3.8, 2.5 Hz, 1H), 4.37 (dd, J = 12.0, 3.8 Hz, 1H), 4.24 (dd, J = 9.7, 2.5 Hz, 1H), 4.22 (dd, J = 12.0, 7.6 Hz, 1H), 2.14 (s, 3H), 2.10 (s, 3H), 2.02 (s, 3H), 2.01 (s, 3H), 1.83 (s, 3H). $^{31}$P-NMR (202.5 MHz, $CDCl_3$)δ(ppm): -13.49 (br s).
FAB-MS m/z 653 $(M+H)^+$.

Compound Ib:

$^1$H-NMR (500 MHz, $CDCl_3$)δ(ppm): 7.47-7.30 (m, 4H), 7.28-7.26 (m, 2H), 7.23-7.17 (m, 4H), 5.56 (dd, J = 7.4, 1.4 Hz, 1H), 5.44 (dd, J = 3.3, 1.7 Hz, 1H), 5.36 (m, 1H), 5.25 (dd, J = 8.6, 8.4 Hz, 1H), 5.07 (dd, J = 8.6, 3.3 Hz, 1H), 4.38 (dd, J = 12.2, 3.3 Hz, 1H), 4.19 (dd, J = 12.2, 6.5 Hz, 1H), 3.87 (dd, J = 8.6, 4.6 Hz, 1H), 2.10 (s, 3H), 2.08 (s, 3H),

2.06 (s, 3H), 2.05 (s, 3H), 1.99 (s, 3H). $^{31}$P-NMR (202.5 MHz, $CDCl_3$)δ(ppm) : -13.27 (br s).
FAB-MS m/z 653 $(M+H)^+$.

Reference Example 11: Synthesis of 2,3,4,6,7-Penta-O-acetyl-D-glycero-α-D-mannoheptopyranosyl Phosphate

(Compound Ja)

To an ethanol solution (2.0 mL) of Compound Ia (0.21 g, 0.32 mmol) was added platinum oxide (0.10 g), followed by stirring at room temperature for 5 days in an atmosphere of hydrogen. The catalyst was removed from the reaction mixture by filtration, and the filtrate was concentrated to obtain Compound Ja (0.011 g, yield 45%). FAB-MS m/z 499 $(M-H)^-$.

Reference Example 1.2: Synthesis of 2,3,4,6,7-Penta-O-acetyl-D-glycero-β-D-mannoheptopyranosyl Phosphate

(Compound Jb)

To an ethanol solution (1.0 mL) of Compound Ib (0.21 g, 0.32 mmol) was added platinum oxide (0.100 g), followed by stirring at room temperature for 5 days in an atmosphere of hydrogen. The catalyst was removed from the reaction mixture by filtration, and the filtrate was concentrated to obtain Compound Jb (0.037 g, yield 73%). FAB-MS m/z 499 $(M-H)^-$.

Reference Example 13: Synthesis of 2,3,4,6-Tetra-O-methyl-α-D-mannopyranose (Compound K)

[Step 1]

D-Mannose (1.8 g, 10 mmol) was dissolved in a mixed solvent of DMSO and water (20/1, 10 mL). To the resulting solution were added sodium hydroxide (4.0 g, 100 mmol) and methyl iodide (3.0 mL, 46 mmol) at room temperature three times (once per day), and the mixture was stirred for 3 days. The reaction mixture was poured into ice water, followed by extraction with chloroform. The organic layer was washed successively with dilute hydrochloric acid and a saturated aqueous solution of sodium chloride, dried over sodium sulfate, and then concentrated under reduced pressure. The residue was purified by silica gel column chromatography (hexane/ethyl acetate = 3/1) to obtain a penta-O-methyl form (1.3 g, yield 52%) as a mixture of anomers (α/β = 2.5/1).
FAB-MS m/z: 273 $(M+Na)^+$.

[Step 2]

To an acetic anhydride solution (20 mL) of the penta-O-methyl form obtained in Step 1 (1.3 g, 5.2 mmol) was added concentrated sulfuric acid (0.050 mL) at 0˚C, and the mixture was stirred at the same temperature for 5 hours. The reaction mixture was poured into a saturated aqueous solution of sodium hydrogencarbonate, followed by extraction with chloroform. The organic layer was washed with a saturated aqueous solution of sodium chloride, dried over sodium sulfate, and then concentrated under reduced pressure to obtain a crude acetyl form (1.0 g).
FAB-MS m/z: 301 $(M+Na)^+$.

[Step 3]

The crude acetyl form obtained in Step 2 (1.0 g, 4.0 mmol) was dissolved in a mixed solution of methanol, 30% aqueous ammonia and water (3/1/1, 20 mL), and the solution was stirred for 30 minutes. The reaction mixture was poured into ice water, followed by extraction with chloroform. The organic layer was washed successively with a saturated aqueous solution of ammonium chloride and a saturated aqueous solution of sodium chloride, dried over sodium sulfate, and then concentrated under reduced pressure to obtain Compound K (0.88 g, yield 72%).
$^1$H-NMR (300 MHz, $CDCl_3$)δ(ppm): 5.33 (br s, 1H), 3.89 (m, 1H), 3.65-3.49 (m, 13H), 3.39 (s, 3H), 3.38 (m, 1H).
FAB-MS m/z: 259 $(M+Na)^+$.

Reference Example 14: Synthesis of 2,3,4,6-Tetra-O-methyl-D-mannopyranosyl Diphosphate (Compound L)

[Step 1]

To a dichloromethane solution (10 mL) of Compound K (0.033 g, 0.25 mmol) was added DMAP (0.033 g, 0.30

mmol), and a dichloromethane solution (7.0 mL) of diphenyl chlorophosphate (0.060 mL, 0.30 mmol) was added dropwise thereto over a period of one hour at room temperature, followed by stirring at the same temperature for one hour. To the reaction mixture was added water, and the mixture was extracted with ethyl acetate. The organic layer was washed with a saturated aqueous solution of sodium chloride, dried over sodium sulfate, and then concentrated under reduced pressure to obtain a crude phosphate (0.040 g, yield 60%) as a mixture of anomers (α/β = 1/1).

[Step 2]

The crude phosphate obtained in Step 1 (0.040 g, 0.015 mmol) was dissolved in a mixed solvent of ethyl acetate and methanol (1/1, 5.0 mL) and platinum oxide (0.040 g) was added thereto, followed by stirring at room temperature for one day in an atmosphere of hydrogen. The catalyst was removed from the reaction mixture by filtration, and the filtrate was concentrated to obtain Compound L (0.013 g, yield 47%) as a mixture of anomers
(α/β = 2/1).
FAB-MS m/z 315 $(M-H)^-$.

Reference Example 15: Synthesis of 1-O-Methyl-6-O-trityl-D-mannopyranose (Compound M)

To a pyridine solution (10 mL) of methylmannose (3.9 g, 20 mmol) was added trityl chloride (5.6 g, 20 mmol), and the mixture was stirred at 40˚C for 4 hours. The reaction mixture was poured into ice water, followed by extraction with chloroform. The organic layer was washed with a saturated aqueous solution of sodium chloride, dried over sodium sulfate, and then concentrated under reduced pressure. The residue was purified by silica gel column chromatography (hexane/ethyl acetate = 1/1) to obtain Compound M (7.1 g, yield 81%).
$^1$H-NMR (300 MHz, $CDCl_3$)δ(ppm): 7.47-7.43 (m, 6H), 7.34-7.21 (m, 9H), 4.71 (d, J = 0.3 Hz, 1H), 3.90 (dd, J = 3.1, 2.5 Hz, 1H), 3.77-3.66 (m, 2H), 3.44-3.41 (m, 3H), 3.37 (s, 3H).
FAB-MS m/z 435 $(M-H)^-$.

Reference Example 16: Synthesis of 1-O-Methyl-2,3,4-tri-O-benzyl-6-deoxy-D-mannoheptopyranose (Compound N)

[Step 1]

To a DMF solution (10 mL) of sodium hydride (2.0 g, 60%, 50 mmol) were added Compound M (7.1 g, 16 mmol) and benzyl bromide (5.7 mL, 48 mmol) under ice-cooling, and after the temperature was raised to room temperature, the mixture was stirred at the same temperature for 12 hours. The reaction mixture was poured into ice water, followed by extraction with diethyl ether. The organic layer was washed with a saturated aqueous solution of sodium chloride, dried over sodium sulfate, and then concentrated under reduced pressure. The residue was purified by silica gel column chromatography (hexane/ethyl acetate = 9/1) to obtain a tri-O-benzyl form (7.2 g, yield 60%).
$^1$H-NMR (300 MHz, $CDCl_3$)δ(ppm): 7.50-7.17 (m, 30H), 4.71-4.56 (m, 6H), 4.26 (d, J = 10.4 Hz, 1H), 4.01 (t, J = 9.4 Hz, 1H), 3.86 (dd, J = 9.4, 3.1 Hz, 1H), 3.82 (dd, J = 3.1, 1.8 Hz, 1H), 3.78 (m, 1H), 3.53 (dd, J = 9.7, 1.7 Hz, 1H), 3.39 (s, 3H), 3.26 (dd, J = 9.7, 5.4 Hz, 1H).
FAB-MS m/z 705 $(M-H)^-$.

[Step 2]

The tri-O-benzyl form obtained in Step 1 (35 g, 50 mmol) was dissolved in a mixed solvent of 1,4-dioxane and methanol (1/1, 200 mL), and trifluoroacetic acid (20 mL) was added thereto under ice-cooling, followed by stirring at room temperature for 12 hours. To the reaction mixture was added potassium carbonate, and the precipitated salt was removed from the mixture by filtration. The filtrate was concentrated and the residue was purified by silica gel column chromatography (hexane/ethyl acetate = 4/1) to obtain a 6-hydroxy form (15.2 g, yield 71%).
$^1$H-NMR (300 MHz, $CDCl_3$)δ(ppm): 7.38-7.29 (m, 15H), 4.95(d, J = 11.1 Hz, 1H), 4.79 (d, J = 11.8 Hz, 1H), 4.70 (d, J = 1.8 Hz, 1H), 4.69 (d, J = 11.8 Hz, 1H), 4.66 (d, J = 11.1 Hz, 1H), 4.64 (s, 2H), 4.09-3.75 (m, 5H), 3.65 (m, 1H), 3.31 (s, 3H) .
FAB-MS m/z 463 $(M-H)^-$.

[Step 3]

To a THF solution (135 mL) of oxalyl chloride (45 mL, 520 mmol) was added a THF solution (86 mL) of dimethyl sulfide (41 mL, 540 mmol) at -78˚C, followed by stirring at the same temperature for one hour. A THF solution (120 mL) of the 6-hydroxy form obtained in Step 2 (120 g, 260 mmol) was slowly added dropwise thereto at -78˚C, followed by stirring at the same temperature for one hour. To the reaction mixture was added triethylamine (150 mL, 1.1 mol), and

after the temperature was raised to room temperature, the mixture was filtered through Celite. The filtrate was concentrated under reduced pressure to obtain a crude aldehyde.

[Step 4]

Methyltriphenylphosphonium bromide (570 g, 1.6 mol) was suspended in THF (1.5 L), and butyllithium (12 mL, 2.6 mol/L hexane solution) was added dropwise thereto under ice-cooling, followed by stirring at the same temperature for 30 minutes. A THF suspension (500 mL) of the crude aldehyde obtained in Step 3 was poured into the reaction mixture, followed by stirring at room temperature for one hour. To the reaction mixture was added water, followed by extraction with diethyl ether. The organic layer was washed successively with a saturated aqueous solution of sodium hydrogencarbonate and water, dried over sodium sulfate, and then concentrated under reduced pressure.
The residue was purified by silica gel column chromatography (hexane/ethyl acetate = 9/1) to obtain a vinyl form (48 g, yield 39%).
$^1$H-NMR (400 MHz, $CDCl_3$)δ(ppm): 7.52-7.27 (m, 15H), 6.04 (ddd, J = 17.2, 10.5, 6.6 Hz, 1H), 5.47 (ddd, J = 17.2, 1.7, 1.2 Hz, 1H), 5.29 (ddd, J = 10.5, 1.7, 1.0 Hz, 1H), 4.85 (d, J = 10.8 Hz, 1H), 4.79 (d, J = 12.5 Hz, 1H), 4.74 (d, J = 1.9 Hz, 1H), 4.72 (d, J = 12.5 Hz, 1H), 4.69-4.62 (m, 3H), 4.04 (m, 1H), 3.89 (dd, J = 9.4, 3.1 Hz, 1H), 3.80 (dd, J = 3.1, 1.9 Hz, 1H), 3.76 (t, J = 9.1 Hz, 1H), 3.32 (s, 3H).
FAB-MS m/z 459 $(M-H)^-$.

[Step 5]

To a THF solution (100 mL) of the vinyl form obtained in Step 4 (3.4 g, 7.4 mmol) was dropwise added a THF solution (11 mL, 1.0 mol/L) of borane-THF complex at -20˚C, and after the temperature was raised to 0˚C, the mixture was stirred at the same temperature for one hour. To the reaction mixture were added a saturated aqueous solution of sodium hydrogencarbonate (11 mL) and aqueous hydrogen peroxide (1.1 mL, 30%). After stirring at room temperature for 2.5 hours, water was added to the mixture, followed by extraction with ethyl acetate. The organic layer was washed with a saturated aqueous solution of sodium chloride, dried over sodium sulfate, and then concentrated under reduced pressure. The residue was purified by silica gel column chromatography (hexane/ethyl acetate = 2/1) to obtain Compound N (2.3 g, yield 66%).
$^1$H-NMR (300 MHz, $CDCl_3$)δ(ppm): 7.38-7.67 (m, 15H), 4.96 (d, J = 10.9 Hz, 1H), 4.78 (d, J = 12.3 Hz, 1H), 4.70 (d, J = 12.3 Hz, 1H), 4.66 (d, J = 1.9 Hz, 1H), 4.63 (d, J = 10.9 Hz, 1H), 4.61 (s, 1H), 3.87-3.74 (m, 6H), 3.31 (s, 3H), 2.12 (m, 1H), 1.85 (m, 1H).
FAB-MS m/z: 501 $(M+Na)^+$, 447 $(M-MeO)^+$.

Reference Example 17: Synthesis of 2,3,4,7-Tetra-O-acetyl-6-dehydro-D-mannoheptopyranose (Compound O)

[Step 1]

To an acetic anhydride solution (20 mL) of Compound N (0.84 g, 1.8 mmol) was dropwise added sulfuric acid (20 mL) at 0˚C, and the mixture was stirred at the same temperature for 30 minutes. The reaction mixture was poured into a saturated aqueous solution of sodium hydrogencarbonate, followed by extraction with chloroform. The organic layer was washed with a saturated aqueous solution of sodium chloride, dried over sodium sulfate, and then concentrated under reduced pressure. The residue (0.85 g) was dissolved in ethyl acetate (20 mL) and 10% palladium carbon (0.2 g) was added thereto, followed by stirring at room temperature for one day in an atmosphere of hydrogen. The catalyst was removed from the reaction mixture by filtration, and the filtrate was concentrated to obtain a crude 2,3,4-triol form (0.88 g).
FAB-MS m/z: 277 $(M-H)^-$.

[Step 2]

To a pyridine solution (6.0 mL) of the crude 2,3,4-triol form obtained in Step 1 (0.88 g) was dropwise added acetic anhydride (5.0 mL), and the mixture was stirred at 0˚C for one hour and then concentrated. The residue was dissolved in DMF (10 mL) and hydrazine acetate (0.19 g, 2.0 mmol) was added thereto, followed by reaction at room temperature for 2 hours. The reaction mixture was poured into cold water, followed by extraction with chloroform. The organic layer was washed with a saturated aqueous solution of sodium chloride, dried over sodium sulfate, and then concentrated under reduced pressure. The residue was purified by silica gel column chromatography (hexane/ethyl acetate = 2/1) to obtain Compound O (0.24 g, yield 38%).
$^1$H-NMR (300 MHz, $CDCl_3$)δ(ppm): 5.38 (dd, J = 10.1, 3.5 Hz, 1H), 5.27 (dd, J = 3.5, 1.7 Hz, 1H), 5.15-5.08 (m, 2H),

4.31-4.03 (m, 3H), 2.47 (s, 3H), 2.06 (s, 6H), 1.99 (s, 3H), 1.89-1.75 (m, 2H).
FAB-MS m/z: 385 $(M+Na)^+$.

Reference Example 18: Synthesis of 2,3,4,7-Tetra-O-acetyl-6-deoxy-D-mannoheptopyranosyl Phosphate (Compound P)

[Step 1]

From Compound O (0.24 g, 0.74 mmol), a phosphate (0.054 g, yield 22%) was obtained in a manner similar to Reference Example 3 using DMAP (0.16 g, 1.3 mmol) and diphenyl chlorophosphate (0.23 mL, 1.1 mmol).
$^1$H-NMR (300 MHz, $CDCl_3$)δ(ppm): 7.32-7.04 (m, 10H), 5.51 (d, J = 6.2 Hz, 1H), 5.41 (br s, 1H), 5.07 (dd, J = 10.6, 9.6 Hz, 1H), 4.99 (dd, J = 9.6, 2.9 Hz, 1H), 4.09-4.02 (m, J = 12.3, 5.5 Hz, 2H), 3.60 (m, 1H), 2.10 (s, 3H), 2.01 (s, 6H), 1.97 (s, 3H), 1.84-1.78 (m, 2H).
FAB-MS m/z 617 $(M+Na)^+$.

[Step 2]

In a manner similar to Reference Example 4, Compound P (0.035 g, yield 88%) was obtained from the phosphate (0.054 g, 0.091 mmol) obtained in Step 1 using platinum oxide (0.020 g).
FAB-MS m/z 441 $(M-H)^-$.

Reference Example 19: Synthesis of 1,2,3,4-Tetra-O-methyl-6-deoxy-D-mannoheptopyranose (Compound Q)

[Step 1]

Compound M (3.9 g, 8.9 mmol) was dissolved in a mixed solvent of DMSO and water (20/1, 10 mL), and sodium hydroxide (7.2 g, 180 mmol) and methyl iodide (3.0 mL, 46 mmol) were added thereto, followed by stirring at room temperature for 30 minutes. The reaction mixture was poured into ice water, followed by extraction with chloroform. The organic layer was washed successively with dilute hydrochloric acid and a saturated aqueous solution of sodium chloride, dried over anhydrous sodium sulfate, and then concentrated under reduced pressure to obtain a crude tetra-O-methyl form (3.8 g, yield 89%).
$^1$H-NMR (300 MHz, $CDCl_3$)δ(ppm): 7.51-7.49 (m, 6H), 7.30-7.21 (m, 9H), 4.86 (d, J = 0.7 Hz, 1H), 3.59-3.37 (m, 5H), 3.52 (s, 3H), 3.47 (s, 3H), 3.44 (s, 3H), 3.18 (dd, J = 9.9, 5.1 Hz, 1H).
FAB-MS m/z: 501 $(M+Na)^+$.

[Step 2]

From the crude tetra-O-methyl form (3.8 g, 0.0093 mmol) obtained in Step 1, a 6-hydroxy form (1.1 g, yield 54%) was obtained in a manner similar to Step 2 of Reference Example 16 using trifluoroacetic acid (18 mL).
$^1$H-NMR (300 MHz, $CDCl_3$)δ(ppm): 4.77 (d, J = 1.6 Hz, 1H), 3.84 (dd, J = 11.7, 2.6 Hz, 1H), 3.75 (dd, J = 11.7, 4.2 Hz, 1H), 3.58 (m, 1H), 3.57 (s, 3H), 3.54-3.41 (m, 3H), 3.494 (s, 3H), 3.492 (s, 3H), 3.36 (s, 3H).
FAB-MS m/z 259 $(M+Na)^+$.

[Step 3]

From the 6-hydroxy form (1.5 g, 6.2 mmol) obtained in Step 2, a vinyl form (0.43 g, yield 30%) was obtained in a manner similar to Steps 3 and 4 of Reference Example 16 using oxalyl chloride (1.1 mL, 12 mmol), dimethyl sulfide (1.0 mL, 13 mmol), triethylamine (3.7 mL, 26 mmol), methyltriphenylphosphonium bromide (19 g, 53 mmol) and n-butyllithium (20 mL, 2.4 mol/L in hexane).
$^1$H-NMR (300 MHz, $CDCl_3$)δ(ppm): 5.96 (ddd, J = 17.2, 10.6, 6.5 Hz, 1H), 5.41 (d, J = 17.2 Hz, 1H), 5.25 (d, J = 10.6 Hz, 1H), 4.77 (d, J = 1.7 Hz, 1H), 3.87 (dd, J = 9.5, 6.4 Hz, 1H), 3.57 (dd, J = 3.2, 1.8 Hz, 1H), 3.50 (m, 1H), 3.498 (s, 3H), 3.496 (s, 3H), 3.490 (s, 3H), 3.36 (s, 3H), 3.25 (t, J = 9.5 Hz, 1H).

[Step 4]

In a manner similar to Step 5 of Reference Example 16, Compound Q (0.22 g, yield 24%) was obtained from the vinyl form obtained in Step 3 (3.4 g, 7.4 mmol) using a THF solution (5.3 mL, 1.0 mol/L) of borane-THF complex, a saturated aqueous solution of sodium hydrogencarbonate (5.3 mL) and aqueous hydrogen peroxide (2.0 mL, 30%).

$^1$H-NMR (300 MHz, $CDCl_3$)δ(ppm): 4.73 (d, J = 1.7 Hz, 1H), 3.81 (t, J = 5.8 Hz, 1H), 3.63 (dd, J = 9.4, 6.8 Hz, 1H), 3.57 (dd, J = 3.4, 1.7 Hz, 1H), 3.54 (s, 3H), 3.50 (s, 3H), 3.48 (s, 3H), 3.47 (dd, J = 6.8, 3.4 Hz, 1H), 3.37 (s, 3H), 3.24 (dd, J = 9.4, 9.3 Hz, 1H), 2.10 (m, 1H), 1.81 (m, 1H). FAB-MS m/z 251 $(M+H)^+$.

Reference Example 20: Synthesis of 2,3,4-Tri-O-methyl-6-deoxy-D-mannoheptopyranose Phosphate (Compound R)

[Step 1]

To an acetic anhydride solution (4.0 mL) of Compound Q (0.22 g, 0.88 mmol) was dropwise added sulfuric acid (0.020 mL) at 0˚C, and the mixture was stirred at room temperature for 3.5 hours. The reaction mixture was poured into a saturated aqueous solution of sodium hydrogencarbonate, followed by extraction with ethyl acetate. The organic layer was washed with a saturated aqueous solution of sodium chloride, dried over anhydrous sodium sulfate, and then concentrated under reduced pressure to obtain a crude diacetyl form (0.10 g).
FAB-MS m/z 343 $(M+Na)^+$.

[Step 2]

From the crude diacetyl form obtained in Step 1 (1.5 g, 14 mmol), a 1-hydroxy form (0.039 g, yield 16%) was obtained in a manner similar to Step 2 of Reference Example 1 using hydrazine acetate (0.10 g, 1.1 mmol).
$^1$H-NMR (300 MHz, $CDCl_3$)δ(ppm): 5.24 (br s, 1H), 4.25 (m, 1H), 4.17 (m, 1H), 3.72 (td, J = 9.9, 2.4 Hz, 1H), 3.60 (dd, J = 3.3, 1.7 Hz, 1H), 3.539 (s, 3H), 3.535 (m ,1H), 3.49 (s, 6H), 3.18 (dd, J = 9.9, 9.3 Hz, 1H), 2.15 (m, 1H), 2.04 (s, 3H), 1.78 (m, 1H).
FAB-MS m/z 301 $(M+Na)^+$.

[Step 3]

To a dichloromethane solution (1.0 mL) of the 1-hydroxy form obtained in Step 2 (0.039 g, 0.14 mmol) was added DMAP (0.021 g, 0.17 mmol), and a dichloromethane solution (1.0 mL) of diphenyl chlorophosphate (0.35 mL, 0.17 mmol) was added dropwise thereto over a period of one hour at room temperature, followed by stirring at the same temperature for one hour. To the reaction mixture was added water, and the mixture was extracted with ethyl acetate. The organic layer was washed with a saturated aqueous solution of sodium chloride, dried over sodium sulfate, and then concentrated under reduced pressure.
The residue was dissolved in ethanol (1.0 mL) and platinum oxide (0.035 g) was added thereto, followed by stirring at room temperature for 5 days in an atmosphere of hydrogen. The catalyst was removed from the reaction mixture by filtration, and the filtrate was concentrated to obtain Compound R (0.027 g, yield 56%) as a mixture of anomers
(α/β = 1/1).
FAB-MS m/z 315$(M-H)^-$.

Reference Example 21: Synthesis of Adenosine=5'-(2,3,4,6-tetra-O-acetyl-α-D-mannopyranosyl)diphosphate (Compound 5)

Compound B (0.40 g, 0.63 mmol) and 4'-morpholine-N,N'-dicyclohexylcarboxamidinium salt of adenosine=5'-phosphomorpholidate (0.45 g, 0.63 mmol) were dissolved in pyridine (3.0 mL), and the solution was concentrated under reduced pressure. To the residue were added pyridine (5.0 mL), triazole (0.084 g, 1.2 mmol) and molecular sieves 4A (1.0 g), followed by stirring at room temperature for one hour. After the molecular sieves were removed from the reaction mixture by filtration, the filtrate was concentrated under reduced pressure. The residue was purified by DEAE Sephadex A-25 column chromatography (Amersham Biosciences, eluted with 0.2-0.3 mmol/L ammonium acetate solution adjusted to pH 5.0 with acetic acid) to obtain an ammonium salt of Compound 5 (0.97 g, quantitative).
$^1$H-NMR (300 MHz, $D_2O$)δ(ppm): 8.56 (s, 1H), 8.26 (s, 1H), 6.11 (d, J = 5.9 Hz, 1H), 5.55 (d, $J_{HP}$ = 7.7 Hz, 1H), 5.35 (br s, 1H), 5.28 (d, J = 9.5 Hz, 1H), 5.17 (t, J = 9.5 Hz, 1H), 4.75-4.66 (m, 2H), 4.47 (m, 1H), 4.36-4.22 (m, 4H), 3.98 (d, J = 12.8 Hz, 1H), 2.13 (s, 3H), 2.03 (s, 3H), 1.95 (s, 3H), 1.91 (s, 3H).
FAB-MS m/z 756 $(M-H)^-$.

Reference Example 22: Synthesis of Adenosine=5'-(α-D-mannopyranosyl)diphosphate (Compound 6)

Compound 5 (0.070 g, 0.092 mmol) was dissolved in a mixed solvent of methanol, water and triethylamine (7/3/1, 50 mL), and the solution was stirred at room temperature for 4 hours. The reaction mixture was concentrated, and the residue was freeze-dried to obtain Compound 6 (0.10 g, quantitative).

$^1$H-NMR (300 MHz, $D_2O$)δ(ppm): 8.48 (s, 1H), 8.19(s, 1H), 6.07 (d, J = 5.9 Hz, 2H), 5.26 (dd, $J_{HH}$ = 1.8 Hz, $J_{HP}$ = 7.4 Hz, 1H), 4.71 (m, 1H), 4.47 (dd, J = 5.1, 3.7 Hz, 1H), 4.34 (m, 1H), 4.19-4.15 (m, 2H), 4.00 (m, 1H), 3.86 (dd, J = 9.5, 3.3 Hz, 1H), 3.89-3.75 (m, 2H), 3.68 (dd, J = 12.9, 5.9 Hz, 1H), 3.15 (q, J = 7.4 Hz, 6H), 1.23 (t, J = 7.4 Hz, 9H).
FAB-MS m/z 588 $(M-H)^-$.

Reference Example 23: Synthesis of Adenosine=5'-(2,3,4,6-tetra-O-acetyl-β-D-mannopyranosyl)diphosphate (Compound 7)

In a manner similar to Reference Example 21, an ammonium salt of Compound 7 (0.020 g, 2.4%) was obtained from Compound D (0.43 g, 1.0 mmol) and 4'-morpholine-N,N'-dicyclohexylcarboxamidinium salt of adenosine=5'-phosphomorpholidate (0.71 g, 1.0 mmol).
$^1$H-NMR (300 MHz, $D_2O$)δ(ppm): 8.57 (s, 1H), 8.33(s, 1H), 6.13 (d, J = 5.1 Hz, 1H), 5.51-5.47 (m, 2H), 5.15-5.12 (m, 2H), 4.60 (m, 1H), 4.47 (dd, J = 4.7, 4.4 Hz, 1H), 4.35-4.29 (m, 2H), 4.30-4.29 (m, 2H), 4.05 (d, J = 12.1 Hz, 1H), 3.81 (m, 1H), 2.18 (s, 3H), 2.17 (s, 3H), 2.05 (s, 3H), 2.03 (s, 3H).
FAB-MS m/z 756$(M-H)^-$.

Reference Example 24: Synthesis of Adenosine=5'-(β-D-mannopyranosyl)diphosphate (Compound 8)

The ammonium salt of Compound 7 (0.018 g, 0.023 mmol) was dissolved in a mixed solution of methanol, triethylammonium bicarbonate buffer (pH=8.0, 0.1 mol/L) and triethylamine (14/13/1, 14 mL), and the solution was stirred at -30˚C for 2 weeks. After the reaction mixture was diluted with water, Dowex 50 ($H^+$ form) was added thereto and the pH was adjusted to about 4.0. The resin was removed by filtration, and the filtrate was purified by DEAE Sephadex A-25 column chromatography (Amersham Biosciences, eluted with 0.2-0.3 mmol/L ammonium acetate solution adjusted to pH 5.0 with acetic acid) to obtain Compound 8 (0.054 g, 13%).
$^1$H-NMR (300 MHz, $D_2O$)δ(ppm): 8.53 (s, 1H), 8.29 (s, 1H), 6.12 (d, J = 5.9 Hz, 1H), 5.20 (d, $J_{HP}$ = 8.7 Hz, 1H), 4.77 (m, 1H), 4.50 (dd, J = 5.0, 3.8 Hz, 1H), 4.37 (m, 1H), 4.22-4.17 (m, 2H), 4.09 (dd, J = 2.9, 1.9 Hz, 1H), 3.84 (d, J = 11.7 Hz, 1H), 3.68-3.60 (m, 2H), 3.50 (dd, J = 9.9, 9.5 Hz, 1H), 3.30 (m, 1H).
$^{31}$P-NMR (202.5 MHz, $D_2O$)δ(ppm): -10.64(d, J = 20.8 Hz), - 12.54 (d, J = 20.8 Hz).
FAB-MS m/z 588 $(M-H)^-$.

Reference Example 25: Synthesis of Adenosine=5'-(2,3,4,6,7-penta-O-acetyl-L-glycero-α-D-mannoheptopyranosyl) diphosphate (Compound 9)

In a manner similar to Reference Example 21, an ammonium salt of Compound 9 (0.083 g, 41%) was obtained from Compound Ga (0.11 g, 0.23 mmol) and 4'-morpholine-N,N'-dicyclohexylcarboxamidinium salt of adenosine=5'-phosphomorpholidate (0.16 g, 0.23 mmol).
FAB-MS m/z 828 $(M-H)^-$.

Reference Example 26: Synthesis of Adenosine=5'-(2,3,4,6,7-penta-O-acetyl-L-glycero-β-D-mannoheptopyranosyl) diphosphate (Compound 10)

In a manner similar to Reference Example 21, an ammonium salt of Compound 10 (0.012 g, 15%) was obtained from Compound Gb (0.046 g, 0.10 mmol) and 4'-morpholine-N,N'-dicyclohexylcarboxamidinium salt of adenosine=5'-phosphomorpholidate (0.070 g, 0.10 mmol).
FAB-MS m/z 828 $(M-H)^-$.

Reference Example 27: Synthesis of Adenosine=5'-(L-glycero-α-D-mannopyranosyl)diphosphate (Compound 11)

In a manner similar to Reference Example 22, Compound 11 (0.017 g, 21%) was obtained from the ammonium salt of Compound 9 obtained in Reference Example 25 (0.083 g, 0.023 mmol).
$^1$H-NMR (300 MHz, $D_2O$)δ(ppm): 8.46 (s, 1H), 8.21 (s, 1H), 6.09 (d, J = 5.9 Hz, 1H), 5.44 (dd, J = 7.5 Hz, 1H), 4.75 (m, 1H), 4.47 (dd, J = 5.0, 3.7 Hz, 1H), 4.34 (m, 1H), 4.18-4.15 (m, 2H), 3.96-3.91 (m, 2H), 3.87-3.78(m, 3H), 3.67 (dd, J = 11.4, 6.1 Hz, 1H), 3.58 (dd, J = 11.4, 7.1 Hz, 1H).
FAB-MS m/z 618 $(M-H)^-$.

Reference Example 28: Synthesis of Adenosine=5'-(L-glycero-β-D-mannopyranosyl)diphosphate (Compound 12)

The ammonium salt of Compound 10 obtained in Reference Example 26 (0.0080 g, 0.0093 mmol) was dissolved

in a mixed solution of methanol, triethylammonium bicarbonate buffer (pH=8.0, 0.1 mol/L) and triethylamine (14/13/1, 7.2 mL), and the solution was stirred at -30°C for 2 weeks. After the reaction mixture was diluted with water, Dowex 50 ($H^+$ form) was added thereto and the pH was adjusted to about 4.0. The resin was removed by filtration, and the filtrate was preparatively purified by high performance liquid chromatography [column; Develosil RPAQURUS (Nomura Chemical Co., Ltd.), diameter; 4.6 mm x 25 cm, detection wavelength; 260 nm, eluting solvent; 0.5% aqueous solution of acetic acid (1.0 mL/minute), detection time; 10 to 15 minutes] to obtain Compound 12 (0.0025 g, 38%).
$^1$H-NMR (300 MHz, $D_2O$)δ(ppm): 8.58 (s, 1H), 8.29 (s, 1H), 6.15 (d, J = 6.1 Hz, 1H), 5.20 (d, J = 8.3 Hz, 1H), 4.78 (d, J = 5.6 Hz, 1H), 4.53 (dd, J = 4.5, 3.7 Hz, 1H), 4.40 (m, 1H), 4.24-4.20 (m, 2H), 4.05 (d, J = 3.2 Hz, 1H), 3.92 (dd, J = 7.4, 6.2 Hz, 1H), 3.79 (d, J = 9.8 Hz, 1H), 3.73-3.64 (m, 3H), 3.34 (d, J = 9.8 Hz, 1H).
FAB-MS m/z 618 $(M-H)^-$.

Reference Example 29: Synthesis of Adenosine=5'-(2,3,4,6,7-penta-O-acetyl-D-glycero-α-D-mannoheptopyranosyl) diphosphate (Compound 13)

In a manner similar to Reference Example 21, an ammonium salt of Compound 13 (0.059 g, 30%) was obtained from Compound Ja (0.11 g, 0.23 mmol) and 4'-morpholine-N,N'-dicyclohexylcarboxamidinium salt of adenosine=5'-phosphomorpholidate (0.16 g, 0.23 mmol).
$^1$H-NMR (300 MHz, $D_2O$)δ(ppm): 8.57 (s, 1H), 8.27 (s, 1H), 6.12 (d, J = 5.3 Hz, 1H), 5.57 (dd, J = 1.2, 7.7 Hz, 1H), 5.33 (m, 1H), 5.27-5.11 (m, 2H), 4.67 (t, J = 5.3 Hz, 1H), 4.49 (dd, J = 4.9, 4.0 Hz, 1H), 4.37-4.28 (m, 4H), 4.27-4.14 (m, 3H), 2.13 (s, 3H), 2.08 (s, 3H), 2.03 (s, 3H), 2.02 (s, 3H), 1.92 (s, 3H).
FAB-MS m/z 828 $(M-H)^-$.

Reference Example 30: Synthesis of Adenosine=5'-(2,3,4,6,7-penta-O-acetyl-D-glycero-β-D-mannoheptopyranosyl) diphosphate (Compound 14)

In a manner similar to Reference Example 21, an ammonium salt of Compound 14 (0.042 g, 49%) was obtained from Compound Jb (0.037 g, 0.074 mmol) and 4'-morpholine-N,N'-dicyclohexylcarboxamidinium salt of adenosine=5'-phosphomorpholidate (0.070 g, 0.10 mmol).
$^1$H-NMR (500 MHz, $D_2O$)δ(ppm): 8.63 (s, 1H), 8.36 (s, 1H), 6.17 (d, J = 5.1 Hz, 1H), 5.19 (d, $J_{HP}$ = 8.6 Hz, 1H), 5.53 (d, J = 1.1 Hz, 1H), 5.48 (d, J = 8.7 Hz, 1H), 5.24-5.19 (m, 2H), 5.11 (d, J = 10.8 Hz, 1H), 4.74 (dd, J = 5.5, 5.2 Hz, 1H), 4.50 (m, 1H), 4.39-4.37 (m, 2H), 4.26-4.22 (m, 3H), 2.19 (s, 3H), 2.10 (s, 6H), 2.04 (s, 3H), 1.97 (s, 3H).
FAB-MS m/z 828 $(M-H)^-$.

Reference Example 31: Synthesis of Adenosine=5'-(D-glycero-α-D-mannoheptopyranosyl)diphosphate (Compound 15)

In a manner similar to Reference Example 22, Compound 15 (0.030 g, quantitative) was obtained from the ammonium salt of Compound 13 obtained in Reference Example 29 (0.028 g, 0.034 mmol).
$^1$H-NMR (500 MHz, $D_2O$)δ(ppm): 8.63 (s, 1H), 8.36 (s, 1H), 6.17 (d, J = 5.1 Hz, 1H), 5.19 (d, $J_{HP}$ = 8.6 Hz, 1H), 5.53 (d, J = 1.1 Hz, 1H), 5.48 (d, J = 8.7 Hz, 1H), 5.24-5.19 (m, 2H), 5.11 (d, J = 10.8 Hz, 1H), 4.74 (dd, J = 5.5, 5.2 Hz, 1H), 4.50 (m, 1H), 4.39-4.37 (m, 2H), 4.26-4.22 (m, 3H), 2.19 (s, 3H), 2.10 (s, 6H), 2.04 (s, 3H), 1.97 (s, 3H).
FAB-MS m/z 618 $(M-H)^-$.

Reference Example 32: Synthesis of Adenosine=5'-(D-glycero-β-D-mannoheptopyranosyl)diphosphate (Compound 16)

In a manner similar to Reference Example 28, Compound 16 (0.0023 g, 15%) was obtained from the ammonium salt of Compound 14 obtained in Reference Example 30 (0.021 g, 0.023 mmol).
1H-NMR (500 MHz, $D_2O$)δ(ppm): 8.58 (s, 1H), 8.31 (s, 1H), 6.16 (d, J = 5.9 Hz, 1H), 5.19 (d, $J_{HP}$ = 8.6 Hz, 1H), 4.78 (dd, J = 5.9, 4.7 Hz, 1H), 4.53 (dd, J = 4.7, 3.6 Hz, 1H), 4.40 (m, 1H), 4.24-4.20 (m, 2H), 4.05 (d, J = 2.5 Hz, 1H), 3.99 (m, 1H), 3.75-3.73 (m, 2H), 3.68-3.61 (m, 2H), 3.44 (dd, J = 9.1, 2.9 Hz, 1H).
FAB-MS m/z 618 $(M-H)^-$.

Reference Example 33: Synthesis of Adenosine=5'-(2,3,4,6-tetra-O-methyl-D-mannopyranosyl)diphosphate (Compound 17)

In a manner similar to Reference Example 21, an ammonium salt of Compound 17 (0.0025 g, α/β = 2/1, yield 38%) was obtained from Compound L (0.030 g, 0.040 mmol) and 4'-morpholine-N,N'-dicyclohexylcarboxamidinium salt

of adenosine=5'-phosphomorpholidate (0.029 g, 0.040 mmol).
FAB-MS m/z 644 $(M-H)^-$.

Compounds 18 to 21 and 23 were purchased from Sigma, and Compound 22 was purchased from Fluka.

Reference Example 34: Synthesis of 1-Hydroxymethyl-2,3,4,6-tetra-O-benzyl-D-mannose (Compound S)

[Step 1]

To a dioxane solution (15 mL) of methyl-D-mannopyranose (5.0 g, 26 mmol) was added potassium hydroxide powder (25 g, 450 mmol) in small portions at 80˚C, followed by stirring at the same temperature for one hour. To the reaction mixture was dropwise added benzyl bromide (16 mL, 130 mmol) over a period of 40 minutes, and the mixture was refluxed for 3 hours. The reaction mixture was poured into ice water, followed by extraction with diethyl ether. The organic layer was washed with a saturated aqueous solution of sodium chloride, dried over sodium sulfate, and then concentrated under reduced pressure. The residue was purified by silica gel column chromatography (hexane/ethyl acetate = 6/1) to obtain a tetra-O-benzyl form (7.0 g, yield 49%).
$^1$H-NMR (300 MHz, $CDCl_3$)δ(ppm): 7.38-7.26 (m, 18H), 7.19-7.16 (m, 2H), 4.89 (d, J = 10.8 Hz, 1H), 4.76 (d, J = 13.7 Hz, 1H), 7.86 (d, J = 12.0 Hz, 1H), 4.68 (d, J = 13.7 Hz, 1H), 4.62 (s, 2H), 4.56 (d, J = 12.0 Hz, 1H), 4.51 (d, J = 10.8 Hz, 1H), 3.99 (dd, J = 9.9, 9.2 Hz, 1H), 3.89 (dd, J = 9.2, 3.0 Hz, 1H), 3.79 (dd, J = 3.0, 1.8 Hz, 1H), 3.76-3.71 (m, 4H), 3.33 (s, 3H).
FAB-MS m/z: 555 $(M+H)^+$.

[Step 2]

To an acetic anhydride solution (10 mL) of the tetra-O-benzyl form obtained in Step 1 (10 g, 18 mmol) was added concentrated sulfuric acid (0.020 mL) at 0˚C, and the mixture was stirred at the same temperature for 15 minutes. The reaction mixture was poured into a saturated aqueous solution of sodium hydrogencarbonate, followed by extraction with chloroform. The organic layer was washed with a saturated aqueous solution of sodium chloride, dried over sodium sulfate, and then concentrated under reduced pressure. The residue was purified by silica gel column chromatography (hexane/ethyl acetate = 1/1) to obtain a 1-O-acetyl form (6.9 g, yield 63%).
$^1$H-NMR (500 MHz, $CDCl_3$)δ(ppm): 7.40 (m, 1H), 7.38-7.22 (m, 17H), 7.19-7.16 (m, 2H), 6.21 (d, J = 2.0 Hz, 1H), 4.89 (d, J = 10.7 Hz, 1H), 4.78 (d, J = 12.2 Hz, 1H), 4.72 (d, J = 12.2 Hz, 1H), 4.65 (d, J = 12.1 Hz, 1H), 4.60 (d, J = 12.0 Hz, 1H), 4.56 (d, J = 12.0 Hz, 1H), 4.55 (d, J = 10.7 Hz, 1H), 4.53 (d, J = 12.1 Hz, 1H), 4.07 (dd, J = 9.9, 9.6 Hz, 1H), 3.88-3.82 (m, 2H), 3.78 (dd, J = 11.0, 4.7 Hz, 1H), 3.74 (dd, J = 3.0, 2.0 Hz, 1H), 3.71 (dd, J = 11.0, 1.8 Hz, 1H), 2.02 (s, 3H).
FAB-MS m/z: 605 $(M+Na)^+$.

[Step 3]

To a dichloromethane solution (10 mL) of the 1-O-acetyl form obtained in Step 2 (580 mg, 1.0 mmol) was added trimethylsilyl iodide (0.14 mL, 1.1 mmol) at 0˚C. After stirring at the same temperature for 40 minutes, the solvent was distilled off under reduced pressure. To the residue was added toluene (1.0 mL), and the solvent was distilled off again under reduced pressure. The residue was dissolved in THF (10 mL), and a THF solution (1.0 mL) of cyanotetrabutylammonium (0.54 g, 2.0 mmol) was added thereto, followed by stirring at room temperature for 2 hours. Then, the obtained reaction solution was poured into ice water, followed by extraction with diethyl ether. The organic layer was washed with a saturated aqueous solution of sodium chloride, dried over sodium sulfate, and then concentrated under reduced pressure. The residue was purified by silica gel column chromatography (hexane/ethyl acetate = 3/1) to obtain a cyano form (0.30 g, yield 65%).
$^1$H-NMR (500 MHz, $CDCl_3$)δ(ppm): 7.52-7.45 (m, 2H), 7.37-7.29 (m, 16H), 7.18-7.15 (m, 2H), 4.98 (d, J = 11.4 Hz, 1H), 4.92 (d, J = 11.4 Hz, 1H), 4.84 (d, J = 10.6 Hz, 1H), 4.66 (s, 2H), 4.61 (d, J = 12.0 Hz, 1H), 4.55 (d, J = 10.6 Hz, 1H), 4.54 (d, J = 12.0 Hz, 1H), 4.23 (d, J = 1.4 Hz, 1H), 4.02 (dd, J = 2.9, 1.4 Hz, 1H), 3.93 (dd, J = 9.5, 9.3 Hz, 1H), 3.76-3.68 (m, 2H), 3.54 (dd, J = 9.3, 2.9 Hz, 1H), 3.44 (m, 1H).
FAB-MS m/z 550 $(M+H)^+$.

[Step 4]

To an ethanol solution (5.0 mL) of the cyano form obtained in Step 3 (140 mg, 0.26 mmol) was added an aqueous solution of sodium hydroxide (1.2 mL, 2 mol/L), and the mixture was stirred at 80˚C for 12 hours. The reaction mixture was poured into hydrochloric acid (10 mL, 2 mol/L), followed by extraction with chloroform. The organic layer was washed with a saturated aqueous solution of sodium chloride, dried over sodium sulfate, and then concentrated

under reduced pressure to obtain carboxylic acid (110 mg, yield 72%).
$^1$H-NMR (300 MHz, $CDCl_3$)δ(ppm): 7.30-7.22 (m, 20H), 4.97 (d, J = 11.7 Hz, 1H), 4.89 (d, J = 10.8 Hz, 1H), 4.78 (d, J = 11.3 Hz, 1H), 4.74 (d, J = 11.3 Hz, 1H), 4.70 (s, 2H), 4.54 (d, J = 10.8 Hz, 1H), 4.52 (d, J = 11.7 Hz, 1H), 3.91 (t, J = 9.5 Hz, 1H), 3.86 (d, J = 2.2 Hz, 1H), 3.83-3.61 (m, 3H), 3.51-3.35 (m, 2H).
FAB-MS m/z 569 $(M+H)^+$.

[Step 5]

To a THF suspension (20 mL) of lithium aluminum hydride (110 mg, 0.19 mmol) was added the carboxylic acid obtained in Step 4 (110 mg, 0.19 mmol) in an atmosphere of nitrogen, and the mixture was stirred at 80˚C for 6 hours. The reaction mixture was poured into hydrochloric acid (20 mL, 2 mol/L), followed by extraction with chloroform. The organic layer was washed with a saturated aqueous solution of sodium hydrogencarbonate and a saturated aqueous solution of sodium chloride, dried over sodium sulfate, and then concentrated under reduced pressure to obtain Compound S (80 mg, yield 77%).
$^1$H-NMR (300 MHz, $CDCl_3$)δ(ppm): 7.38-7.22 (m, 20H), 4.97 (d, J = 11.7 Hz, 1H), 4.89 (d, J = 10.8 Hz, 1H), 4.78 (d, J = 11.7 Hz, 1H), 4.73 (d, J = 11.7 Hz, 1H), 4.70 (s, 2H), 4.60-4.51 (m, 4H), 3.91 (dd, J = 9.6, 9.5 Hz, 1H), 3.86 (d, J = 2.5 Hz, 1H), 3.83-3.70 (m, 2H), 3.62 (dd, J = 9.5, 2.5 Hz, 1H), 3.50-3.35 (m, 2H).
FAB-MS m/z 555 $(M+H)^+$.

Reference Example 35: Synthesis of 1-Hydroxymethyl-2,3,4,7-tetra-O-benzyl-6-deoxy-D-mannoheptose (Compound T)

[Step 1]

To a methanol solution (2.0 mL) of L-galactose (0.51 g, 2.8 mmol) was added acetyl chloride (0.26 mL), and after the mixture was stirred at room temperature for 20 hours, the solvent was distilled off under reduced pressure. From the obtained crude 1-O-methyl form, benzyl bromide (2.6 mL, 4.4 mmol) and sodium hydride (55% in oil, 0.53 g, 4.4 mmol), a 1-O-methyl-2,3,4,6-tetra-O-benzyl form (0.69 g, 44%) was obtained as a mixture of anomers (α/β mixture ratio unknown) in a manner similar to Step 1 of Reference Example 16.
FAB-MS m/z 555 $(M+H)^+$.

[Step 2]

To an acetic acid solution (700 mL) of the tetrabenzyl form obtained in Step 1 (76 g, 140 mmol) was added trifluoromethanesulfonic acid (2.0 mol/L in water, 280 mL) in two portions. The mixture was stirred at 80˚C for 6 hours, and the solvent was distilled off under reduced pressure. After stirring at room temperature for 16 hours, the reaction mixture was poured into ice water, followed by extraction with ethyl acetate. The organic layer was washed with a saturated aqueous solution of sodium chloride, dried over sodium sulfate, and then concentrated under reduced pressure. The residue was purified by silica gel column chromatography (hexane/ethyl acetate = 5/1) to obtain a 1-hydroxy form (48 g, yield 64%) as a mixture of anomers (α/β = 2/1).
$^1$H-NMR (300 MHz, $CDCl_3$)δ(ppm): 7.60-7.25 (m, 20H), 5.28 (d, J = 1.5 Hz, 1H), 4.93 (d, J = 11.6 Hz, 1H), 4.89 (d, J = 11.7 Hz, 1H), 4.79 (d, J = 10.1 Hz, 1H), 4.74 (d, J = 10.1 Hz, 1H), 4.70 (d, J = 11.7 Hz, 1H), 4.58 (d, J = 11.6 Hz, 1H), 4.48 (d, J = 11.9 Hz, 1H), 4.40 (d, J = 11.9 Hz, 1H), 4.03 (dd, J = 9.8, 3.6 Hz, 1H), 3.96 (m, 1H), 3.90 (dd, J = 9.8, 2.7 Hz, 1H), 3.61-3.46 (m, 2H), 1.98 (d, J = 1.8 Hz, 1H).
FAB-MS m/z 535 $(M+H)^+$.

[Step 3]

To a THF suspension (32 mL) of sodium hydride (55% in oil, 3.2 g, 81 mmol) was added triethyl phosphonoacetate (16 mL, 81 mmol) under ice-cooling, and after stirring for one hour, the 1-hydroxy form obtained in Step 2 (4.4 g, 8.1 mmol) was added thereto. The mixture was stirred at room temperature for 18 hours and then at 70˚C for 2 hours. The reaction mixture was poured into ice water, followed by extraction with ethyl acetate. The organic layer was washed with a saturated aqueous solution of sodium chloride, dried over sodium sulfate, and then concentrated under reduced pressure. The residue was purified by silica gel column chromatography (hexane/ethyl acetate = 3/1) to obtain an ethyl ester form (3.9 g, yield 79%) as a mixture of anomers (α/β = 1/11). $^1$H-NMR (300 MHz, $CDCl_3$)δ(ppm): 7.53-7.22 (m, 20H), 4.95 (d, J = 11.0 Hz, 1H), 4.90 (d, J = 11.6 Hz, 1H), 4.73 (d, J = 11.6 Hz, 1H), 4.64 (d, J = 11.6 Hz, 1H), 4.60 (d, J = 11.0 Hz, 1H), 4.59 (d, J = 11.6 Hz, 1H), 4.44 (d, J = 11.7 Hz, 1H), 4.37 (d, J = 11.7 Hz, 1H), 4.44-4.21 (m, 3H), 3.77-3.51 (m, 6H), 2.73 (dd, J = 15.3, 3.0 Hz, 1H), 2.44 (dd, J = 15.3, 8.0 Hz, 1H), 1.14 (t, J = 7.2 Hz, 3H).

ESI-MS m/z 611 $(M+H)^+$.

[Step 4]

To an acetic acid/acetic anhydride solution (1/1, 20 mL) of the ethyl ester form obtained in Step 3 (3.9 g, 8.1 mmol) was added concentrated sulfuric acid (0.030 mL) at 0˚C, and the mixture was stirred at the same temperature for 15 minutes. The reaction mixture was poured into a saturated aqueous solution of sodium hydrogencarbonate, followed by extraction with chloroform. The organic layer was washed with a saturated aqueous solution of sodium chloride, dried over sodium sulfate, and then concentrated under reduced pressure. The residue was purified by silica gel column chromatography (hexane/ethyl acetate = 1/1) to obtain an acetyl form (2.7 g, yield 58%).
$^1$H-NMR (300 MHz, $CDCl_3$)δ(ppm): 7.37-7.21 (m, 15H), 4.97 (d, J = 11.4 Hz, 2H), 4.80 (d, J = 11.6 Hz, 1H), 4.73 (d, J = 11.4 Hz, 1H), 4.65 (d, J = 11.6 Hz, 1H), 4.64 (d, J = 11.4 Hz, 1H), 4.14-3.98 (m, 4H), 3.88 (m, 1H), 3.76-3.53 (m, 4H), 2.78 (dd, J = 15.4, 3.1 Hz, 1H), 2.48 (m, 1H), 1.96 (s, 3H), 1.19 (t, J = 7.2 Hz, 3H).

[Step 5]

To an ethanol solution (300 mL) of the acetyl form obtained in Step 4 (2.7 g, 4.7 mmol) was added sodium ethoxide (0.45 g, 6.6 mmol), and the mixture was stirred at room temperature for 30 minutes. The reaction mixture was poured into hydrochloric acid (10 mL, 1.0 mol/L), followed by extraction with ethyl acetate. The organic layer was washed with a saturated aqueous solution of sodium chloride, dried over sodium sulfate, and then the solvent was distilled off under reduced pressure to obtain a hydroxy form (2.8 g, quantitative).
$^1$H-NMR (300 MHz, $CDCl_3$)δ(ppm): 7.20-7.13 (m, 15H), 4.89 (d, J = 11.0 Hz, 1H), 4.86 (d, J = 11.6 Hz, 1H), 4.69 (d, J = 11.6 Hz, 1H), 4.63 (d, J = 11.7 Hz, 1H), 4.56 (d, J = 11.7 Hz, 1H), 4.55 (d, J = 11.0 Hz, 1H), 3.98 (qd, J = 7.2, 1.6 Hz, 2H), 3.79 (d, J = 2.6 Hz, 2H), 3.69-3.53 (m, 4H), 3.36-3.31 (m, 2H), 2.69 (dd, J = 15.4, 2.8 Hz, 1H), 2.34 (m, 1H), 1.09 (t, J = 7.2 Hz, 3H).
ESI-MS m/z 521 $(M+H)^+$.

[Step 6]

To a DMF solution (10 mL) of the hydroxy form obtained in Step 5 (5.5 g, 14 mmol) were added tertbutyldimethylsilyl chloride (0.71 g, 4.7 mmol) and imidazole (0.64 g, 9.4 mmol) under ice-cooling, and the mixture was stirred at room temperature for one day. To the reaction mixture was added water, followed by extraction with ethyl acetate. The organic layer was washed with a saturated aqueous solution of ammonium chloride and a saturated aqueous solution of sodium chloride, dried over anhydrous sodium sulfate, and then concentrated under reduced pressure. The residue was purified by silica gel column chromatography (hexane/ethyl acetate = 20/1) to obtain a silyl form (2.4 g, yield 81%). $^1$H-NMR (300 MHz, $CDCl_3$)δ(ppm): 7.33-7.25 (m, 15H), 4.95 (d, J = 11.2 Hz, 1H), 4.92 (d, J = 10.6 Hz, 1H), 4.72 (d, J = 12.0 Hz, 1H), 4.66 (d, J = 12.0 Hz, 1H), 4.61 (d, J = 11.2, Hz, 1H), 4.60 (d, J = 10.6 Hz, 1H), 4.03 (q, J = 7.2 Hz, 2H), 3.96 (d, J = 3.2 Hz, 1H), 3.75-3.61 (m, 5H), 3.39 (m, 1H), 2.72 (dd, J = 15.3, 2.6 Hz, 1H), 2.43 (m, 1H), 1.15 (t, J = 7.2 Hz, 3H), 0.85 (s, 9H), 0.068 (s, 3H), 0.070 (s, 3H).
ESI-MS m/z 635 $(M+H)^+$, 657 $(M+Na)^+$.

[Step 7]

To a THF suspension (120 mL) of lithium aluminum hydride (0.16 g, 4.2 mmol) was added the silyl form obtained in Step 6 (2.4 g, 3.8 mmol) in an atmosphere of nitrogen under ice-cooling, and the mixture was stirred at the same temperature for 1.5 hours. To the reaction mixture was added sodium sulfate decahydrate (3.2 g, 10 mmol), followed by stirring at room temperature for one hour. The precipitate was removed by filtration and the filtrate was concentrated under reduced pressure to obtain a crude hydroxy form (2.3 g).
FAB-MS m/z 591 $(M-H)^-$.

[Step 8]

From the crude hydroxy form obtained in Step 7 (2.3 g, 3.9 mmol), benzyl bromide (0.93 mL, 7.8 mmol) and sodium hydride (55% in oil, 0.31 g, 7.8 mmol), a crude tetrabenzyl form was obtained in a manner similar to Step 1 of Reference Example 16. To a methylene chloride solution (10 mL) of the obtained crude tetrabenzyl form was added trifluoroacetic acid (1.0 mL), and the mixture was stirred at room temperature for one hour. The reaction mixture was poured into ice-cooled water, followed by extraction with chloroform. The organic layer was washed with a saturated aqueous solution of sodium hydrogencarbonate and a saturated aqueous solution of sodium chloride, dried over anhydrous sodium sulfate, and then concentrated under reduced pressure. The residue was purified by silica gel column chromatography (hexane/ethyl acetate = 4/1) to obtain Compound T (0.47 g, yield 22%).
$^1$H-NMR (300 MHz, $CDCl_3$)δ(ppm): 7.37-7.27 (m, 20H), 4.97 (d, J = 11.4 Hz, 1H), 4.95 (d, J = 11.4 Hz, 1H), 4.79 (d, J = 11.8 Hz, 1H), 4.74 (d, J = 11.8 Hz, 1H), 4.67 (d, J = 11.4 Hz, 2H), 4.51 (d, J = 12.1 Hz, 1H), 4.43 (d, J = 12.1 Hz, 1H), 3.85 (d, J = 2.6 Hz, 1H), 3.71 (t, J = 9.5 Hz, 1H), 3.68-3.56 (m, 4H), 3.43-3.31 (m, 3H), 2.21 (m, 1H), 1.79 (m, 1H).

Reference Example 36: Synthesis of 2,3,4,8-Tetra-O-acetyl-6,7-dideoxy-D-octose (Compound U)

[Step 1]

To a DMF suspension (10 mL) of sodium hydride (2.0 g, 60%, 50 mmol) were added Compound M (7.1 g, 16 mmol) and benzyl bromide (5.7 mL, 48 mmol) under ice-cooling, and after the temperature was raised to room temperature, the mixture was stirred at the same temperature for 12 hours. The reaction mixture was poured into ice water, followed by extraction with diethyl ether. The organic layer was washed with a saturated aqueous solution of sodium chloride, dried over sodium sulfate, and then concentrated under reduced pressure. The residue was purified by silica gel column chromatography (hexane/ethyl acetate = 9/1) to obtain a tri-O-benzyl form (7.2 g, yield 60%).
$^1$H-NMR (300 MHz, $CDCl_3$)δ(ppm): 7.50-7.17 (m, 30H), 4.71-4.56 (m, 6H), 4.26 (d, J = 10.4 Hz, 1H), 4.01 (t, J = 9.4 Hz, 1H), 3.86 (dd, J = 9.4, 3.1 Hz, 1H), 3.82 (dd, J = 3.1, 1.8 Hz, 1H), 3.78 (m, 1H), 3.53 (dd, J = 9.7, 1.7 Hz, 1H), 3.39 (s, 3H), 3.26 (dd, J = 9.7, 5.4 Hz, 1H).
FAB-MS m/z 705 $(M-H)^-$.

[Step 2]

The tri-O-benzyl form obtained in Step 1 (35 g, 50 mmol) was dissolved in a mixed solvent of 1,4-dioxane and methanol (1/1, 200 mL), and trifluoroacetic acid (20 mL) was added thereto under ice-cooling, followed by stirring at room temperature for 12 hours. To the reaction mixture was added potassium carbonate, and the precipitated salt was removed from the mixture by filtration. The filtrate was concentrated and the residue was purified by silica gel column chromatography (hexane/ethyl acetate = 4/1) to obtain a 6-hydroxy form (15.2 g, yield 71%).
$^1$H-NMR (300 MHz, $CDCl_3$)δ(ppm): 7.38-7.29 (m, 15H), 4.95(d, J = 11.1 Hz, 1H), 4.79 (d, J = 11.8 Hz, 1H), 4.70 (d, J = 1.8 Hz, 1H), 4.69 (d, J = 11.8 Hz, 1H), 4.66 (d, J = 11.1 Hz, 1H), 4.64 (s, 2H), 4.09-3.75 (m, 5H), 3.65 (m, 1H), 3.31 (s, 3H).
FAB-MS m/z 463 $(M-H)^-$.

[Step 3]

To a THF solution (100 mL) of oxalyl chloride (2.2 mL, 24 mmol) was added a THF solution (5.0 mL) of dimethyl sulfide (2.0 mL, 26 mmol) at -78°C, followed by stirring at the same temperature for one hour. To the reaction mixture was slowly added dropwise a THF solution (20 mL) of the 6-hydroxy form obtained in Step 2 (3.0 g, 12 mmol) at -78°C, followed by stirring at the same temperature for one hour. To the reaction mixture was added triethylamine (7.4 mL, 52 mmol), and after the temperature was raised to room temperature, the mixture was stirred for 30 minutes. Then, triphenylphosphoranylidene methyl acetate (10 g, 30 mmol) was added thereto, and the mixture was stirred for one hour. To the reaction mixture was added water, followed by stirring for 10 minutes and then extraction with diethyl ether. The organic layer was washed with a saturated aqueous solution of sodium chloride, dried over sodium sulfate, and then concentrated under reduced pressure. The residue was purified by silica gel column chromatography (hexane/ethyl acetate = 4/1) to obtain an α,β-unsaturated ester (5.1 g, yield 82%).
$^1$H-NMR (300 MHz, $CDCl_3$)δ(ppm): 7.37-7.28 (m, 15H), 7.14 (dd, J = 15.8, 4.7 Hz, 1H), 6.24 (dd, J = 15.8, 1.7 Hz, 1H), 4.90 (d, J = 10.7 Hz, 1H), 4.78 (d, J = 12.5 Hz, 1H), 4.72 (d, J = 12.5 Hz, 1H), 4.65 (s, 2H), 4.61 (d, J = 10.7 Hz, 1H), 3.92 (dd, J = 9.3, 3.0 Hz, 1H), 3.82-3.71 (m, 4H), 3.62 (s, 3H), 3.15 (s, 3H).
API-MS m/z 536 $(M+H20)^+$, 487 $(M-MeO)^+$.

[Step 4]

To a THF solution (100 mL) of the α,β-unsaturated ester obtained in Step 3 (3.3 g, 6.4 mmol) was dropwise added a THF solution (13 mL, 1.0 mol/L) of lithium diisopropylaluminum hydride in an atmosphere of nitrogen at -78°C, and the mixture was stirred at the same temperature for one hour. A THF solution (13 mL, 1.0 mol/L) of lithium diisopropylaluminum hydride was further added dropwise thereto, and the mixture was stirred at the same temperature for 30 minutes. The reaction mixture was poured into 1.0 mol/L hydrochloric acid (50 mL) under ice-cooling, followed by stirring for 2.5 hours and then extraction with a mixture of ethyl acetate and hexane (1/1). The organic layer was washed

with a saturated aqueous solution of sodium chloride, dried over sodium sulfate, and then concentrated under reduced pressure to obtain an alcohol form (4.1 g).

To a methanol solution (20 mL) of the obtained alcohol form (4.1 g) was added 10% palladium carbon (90 mg), followed by stirring at room temperature for 14 hours in an atmosphere of hydrogen. The catalyst was removed from the reaction mixture by filtration, and the filtrate was concentrated to obtain a tetrahydroxy form (1.4 g).

To a pyridine solution (15 mL) of the obtained tetrahydroxy form (1.4 g) was added acetic anhydride (15 mL), followed by stirring at room temperature for one hour. Then the mixture was concnetrated under reduced pressure, and the residue was purified by silica gel column chromatography (hexane/ethyl acetate = 3/1) to obtain a tetraacetoxy form (0.53 g, yield 21%).
$^1$H-NMR (300 MHz, $CDCl_3$)δ(ppm): 5.29 (dd, J = 9.9, 3.4 Hz, 1H), 5.23 (dd, J = 3.4, 1.7 Hz, 1H), 5.11 (t, J = 9.9 Hz, 1H), 4.65 (d, J = 1.7 Hz, 1H), 4.07 (t, J = 6.6 Hz, 2H), 3.75 (m, 1H), 3.39 (s, 3H), 2.15 (s, 3H), 2.05 (s, 6H), 1.99 (s, 3H) 1.71-1.54 (m, 4H).
FAB-MS m/z: 413 $(M+Na)^+$, 391 $(M+H)^+$, 359 $(M-OMe)^-$.

[Step 5]

To an acetic anhydride solution (6.0 mL) of the tetraacetoxy form obtained in Step 3 (0.25 g, 0.64 mmol) was added concentrated sulfuric acid (0.010 mL) at 0˚C. After stirring for 30 minutes, the mixture was poured into a saturated aqueous solution of sodium hydrogencarbonate, followed by extraction with chloroform. The organic layer was washed with a saturated aqueous solution of sodium chloride, dried over sodium sulfate, and then concentrated under reduced pressure. The residue was dissolved in DMF (15 mL) and hydrazine acetate (0.51 g, 5.6 mmol) was added thereto, followed by stirring at room temperature for 2 hours. The reaction mixture was poured into water, followed by extraction with ethyl acetate. The organic layer was washed with a saturated aqueous solution of sodium chloride, dried over sodium sulfate, and then concentrated under reduced pressure. The residue was purified by silica gel column chromatography (hexane/ethyl acetate = 4/1) to obtain Compound U (0.52 g, quantitative).
$^1$H-NMR (300 MHz, $CDCl_3$)δ(ppm): 5.39 (dd, J = 9.9, 3.4 Hz, 1H), 5.27 (dd, J = 3.4, 1.6 Hz, 1H), 5.15-5.05 (m, 2H), 4.07 (t, J = 6.0 Hz, 2H), 3.46 (m, 1H), 2.14 (s, 3H), 2.06 (s, 3H), 2.04 (s, 3H), 1.99 (s, 3H), 1.91 (m, 1H), 1.70-1.56 (m, 3H).
FAB-MS m/z: 359 $(M-OH)^+$, 399 $(M+Na)^+$.

Reference Example 37: Synthesis of Adenosine=5'-(2,3,4,6-tetra-O-acetyl-β-D-mannopyranylmethoxy)diphosphate (Compound 24)

[Step 1]

From Compound S (70 mg, 0.12 mmol), DMAP (18 mg, 0.14 mmol) and diphenyl chlorophosphate (0.030 mL, 0.14 mmol), a diphenylphosphate (90 mg, yield 96%) was obtained in a manner similar to Reference Example 3.
$^1$H-NMR (300 MHz, $CDCl_3$)δ(ppm): 7.61-7.21 (m, 30H), 4.95 (d, J = 11.4 Hz, 1H), 4.85 (d, J = 10.7 Hz, 1H), 4.72 (d, J = 11.8 Hz, 1H), 4.66 (d, J = 11.8 Hz, 1H), 4.57 (d, J = 11.4 Hz, 1H), 4.54 (s, 2H), 4.52 (d, J = 10.7 Hz, 1H), 4.48 (m, 1H), 4.41-4.91 (m, 3H), 3.88 (m, 1H), 3.72-3.68 (m, 2H), 3.50 (m, 1H), 3.41 (m, 1H).
ESI-MS m/z 787 $(M+H)^+$.

[Step 2]

To a methanol solution (10 mL) of the diphenylphosphate obtained in Step 1 (90 mg, 0.12 mmol) was added 10% palladium carbon (90 mg), followed by stirring at room temperature for 14 hours in an atmosphere of hydrogen. The catalyst was removed from the reaction mixture by filtration, and the filtrate was concentrated to obtain a tetrahydroxy form (51 mg).
ESI-MS m/z 427 $(M+H)^+$.

[Step 3]

To a pyridine solution (1.0 mL) of the tetrahydroxy form obtained in Step 2 (51 mg) was added acetic anhydride (1.0 mL), followed by stirring at room temperature for one hour. The reaction mixture was concentrated, and the residue was purified by silica gel column chromatography (hexane/ethyl acetate = 3/1) to obtain an acetyl form (22 mg, yield 31%).
ESI-MS m/z 595 $(M+H)^+$.

[Step 4]

To an ethanol solution (1.0 mL) of the acetyl form obtained in Step 3 (22 mg, 0.037 mmol) was added platinum oxide (5.0 mg), followed by stirring at room temperature for 5 days in an atmosphere of hydrogen. The catalyst was removed from the reaction mixture by filtration, and the filtrate was concentrated. The residue was purified by silica gel column chromatography (chloroform/methanol = 20/1) to obtain a diphosphate. The obtained diphosphate was dissolved in ethanol (0.50 mL) and platinum oxide (10 mg) was added thereto, followed by stirring at room temperature for one day in an atmosphere of hydrogen. The catalyst was removed from the reaction mixture by filtration, and the filtrate was concentrated to obtain a dephenylated form (11 mg, yield 68%).
FAB-MS m/z 441 $(M-H)^-$.

[Step 5]

In a manner similar to Reference Example 21, an ammonium salt of Compound 24 (15 mg, yield 72%) was obtained from the dephenylated form obtained in Step 4 (11 mg, 0.026 mmol), 4'-morpholine-N,N'-dicyclohexylcarboxamidinium salt of adenosine=5'-phosphomorpholidate (11 mg, 0.026 mmol) and tetrazole (18 mg, 0.026 mmol).
$^1$H-NMR (300 MHz, $D_2O$)δ(ppm): 8.52 (s, 1H), 8.23 (br s, 1H), 6.13 (dd, J = 6.5, 1.1 Hz, 1H), 5.37 (d, J = 2.6 Hz, 1H), 5.09 (dd, J = 10.1, 9.9 Hz, 1H), 4.94 (m, 1H), 4.67 (dd, J = 5.1, 4.4 Hz, 1H), 4.49 (dd, J =4.2, 3.9 Hz, 1H), 4.29 (m, 1H), 4.25-4.15 (m, 3H), 4.00-3.82 (m, 3H), 3.53 (d, J = 3.0 Hz, 1H), 2.14 (s, 3H), 2.05 (s, 3H), 2.02 (s, 3H), 1.95 (s, 3H).
FAB-MS m/z 770 $(M-H)^-$.

Reference Example 38: Synthesis of Adenosine=5'-(β-D-mannopyranylmethoxy)diphosphate (Compound 25)

In a manner similar to Example 2, Compound 25 (1.8 mg, yield 17%) was obtained from the ammonium salt of Compound 24 (15 mg, 0.019 mmol) obtained in Reference Example 37.
$^1$H-NMR (300 MHz, $D_2O$)δ(ppm); 8.49 (s, 1H), 8.23 (br s, 1H), 6.11 (d, J = 5.9 Hz, 1H), 4.70 (m, 1H), 4.48 (t, J = 5.5 Hz, 1H), 4.35 (dd, J = 4.9, 3.6 Hz, 1H), 4.19-4.15 (m, 2H), 4.04-3.93 (m, 2H), 3.81(dd, J = 10.1, 3.3 Hz, 1H), 3.70-3.60 (m, 2H), 3.54-3.49 (m, 2H), 3.20 (m, 1H), 3.13 (m, 1H).
FAB-MS m/z 602 $(M-H)^-$.

Example 1

Synthesis of Adenosine=5'-(2,3,4,7-tetra-O-acetyl-6-deoxy-β-D-mannoheptopyranosyl)diphosphate (Compound 1)

Compound P (0.030 g, 0.070 mmol) and 4'-morpholine-N,N'-dicyclohexylcarboxamidinium salt of adenosine=5'-phosphomorpholidate (0.050 g, 0.070 mmol) were dissolved in pyridine (1.0 mL), and the solution was concentrated to dryness under reduced pressure. To the residue were added pyridine (1.0 mL) and molecular sieves 4A (5.0 mg), followed by stirring at room temperature for 5 days. After the molecular sieves were removed from the reaction mixture by filtration, the filtrate was concentrated under reduced pressure. The residue was purified by DEAE Sephadex A-25 column chromatography (Amersham Biosciences, eluted with 0.2-0.3 mmol/L ammonium acetate solution adjusted to pH 5.0 with acetic acid) to obtain an ammonium salt of Compound 1 (0.013 g, yield 23%).
$^1$H-NMR (500 MHz, $D_2O$)δ(ppm): 8.53 (s, 1H), 8.28 (s, 1H), 6.12 (d, J = 5.5 Hz, 1H), 5.48 (br s, 1H), 5.42 (d, J = 9.4 Hz, 1H), 5.06 (d, J = 10.1 Hz, 1H), 4.96 (dd, J = 10.1, 9.4 Hz, 1H), 4.72-4.68 (m, 2H), 4.47 (m, 1H), 4.34 (m, 1H), 4.16-4.04 (m, 3H), 3.66 (m, 1H), 2.16 (s, 3H), 2.04 (s, 3H), 2.01 (s, 3H), 1.99 (s, 3H), 1.88-1.75 (m, 2H).
FAB-MS m/z 770 $(M-H)^-$.

Example 2

Synthesis of Adenosine=5'-(6-deoxy-β-D-mannoheptopyranosyl)diphosphate (Compound 2)

The ammonium salt of Compound 1 (0.0080 g, 0.0093 mmol) was dissolved in a mixed solution of methanol, triethylammonium bicarbonate buffer (pH=8.0, 0.1 mol/L) and triethylamine (14/13/1, 7.2 mL), and the solution was stirred at -30°C for 2 weeks. After the reaction mixture was diluted with water, Dowex 50 ($H^+$ form) was added thereto and the pH was adjusted to about 4.0. The resin was removed by filtration, and the filtrate was preparatively purified by high performance liquid chromatography [column; Develosil RPAQURUS (Nomura Chemical Co., Ltd.), diameter; 4.6 mm x 25 cm, detection wavelength; 260 nm, eluting solvent; 0.5% aqueous solution of acetic acid (1.0 mL/minute), detection time; 10 to 15 minutes] to obtain Compound 2 (0.0016 g, 28%).
$^1$H-NMR(500 MHz, $D_2O$)δ(ppm): 8.52 (s, 1H), 8.27 (s, 1H), 6.15 (d, J = 6.0 Hz, 1H), 5.20 (d, J = 8.6 Hz, 1H), 4.70 (m,

1H), 4.54 (dd, J = 5.1, 3.5 Hz, 1H), 4.41 (dd, J = 2.8, 2.7 Hz, 1H), 4.23-4.20 (m, 2H), 4.09 (d, J = 3.3 Hz, 1H), 3.77 (m, 1H), 3.72 (m, 1H), 3.63 (dd, J = 9.4, 3.3 Hz, 1H), 3.44 (dd, J = 9.5, 9.4 Hz, 1H), 3.38 (ddd, J = 9.8, 9.5, 2.4 Hz, 1H), 2.16 (m, 1H), 1.66 (m, 1H).
FAB-MS m/z 602 (M-H)$^-$.

Example 3

Synthesis of Adenosine=5'-(7-O-acetyl-6-deoxy-2,3,4-tri-O-methyl-β-D-mannoheptopyranosyl)diphosphate (Compound 3)

In a manner similar to Example 1, an ammonium salt of Compound 3 (0.0085 g, yield 15%) was obtained as a mixture of anomers (α/β = 2/1) from Compound R (0.027 g, 0.078 mmol) and 4'-morpholine-N,N'-dicyclohexylcarboxamidinium salt of adenosine=5'-phosphomorpholidate (0.056 g, 0.079 mmol).
FAB-MS m/z 686 (M-H)$^-$.

Example 4

Synthesis of Adenosine=5'-(6-deoxy-2,3,4-tri-O-methyl-β-D-mannoheptopyranosyl)diphosphate (Compound 4)

In a manner similar to Example 2, an ammonium salt of Compound 4 (0.0018 g, yield 16%) was obtained as a mixture of anomers (α/β = 2/1) from Compound 3 (0.0050 g, 0.0073 mmol).
FAB-MS m/z 644 (M-H)$^-$.

Example 5

Synthesis of Adenosine=5'-(2,3,4,7-hexa-O-acetyl-6-deoxy-β-D-mannoheptopyranylmethoxy)diphosphate (Compound 26)

[Step 1]

From Compound T (0.19 g, 0.33 mmol), DMAP (48 mg, 0.40 mmol) and diphenyl chlorophosphate (0.082 mL, 0.40 mmol), a crude diphenylphosphate (0.39 g) was obtained in a manner similar to Reference Example 3.
$^1$H-NMR (300 MHz, $CDCl_3$)δ(ppm): 7.37-7.17 (m, 30H), 4.93 (d, J = 11.3 Hz, 1H), 4.91 (d, J = 10.9 Hz, 1H), 4.72 (d, J = 11.7 Hz, 1H), 4.66 (d, J = 11.7 Hz, 1H), 4.55 (d, J = 11.3 Hz, 2H), 4.45 (d, J = 12.1 Hz, 1H), 4.39 (d, J = 12.1 Hz, 1H), 4.38 (m, 1H), 4.17 (m, 1H), 3.86 (d, J = 2.2 Hz, 1H), 3.68 (t, J = 9.5 Hz, 1H), 3.59-3.51 (m, 4H), 3.35 (td, J = 9.3, 2.6 Hz, 1H), 2.15 (m, 1H), 1.74 (m, 1H).
ESI-MS m/z 801 (M+H)$^4$.

[Step 2]

From the crude diphenylphosphate obtained in Step 1 (0.39 g, 0.33 mmol) and 10% palladium carbon (18 mg), a crude tetrahydroxy form (0.20 g) was obtained in a manner similar to Step 2 of Reference Example 37.
$^1$H-NMR (300 MHz, $CDCl_3$)δ(ppm): 7.42-7.38 (m, 4H), 7.26-7.20 (m, 6H), 4.46-4.36 (m, 2H), 3.82 (br s, 1H), 3.71-3.65 (m, 3H), 3.42-3.39 (m, 2H), 3.22 (m, 1H), 2.08 (m, 1H), 1.69 (m, 1H).
ESI-MS m/z 441 (M+H)$^+$.

[Step 3]

From the crude tetrahydroxy form obtained in Step 2 (0.20 g), pyridine (1.0 mL) and acetic anhydride (1.0 mL), a tetraacetyl form (0.14 g, yield 70%) was obtained in a manner similar to Step 3 of Reference Example 37.
$^1$H-NMR (300 MHz, $CDCl_3$)δ(ppm): 7.38-7.30 (m, 4H), 7.22-7.18 (m, 6H), 5.45 (d, J = 2.2 Hz, 1H), 5.09 (dd, J = 10.0, 9.8 Hz, 1H), 4.99 (dd, J = 10.0, 3.3 Hz, 1H), 4.33-4.06 (m, 4H), 3.86 (m, 1H), 3.48 (m, 1H), 2.11 (s, 3H), 2.05 (s, 3H), 2.04 (s, 3H), 1.98 (s, 3H), 1.87-1.72 (m, 2H).
ESI-MS m/z 609 (M+H)$^+$.

[Step 4]

From the tetraacetyl form obtained in Step 3 (0.14 g, 0.23 mmol) and platinum oxide (40 mg), a dephenylated form (60 mg, yield 57%) was obtained in a manner similar to Step 4 of Reference Example 37.
$^1$H-NMR (300 MHz, $CD_3OD$)δ(ppm): 5.48 (d, J = 2.7 Hz, 1H), 5.07 (m, 1H), 4.22-4.14 (m, 2H), 4.01-3.80 (m, 3H), 3.70-. 3.58 (m, 2H), 2.13 (s, 3H), 2.04 (s, 3H), 2.03 (s, 3H), 1.93 (s, 3H), 1.88 (m, 1H), 1.75 (m, 1H).
FAB-MS m/z 455 $(M-H)^-$.

[Step 5]

In a manner similar to Reference Example 21, an ammonium salt of Compound 26 (30 mg, yield 48%) was obtained from the dephenylated form obtained in Step 4 (35 mg, 0.077 mmol), 4'-morpholine-N,N'-dicyclohexylcarboxamidinium salt of adenosine=5'-phosphomorpholidate (54 mg, 0.077 mmol) and tetrazole (54 mg, 0.077 mmol).
$^1$H-NMR (500 MHz, $D_2O$)δ(ppm): 8.59 (s, 1H), 8.32 (br s, 1H), 6.19 (d, J = 5.6 Hz, 1H), 5.42 (d, J = 1.9 Hz, 1H), 5.02-5.00 (m, 2H), 4.73 (dd, J = 5.3, 5.2 Hz, 1H), 4.65 (m, 1H), 4.54 (dd, J = 5.2, 3.8 Hz, 1H), 4.40 (m, 1H), 4.25-4.24 (m, 2H), 4.22-4.10 (m, 2H), 4.02-3.88 (m, 2H), 3.69 (m, 1H), 3.56 (m, 1H), 2.19 (s, 3H), 2.11 (s, 3H), 2.08 (s, 3H), 1.99 (s, 3H), 1.91 (m, 1H), 1.77 (m, 1H).
FAB-MS m/z 784 $(M-H)^-$.

Example 6

Synthesis of Adenosine=5'-(6-deoxy-β-D-mannoheptopyranylmethoxy)diphosphate (Compound 27)

In a manner similar to Example 2, Compound 27 (3.0 mg, yield 27%) was obtained from the ammonium salt of Compound 26 (15 mg, 0.018 mmol).
$^1$H-NMR (300 MHz, $D_2O$, 293K)δ(ppm): 8.43 (s, 1H), 8.34 (br s, 1H), 6.05 (d, J = 6.0 Hz, 1H), 4.67 (t, J = 5.5 Hz, 1H), 4.43 (dd, J = 4.9, 3.6 Hz, 1H), 4.30 (m, 1H), 4.16-4.14 (m, 2H), 3.92-3.86 (m, 2H), 3.86 (d, J = 3.3 Hz, 1H), 3.58 (dd, J = 7.6, 5.3 Hz, 2H), 3.54 (t, J = 6.9 Hz, 1H), 3.40 (dd, J = 9.6, 3.4 Hz, 1H), 3.30 (dd, J = 9.6, 9.5 Hz, 1H), 3.13 (td, J = 9.5, 2.5 Hz, 1H), 1.92 (m, 1H), 1.50 (ddt, J = 14.6, 9.5, 5.3 Hz, 1H).
FAB-MS m/z 616 $(M-H)^-$.

Example 7

Synthesis of Adenosine=5'-(6-deoxy-β-D-mannoheptopyranylmethyl)diphosphate (Compound 28)

[Step 1]

To a toluene solution (10 mL) of Compound T (1.1 g, 2.0 mmol) were added triphenylphosphine (0.79 g, 3.0 mmol), iodine (0.66 g, 2.6 mmol) and imidazole (0.41 g, 6.0 mmol) at room temperature, followed by stirring at 60˚C for 4 hours. The reaction mixture was concentrated under reduced pressure, and the residue was purified by silica gel column chromatography (hexane/ethyl acetate = 10/1) to obtain an iodine form (0.66 g, yield 45%).
$^1$H-NMR (300 MHz, $CDCl_3$)δ(ppm): 7.35-7.28 (m, 20H), 4.94 (d, J = 11.0 Hz, 1H), 4.93 (d, J = 11.7 Hz, 1H), 4.75 (d, J = 11.6 Hz, 1H), 4.67 (d, J = 11.6 Hz, 1H), 4.63 (d, J = 11.0 Hz, 1H), 4.62 (d, J = 11.7 Hz, 1H), 4.47 (d, J = 11.8 Hz, 1H), 4.41 (d, J = 11.8 Hz, 1H), 3.98 (d, J = 2.6 Hz, 1H), 3.70 (dd, J = 9.4, 9.1 Hz, 1H), 3.62 (dd, J = 9.4, 2.7 Hz, 1H), 3.56-3.52 (m, 3H), 3.34-3.17 (m, 3H), 2.34 (m, 1H), 1.93 (m, 1H).
ESI-MS m/z 679 $(M+H)^+$.

[Step 2]

The iodine form obtained in Step 1 (0.61 g, 0.90 mmol) was dissolved in triethyl phosphite (6.0 mL), followed by stirring at 130˚C for one day. The reaction mixture was concentrated under reduced pressure, and the residue was purified by silica gel column chromatography (chloroform/methanol = 20/1) to obtain a crude phosphate (0.95 g).
ESI-MS m/z 689 $(M+H)^+$.

[Step 3]

To methylene chloride (1.0 mL) of the crude phosphate obtained in Step 2 (0.050 g) was dropwise added a

methylene chloride solution (6.0 mL) of trimethylsilyl bromide (0.019 mL, 0.14 mmol), followed by stirring at room temperature for 4 days. The reaction mixture was concentrated under reduced pressure, and the residue was purified by silica gel column chromatography (chloroform/methanol/30% aqueous ammonia = 7/4/1) to obtain a phosphate form (6.5 mg, yield 51%).
$^1$H-NMR (300 MHz, $CD_3OD$)δ(ppm): 7.44-7.20 (m, 20H), 4.97 (d, J = 10.8 Hz, 1H), 4.90-4.82 (m, 3H), 4.67 (d, J = 11.7 Hz, 1H), 4.62 (d, J = 11.0 Hz, 1H), 4.47 (d, J = 12.0 Hz, 1H), 4.41 (d, J = 12.0 Hz, 1H), 4.34 (d, J = 2.4 Hz, 1H), 3.79 (m, 1H), 3.69 (dd, J = 9.2, 2.7 Hz, 1H), 3.64-3.56 (m, 3H), 2.30-2.10 (m, 2H), 1.92 (m, 1H), 1.65 (m, 1H).
ESI-MS m/z 631 $(M-H)^-$.

[Step 4]

In a manner similar to Reference Example 21, an ammonium salt of a tetrabenzyl form of Compound 28 (3.0 mg, yield 2.0%) was obtained from the phosphate form obtained in Step 3 (0.61 g, 0.96 mmol), 4'-morpholine-N,N'-dicyclohexylcarboxamidinium salt of adenosine=5'-phosphomorpholidate (0.68 g, 0.96 mmol) and tetrazole (67 mg, 0.96 mmol).
FAB-MS m/z 960 $(M-H)^-$.

[Step 5]

To a methanol/water/acetic acid mixed solution (50/50/1, 10 mL) of the ammonium salt of the tetrabenzyl form of Compound 28 obtained in Step 4 (3.0 mg, 0.0031 mmol) was added 10% palladium carbon (90 mg), followed by stirring at 60°C for 30 minutes in an atmosphere of hydrogen. The catalyst was removed from the reaction mixture by filtration. The filtrate was concentrated, and the residue was preparatively purified by high performance liquid chromatography [column; Develosil RPAQURUS (Nomura Chemical Co., Ltd.), diameter; 4.6 mm x 25 cm, detection wavelength; 260 nm, eluting solvent; 0.5% aqueous solution of acetic acid (1.0 mL/minute), detection time; 10 to 15 minutes] to obtain Compound 28 (0.3 mg, yield 16%).
$^1$H-NMR (300 MHz, $D_2O$)δ(ppm): 8.47 (s, 1H), 8.23 (s, 1H), 6.10 (d, J = 5.5 Hz, 1H), 4.87 (m, 1H), 4.46 (m, 1H), 4.44 (m, 1H), 4.37-4.30 (m, 2H), 4.26 (d, J = 3.1 Hz, 1H), 4.20 (m, 1H), 4.09-4.06 (m, 2H), 3.72-3.65 (m, 3H), 3.50 (dd, J = 9.8, 9.1 Hz, 1H), 3.31 (m, 1H), 2.01 (m, 1H), 1.88 (m, 1H).
ESI-MS m/z 600 $(M-H)^-$.

Example 8

Synthesis of Adenosine=5'-(6-deoxy-β-D-mannoheptopyranyl)diphosphate (Compound 29)

[Step 1]

From Compound N (0.98 g, 2.1 mmol), acetic acid (20 mL) and concentrated sulfuric acid (0.010 mL), a crude diacetyl form (1.2 g) was obtained in a manner similar to Step 2 of Reference Example 34.
FAB-MS m/z: 571 $(M+Na)^+$.

[Step 2]

To a methylene chloride solution (20 mL) of the crude diacetyl form obtained in Step 1 (1.2 g, 2.1 mmol) were added trimethyl phosphate (0.63 mL, 5.0 mmol) and a methylene chloride solution (5.0 mL) of trimethylsilyl trifluoromethanesulfonate (0.56 mL, 2.5 mmol) under ice-cooling, and the mixture was stirred at the same temperature for 2 hours. The reaction mixture was poured into ice water, followed by extraction with chloroform.
The organic layer was washed with a saturated aqueous solution of sodium chloride, dried over sodium sulfate, and then concentrated under reduced pressure. The residue was purified by silica gel column chromatography (hexane/ethyl acetate = 3/1) to obtain a phosphate (0.32 g, yield 25%).
$^1$H-NMR (500 MHz, $CDCl_3$)δ(ppm): 7.45-7.43 (m, 2H), 7.35-7.26 (m, 13H), 5.00 (d, J = 10.7 Hz, 1H), 4.94 (d, J = 10.8 Hz, 1H), 4.82 (d, J = 10.7 Hz, 1H), 4.76 (d, J = 11.7 Hz, 1H), 4.66 (d, J = 10.8 Hz, 1H), 4.65 (d, J = 11.7 Hz, 1H), 4.34 (dd, J = 2.8, 0.9 Hz, 1H), 4.22-4.15 (m, 2H), 3.78-3.76 (m, 2H), 3.74-3.68 (m, 6H), 3.58 (dd, J = 9.3, 2.8 Hz, 1H), 3.33 (td, J = 9.3, 2.6 Hz, 1H), 2.20 (m, 1H), 2.01 (s, 3H), 1.83 (m, 1H).
FAB-MS m/z 599 $(M+H)^+$.

[Step 3]

From the phosphate obtained in Step 2 (0.32 g, 0.54 mmol) and trimethylsilyl bromide (0.33 g, 22 mmol), a phosphate form (0.22 g, yield 72%) was obtained in a manner similar to Step 3 of Example 7.
$^1$H-NMR (500 MHz, $CD_3OD$)δ(ppm): 7.49-7.47 (m, 2H), 7.35-7.33 (m, 2H), 7.29-7.26 (m, 11H), 4.93 (d, J = 10.8 Hz, 1H), 4.89 (d, J = 11.0 Hz, 1H), 4.85 (d, J = 10.8 Hz, 1H), 4.71 (d, J = 11.6 Hz, 1H), 4.65 (d, J = 11.0 Hz, 1H), 4.59 (d, J = 11.6 Hz, 1H), 4.40 (br s, 1H), 4.26-4.15 (m, 2H), 3.64-3.58 (m, 3H), 3.30 (m, 1H), 2.12 (m, 1H), 1.97 (s, 3H), 1.74 (m, 1H).
FAB-MS m/z 571 $(M+H)^+$.

[Step 4]

In a manner similar to Reference Example 21, an ammonium salt of a protected form of Compound 29 (45 mg, yield 5.0%) was obtained from the phosphate form obtained in Step 3 (0.14 g, 0.25 mmol), 4'-morpholine-N,N'-dicyclohexylcarboxamidinium salt of adenosine=5'-phosphomorpholidate (0.17 g, 0.25 mmol) and tetrazole (18 mg, 0.25 mmol).
$^1$H-NMR (500 MHz, $D_2O$)δ(ppm): 8.55 (s, 1H), 8.18 (s, 1H), 7.51 (d, J = 6.9 Hz, 1H), 7.35-7.20 (m, 14H), 6.08 (d, J = 5.2 Hz, 1H), 4.98 (d, J = 10.8 Hz, 1H), 4.88 (d, J = 11.0 Hz, 1H), 4.85-4.78 (m, 2H), 4.71 (d, J = 11.6 Hz, 1H), 4.65-4.62 (m, 2H), 4.57 (d, J = 11.6 Hz, 1H), 4.52-4.48 (m, 2H), 4.45 (m, 1H), 4.26-4.18 (m, 2H), 4.05 (m, 1H), 3.82 (d, J = 16.3 Hz, 1H), 3.66 (dd, J = 9.3, 2.9 Hz, 1H), 3.59 (dd, J = 9.4, 9.3 Hz, 1H), 3.33 (m, 1H), 2.08 (m, 1H), 1.95 (s, 3H), 1.70 (m, 1H).
FAB-MS m/z 944 $(M-H+2Na)^+$; 922 $(M+Na)^+$; 898 $(M-H)^-$.

[Step 5]

From the ammonium salt of the protected form of Compound 29 obtained in Step 4 (24 mg, 0.027 mmol), a deacetylated form (14 mg, yield 44%) was obtained in a manner similar to Example 2.
FAB-MS m/z 887 $(M+Na)^+$; 856 $(M-H)^-$.

[Step 6]

In a manner similar to Step 5 of Example 7, Compound 29 (3.0 mg, yield 43%) was obtained from the deacetylated form obtained in Step 5 (10 mg, 0.012 mmol) and 10% palladium carbon (10 mg).
$^1$H-NMR (500 MHz, $D_2O$)δ(ppm): 8.62 (s, 1H), 8.38 (s, 1H), 6.19 (d, J = 5.6 Hz, 1H), 4.80 (dd, J = 5.6, 5.0 Hz, 1H), 4.56 (dd, J = 5.0, 3.8 Hz, 1H), 4.41 (m, 1H), 4.25-4.24 (m, 3H), 3.82 (d, J = 15.5 Hz, 1H), 3.79 (m, 1H), 3.72 (m, 1H), 3.59 (dd, J = 9.6, 3.3 Hz, 1H), 3.52 (dd, J = 9.5, 9.4 Hz, 1H), 3.33 (td, J = 9.5, 2.6 Hz, 1H), 2.05 (m, 1H), 1.67 (m, 1H).
ESI-MS m/z 588 $(M+H)^+$; 586 $(M-H)^-$.

Example 9

Synthesis of Adenosine=5'-(6-deoxy-β-D-mannoheptopyranosyl)-α,β-methylene diphosphate (Compound 30)

[Step 1]

Tribenzyl methylene diphosphate (1.1 g, 2.5 mmol) and 2',3'-diacetyladenosine (1.8 g, 5.0 mmol) were dissolved in pyridine (1.0 mL), and the solvent was distilled off under reduced pressure until the moisture was completely removed. The residue was dissolved in pyridine (2.0 mL), and a toluene solution (5.2 mL) of DIAD (10 mmol) and triphenylphosphine (2.6 g, 10 mmol) were added thereto, followed by stirring at 40°C for 2 hours. The reaction mixture was concentrated under reduced pressure, and the residue was purified by silica gel column chromatography (chloroform/methanol = 9/1) to obtain a tetraphosphate (0.36 g, yield 18%) as a diastereomixture.
ESI-MS m/z 779 $(M+H)^+$.

[Step 2]

To a DMF solution (5.0 mL) of the tetraphosphate obtained in Step 1 (0.36 g, 0.46 mmol) was added quinuclidine (48 mg, 0.43 mmol), followed by stirring at 120°C for 30 minutes. The reaction mixture was concentrated under reduced pressure, and the residue was purified by high performance liquid chromatography [column; ODS-3 (GL-Sciences), diameter; 4.6 mm x 25 cm, detection wavelength; 260 nm, eluting solvent; acetoritrile/10 mmol/L aqueous solution of ammonium acetate = 15/85 to 45/65 gradient (20 minutes, 1.0 mL/minute), detection time; 15 to 20 minutes] to obtain

a triphosphate (96 mg, yield 30%) as a diastereomixture.
ESI-MS m/z 687 $(M-H)^-$.

[Step 3]

To a THF solution (1.0 mL) of the triphosphate obtained in Step 2 (90 mg, 0.26 mmol) were added Compound O (180 mg, 0.52 mmol), triphenylphosphine (200 mg, 0.78 mmol) and a toluene solution (0.39 mL) of DIAD (0.78 mmol), followed by stirring at room temperature for one hour.
The reaction mixture was concentrated, and the residue was purified by silica gel column chromatography (chloroform/methanol = 9/1) to obtain a sugar phosphate form (82 mg, yield 61%).
ESI-MS m/z 1034 $(M+H)^+$.

[Step 4]

To an ethanol solution (1.0 mL) of the sugar phosphate form obtained in Step 3 (82 mg, 0.079 mmol) was added palladium carbon (8.0 mg), followed by stirring at room temperature for one hour in an atmosphere of hydrogen. The catalyst was removed from the reaction mixture by filtration, and the filtrate was concentrated. The residue was dissolved in methanol/water/triethylamine (3.7 mL), followed by stirring at room temperature for 5 hours. Then the solution was filtered and the filtrate was concentrated. The residue was purified by DEAE Sephadex A-25 column chromatography (Amersham Biosciences, eluted with 0.2-0.3 mmol/L ammonium acetate solution adjusted to pH 5.0 with acetic acid) to obtain Compound 30 (11 mg, yield 17%) as an ammonium salt of a mixture of anomers ($\alpha$:$\beta$ = 1:2).
$\alpha$ form
$^1$H-NMR (500 MHz, $D_2O$)$\delta$(ppm): 8.62 (s, 1H), 8.34 (s, 1H), 6.16 (d, J = 5.5 Hz, 1H), 5.45 (d, J = 7.4 Hz, 1H), 4.81 (dd, J = 5.4, 5.3 Hz, 1H), 4.56 (m, 1H), 4.39 (m, 1H), 4.19-4.18 (m, 2H), 4.01 (m, 1H), 3.89 (m, 1H), 3.88 (m, 1H), 3.74 (m, 1H), 3.52-3.42 (m, 2H), 2.27 (dd, J = 20.8, 20.5 Hz, 2H), 2.16 (m, 1H), 1.76 (m, 1H).
ESI-MS m/z 600 $(M-H)^-$.
$\beta$ form
$^1$H-NMR (500 MHz, $D_2O$)$\delta$(ppm): 8.62 (s, 1H), 8.34 (s, 1H), 6.16 (d, J = 5.5 Hz, 1H), 5.29 (d, J = 8.5 Hz, 1H), 4.81 (dd, J = 5.4, 5.3 Hz, 1H), 4.56 (m, 1H), 4.39 (m, 1H), 4.19-4.18 (m, 2H), 4.05(d, J = 3.2 Hz,1H), 3.89 (m, 1H), 3.73-3.69 (m, 2H), 3.65 (dd, J = 9.1, 3.3 Hz, 1H), 3.47-3.38 (m, 2H), 2.27 (dd, J = 20.8, 20.5 Hz, 2H), 2.09 (m, 1H), 1.65 (m, 1H).
ESI-MS m/z 600 $(M-H)^-$.

Example 10

Synthesis of Adenosine=5'-(6,7-dideoxy-$\beta$-D-mannooctopyranosyl)diphosphate (Compound 31)

[Step 1]

From Compound U (0.52 g, 1.4 mmol), DMAP (0.20 g, 1.7 mmol) and diphenyl chlorophosphate (0.37 mL, 1.7 mmol), a crude diphenylphosphate (0.39 g, quantitative) was obtained in a manner similar to Reference Example 3.
$^1$H-NMR (300 MHz, $CDCl_3$)$\delta$(ppm): 7.61-7.14 (m, 10H), 5.54 (dd, J = 6.8, 1.1 Hz, 1H), 5.48 (d, J = 3.1 Hz, 1H), 5.11 (dd, J = 9.9, 9.6 Hz, 1H), 5.00 (dd, J = 9.9, 3.1 Hz, 1H), 4.00 (t, J = 6.0 Hz, 2H), 3.51 (m, 1H), 2.12 (s, 3H), 2.06 (s, 3H), 2.01 (s, 3H), 1.99 (s, 3H), 1.77 (m, 1H), 1.61-1.58
(m, 3H).
FAB-MS m/z: 609 $(M+H)^+$.

[Step 2]

From the crude diphenylphosphate obtained in Step 1 (0.17 g, 0.28 mmol) and platinum oxide (20 mg), a dephenylated form (98 mg, yield 80%) was obtained in a manner similar to Step 2 of Example 5.
$^1$H-NMR (300 MHz, $CDCl_3$)$\delta$(ppm): 5.48 (dd, J = 3.2, 1.4 Hz, 1H), 5.40 (d, J = 8.2, 1.4 Hz, 1H), 5.14 (dd, J = 9.9, 3.2 Hz, 1H), 5.02 (dd, J = 9.9, 9.8 Hz, 1H), 4.08 (t, J = 6.3 Hz, 2H), 3.62 (m, 1H), 2.11 (s, 3H), 2.05 (s, 3H), 2.04 (s, 3H), 1.94 (s, 3H), 1.78-1.64 (m, 3H), 1.53 (m, 1H).
ESI-MS m/z: 455 $(M-H)^-$.

[Step 3]

In a manner similar to Reference Example 21, an ammonium salt of Compound 31 (1.6 mg, yield 2.0%) was

obtained from the dephenylated form obtained in Step 2 (97 mg, 0.20 mmol), 4'-morpholine-N,N'-dicyclohexylcarboxamidinium salt of adenosine=5'-phosphomorpholidate (0.14 g, 0.2 mmol) and tetrazole (14 mg, 0.20 mmol).
$^1$H-NMR (300 MHz, $CDCl_3$)δ(ppm): 8.53 (br s, 1H), 8.27 (s, 1H), 6.12 (d, J = 6.0 Hz, 1H), 5.47 (d, J = 1.8 Hz, 1H), 5.38 (d, J = 9.3 Hz, 1H), 5.04 (dd, J = 9.6, 3.2 Hz, 1H), 4.90 (dd, J = 9.7, 9.6 Hz, 1H), 4.69 (t, J = 5.5 Hz, 1H), 4.45 (t, J = 4.5 Hz, 1H), 4.35 (m, 1H), 4.19-4.16 (m, 2H), 3.96-3.93 (m, 2H), 3.37 (m, 1H), 2.15 (s, 3H), 2.03 (s, 3H), 1.99 (s, 3H), 1.94 (s, 3H), 1.73 (m, 1H), 1.59-1.51 (m, 2H), 1.40 (m, 1H).
FAB-MS m/z 784 $(M-H)^-$.

Example 11

Process for Producing Adenosine=5'-(D-glycero-β-D-mannoheptopyranosyl)diphosphate (Compound 16) Utilizing a Microorganism

The strain KY 13101 was used as a seed strain. The strain was inoculated into a test tube containing a medium having the following composition prior to sterilization (pH 7.0, 10 mL: hereinafter referred to as a seed medium). Seed culture was carried out by shaking (220 rpm) using a reciprocal shaker at 28˚C for 3 days to prepare a first seed culture.
Composition of the seed medium: glucose (10 g/L), soluble starch (10 g/L), meat extract (3 g/L), yeast extract (5 g/L), tryptone (5 g/L), $KH_2PO_4$ (1 g/L), $MgSO_4 \cdot 7H_2O$ (0.5 g/L) and $Mg_3(PO_4)_2 \cdot 8H_2O$ (0.5 g/L)
The first culture was inoculated into a 300-mL Erlenmeyer flask containing the seed medium (50 mL) at a rate of 2.5% (volume), and subjected to shaking (320 rpm) using a rotary shaker at 28˚C for 2 days to prepare a second culture.
The second culture was inoculated into a 300-mL Erlenmeyer flask containing the seed medium (50 mL) at a rate of 5% (volume), and subjected to shaking (320 rpm) using a rotary shaker at 28˚C for 2 days to prepare a third culture.
The obtained third culture was inoculated into a 5-L jar fermentor containing a fermentation medium having the following composition (2.5 L) at a rate of 5% (volume), and subjected to spinner culture under aeration (rotation 400 rpm, aeration 1.25 L/minute) at 28˚C. Culturing was carried out for 5 days without particularly controlling the pH of the medium.
Composition of the fermentation medium: glucose (10 g/L), soluble starch (10 g/L), pupa powder (10 g/L), yeast extract (2 g/L), nitrohumic acid (0.25 g/L), 3-morpholinopropanesulfonic acid (2 g/L: MOPS), $ZnSO_4 \cdot 7H_2O$ (0.01 g/L), $MnSO_4 \cdot 4\text{-}5H_2O$ (0.01 g/L), $FeSO_4 \cdot 7H_2O$) (0.01 g/L), $CoCl_2 \cdot 6H_2O$ (0.01 g/L) and $MgSO_4 \cdot 7H_2O$ (0.5 g/L) (medium prior to sterilization: pH 7.0, adjusted with NaOH and $H_2SO_4$)
The culture resulting from the culturing in the 5-L jar fermentor (12.3 L) was centrifuged to obtain a precipitate containing the mycelium, and ethanol (6.15 L) was added thereto, followed by further centrifugation to obtain the supernatant as an extract. After this extract was filtered, a 0.2% aqueous solution of acetic acid was added and ethanol was distilled off using a rotary evaporator. The residue was passed through Diaion HP-20 (Mitsubishi Chemical Corporation). The unadsorbed fraction and the fraction obtained by washing with water containing 0.2% acetic acid were purified by DEAE Sephadex A-25 column chromatography (Pharmacia) (eluted stepwise with 0.1 mol/L, 0.2 mol/L, 0.3 mol/L, 0.4 mol/L and 0.5 mol/L aqueous solutions of ammonium acetate adjusted to pH 5 with acetic acid). The active fractions eluted with 0.2 mol/L and 0.3 mol/L solutions were freeze-dried, and the obtained powder was purified by column chromatography [column; YMC-GEL ODS-AQ 120-S50 (YMC Co., Ltd.), eluting solvent; 0.2% aqueous solution of acetic acid (5 mL/minute)]. Then, the powder obtained by freeze-drying the active fraction was purified by Toyopearl HW40 superfine (Tosoh Corporation) (eluted with 0.2% aqueous solution of acetic acid), and the active fraction was freeze-dried. The obtained powder was purified by reversed-phase high performance liquid chromatography [column; Develosil RPAQUEOUS column (Nomura Chemical Co., Ltd.), diameter; 4.6 x 250 mm, detection wavelength; UV absorption at 260 nm, eluting solvent; 0.5% aqueous solution of acetic acid (1 mL/minute), column temperature; 30˚C], and the active fraction (retention time 13.1 minutes) was freeze-dried. The obtained powder was further purified by reversed-phase high performance liquid chromatography [column; Develosil RPAQUEOUS column (Nomura Chemical Co., Ltd.), diameter; 4.6 x 250 mm, detection wavelength; UV absorption at 260 nm, eluting solvent; a mixed solvent of 10 mmol/L aqueous solution of ammonium acetate and acetonitrile (99/1, 1 mL/minute), column temperature; 30˚C] to obtain Compound 16 (0.07 mg).

Industrial Applicability

The present invention provides compounds having TLR9 agonist activity which are useful as TLR9 agonists, immunostimulants, anti-allergic agents, anti-tumor agents and anti-infection agents, and the like.

SEQUENCE LISTING FREE TEXT

SEQ ID NO: 1 - Description of Artificial Sequence: Forward Primer

SEQ ID NO: 2 - Description of Artificial Sequence: Reverse Primer

SEQ ID NO: 3 - Description of Artificial Sequence: Forward Primer

SEQ ID NO: 4 - Description of Artificial Sequence: Reverse Primer

SEQ ID NO: 5 - Description of Artificial Sequence: Forward Primer

SEQ ID NO: 6 - Description of Artificial Sequence: Reverse Primer

SEQ ID NO: 7 - Description of Artificial Sequence: Forward Primer

SEQ ID NO: 8 - Description of Artificial Sequence: Reverse Primer

## Claims

**1.** A Toll-like receptor 9 (TLR9) agonist comprising, as an active ingredient, a compound represented by formula (I):

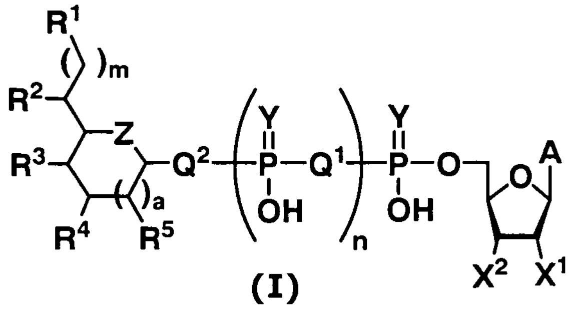

(I)

(wherein a represents 0 or 1;
n represents an integer of 0 to 2;
m represents an integer of 0 to 5;
$X^1$ and $X^2$ each independently represents a hydrogen atom or hydroxy;
Y represents an oxygen atom or a sulfur atom (when n is 1 or 2, two or three Ys may be the same or different);
$-Q^1-$ represents -O-, $-CH_2-$ or $-CF_2-$;
$-Q^2-$ represents a single bond, -O-, $-CH_2-O-$ or $-CH_2-$;
-Z- represents -O- or $-CH_2-$;
$R^1$, $R^3$ and $R^4$ each independently represents hydroxy, substituted or unsubstituted lower alkanoyloxy, substituted or unsubstituted lower alkoxy, substituted or unsubstituted lower alkenyloxy, substituted or unsubstituted aralkyloxy, or substituted or unsubstituted heterocyclic alkyloxy;
$R^2$ and $R^5$ each independently represents a hydrogen atom, hydroxy, substituted or unsubstituted lower alkanoyloxy, substituted or unsubstituted lower alkoxy, substituted or unsubstituted lower alkenyloxy, substituted or unsubstituted aralkyloxy, or substituted or unsubstituted heterocyclic alkyloxy; and
A represents a group selected from the following substituents (A)) or a pharmaceutically acceptable salt thereof

Substituents (A)

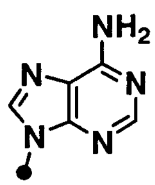

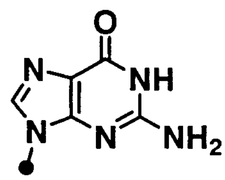

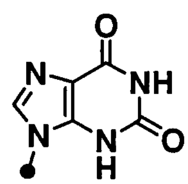

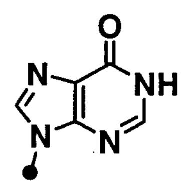

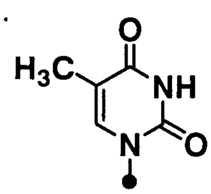

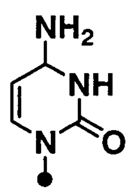

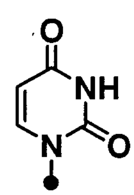

**2.** The Toll-like receptor 9 (TLR9) agonist according to Claim 1, wherein m is 1.

**3.** The Toll-like receptor 9 (TLR9) agonist according to Claim 1 or 2, wherein a is 1.

**4.** The Toll-like receptor 9 (TLR9) agonist according to Claim 1 or 2, wherein a is 0.

**5.** The Toll-like receptor 9 (TLR9) agonist according to any of Claims 1 to 4, wherein $R^2$ is a hydrogen atom.

**6.** The Toll-like receptor 9 (TLR9) agonist according to any of Claims 1 to 4, wherein $R^1$ and $R^2$ each independently represents hydroxy or lower alkanoyloxy.

**7.** The Toll-like receptor 9 (TLR9) agonist according to any of Claims 1 to 6, wherein $X^1$ and $X^2$ are hydroxy.

**8.** A compound represented by formula (IA):

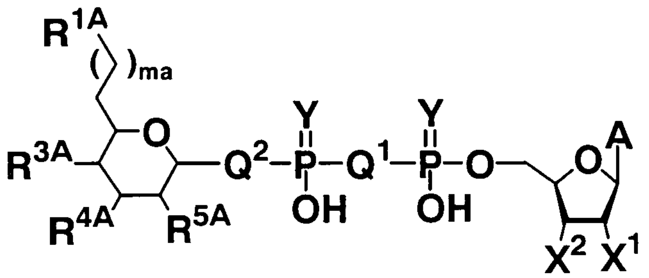 (IA)

(wherein ma represents an integer of 1 to 5;
$X^1$, $X^2$, Y, $Q^1$, $Q^2$ and A each have the same meanings as defined above;
$R^{1A}$, $R^{3A}$, $R^{4A}$ and $R^{5A}$ each independently represents hydroxy, substituted or unsubstituted lower alkanoyloxy, or substituted or unsubstituted lower alkoxy) or a pharmaceutically acceptable salt thereof.

**9.** The compound or the pharmaceutically acceptable salt thereof according to Claim 8, wherein ma is 1.

**10.** The compound or the pharmaceutically acceptable salt thereof according to Claim 8 or 9, wherein $X^1$ and $X^2$ are hydroxy.

**11.** The compound or the pharmaceutically acceptable salt thereof according to any of Claims 8 to 10, wherein $R^{1A}$ is hydroxy or lower alkanoyloxy.

**12.** The compound or the pharmaceutically acceptable salt thereof according to any of Claims 8 to 11, wherein A is

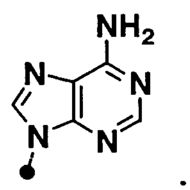

**13.** A Toll-like receptor 9 (TLR9) agonist comprising, as an active ingredient, the compound or the pharmaceutically acceptable salt thereof described in any of Claims 8 to 12.

**14.** An anti-allergic agent comprising, as an active ingredient, the compound or the pharmaceutically acceptable salt thereof described in any of Claims 8 to 12.

**15.** An anti-tumor agent comprising, as an active ingredient, the compound or the pharmaceutically acceptable salt thereof described in any of Claims 8 to 12.

**16.** An anti-infection agent comprising, as an active ingredient, the compound or the pharmaceutically acceptable salt thereof described in any of Claims 8 to 12.

**17.** An immunostimulant comprising, as an active ingredient, the compound or the pharmaceutically acceptable salt thereof described in any of Claims 8 to 12.

**18.** A treating and/or preventing agent for a Toll-like receptor 9 (TLR9)-associated disease comprising, as an active ingredient, the compound or the pharmaceutically acceptable salt thereof described in any of Claims 8 to 12.

**19.** An anti-allergic agent comprising, as an active ingredient, the compound or the pharmaceutically acceptable salt thereof described in any of Claims 1 to 7.

**20.** An anti-tumor agent comprising, as an active ingredient, the compound or the pharmaceutically acceptable salt thereof described in any of Claims 1 to 7.

**21.** An anti-infection agent comprising, as an active ingredient, the compound or the pharmaceutically acceptable salt thereof described in any of Claims 1 to 7.

**22.** An immunostimulant comprising, as an active ingredient, the compound or the pharmaceutically acceptable salt thereof described in any of Claims 1 to 7.

**23.** A treating and/or preventing agent for a Toll-like receptor 9 (TLR9)-associated disease comprising, as an active ingredient, the compound or the pharmaceutically acceptable salt thereof described in any of Claims 1 to 7.

**24.** Massilia sp. KY 13101 strain (accession no.: NITE BP-348).

**25.** A process for producing the compound described in any of Claims 1 to 12 utilizing the microorganism described in Claim 24.

**26.** A method for activating Toll-like receptor 9 (TLR9) which comprises a step of administering an effective amount of the compound or the pharmaceutically acceptable salt thereof described in any of Claims 1 to 7.

**27.** A method for treating allergy which comprises a step of administering an effective amount of the compound or the pharmaceutically acceptable salt thereof described in any of Claims 1 to 7.

**28.** A method for treating tumor which comprises a step of administering an effective amount of the compound or the pharmaceutically acceptable salt thereof described in any of Claims 1 to 7.

**29.** A method for treating an infective disease which comprises a step of administering an effective amount of the compound or the pharmaceutically acceptable salt thereof described in any of Claims 1 to 7.

**30.** An immunostimulatory method which comprises a step of administering an effective amount of the compound or the pharmaceutically acceptable salt thereof described in any of Claims 1 to 7.

**31.** A method for treating and/or preventing a Toll-like receptor 9 (TLR9)-associated disease which comprises a step of administering an effective amount of the compound or the pharmaceutically acceptable salt thereof described in any of Claims 1 to 7.

**32.** A method for activating Toll-like receptor 9 (TLR9) which comprises a step of administering an effective amount of the compound or the pharmaceutically acceptable salt thereof described in any of Claims 8 to 12.

**33.** A method for treating allergy which comprises a step of administering an effective amount of the compound or the pharmaceutically acceptable salt thereof described in any of Claims 8 to 12.

**34.** A method for treating tumor which comprises a step of administering an effective amount of the compound or the pharmaceutically acceptable salt thereof described in any of Claims 8 to 12.

**35.** A method for treating an infective disease which comprises a step of administering an effective amount of the compound or the pharmaceutically acceptable salt thereof described in any of Claims 8 to 12.

**36.** An immunostimulatory method which comprises a step of administering an effective amount of the compound or

the pharmaceutically acceptable salt thereof described in any of Claims 8 to 12.

**37.** A method for treating and/or preventing a Toll-like receptor 9 (TLR9)-associated disease which comprises a step of administering an effective amount of the compound or the pharmaceutically acceptable salt thereof described in any of Claims 8 to 12.

**38.** Use of the compound or the pharmaceutically acceptable salt thereof described in any of Claims 1 to 7 for the manufacture of a Toll-like receptor 9 (TLR9) agonist.

**39.** Use of the compound or the pharmaceutically acceptable salt thereof described in any of Claims 1 to 7 for the manufacture of an anti-allergic agent.

**40.** Use of the compound or the pharmaceutically acceptable salt thereof described in any of Claims 1 to 7 for the manufacture of an anti-tumor agent.

**41.** Use of the compound or the pharmaceutically acceptable salt thereof described in any of Claims 1 to 7 for the manufacture of an anti-infection agent.

**42.** Use of the compound or the pharmaceutically acceptable salt thereof described in any of Claims 1 to 7 for the manufacture of an immunostimulant.

**43.** Use of the compound or the pharmaceutically acceptable salt thereof described in any of Claims 1 to 7 for the manufacture of a treating and/or preventing agent for a Toll-like receptor 9 (TLR9)-associated disease.

**44.** Use of the compound or the pharmaceutically acceptable salt thereof described in any of Claims 8 to 12 for the manufacture of a Toll-like receptor 9 (TLR9) agonist.

**45.** Use of the compound or the pharmaceutically acceptable salt thereof described in any of Claims 8 to 12 for the manufacture of an anti-allergic agent.

**46.** Use of the compound or the pharmaceutically acceptable salt thereof described in any of Claims 8 to 12 for the manufacture of an anti-tumor agent.

**47.** Use of the compound or the pharmaceutically acceptable salt thereof described in any of Claims 8 to 12 for the manufacture of an anti-infection agent.

**48.** Use of the compound or the pharmaceutically acceptable salt thereof described in any of Claims 8 to 12 for the manufacture of an immunostimulant.

**49.** Use of the compound or the pharmaceutically acceptable salt thereof described in any of Claims 8 to 12 for the manufacture of a treating and/or preventing agent for a Toll-like receptor 9 (TLR9)-associated disease.

[Fig. 1]

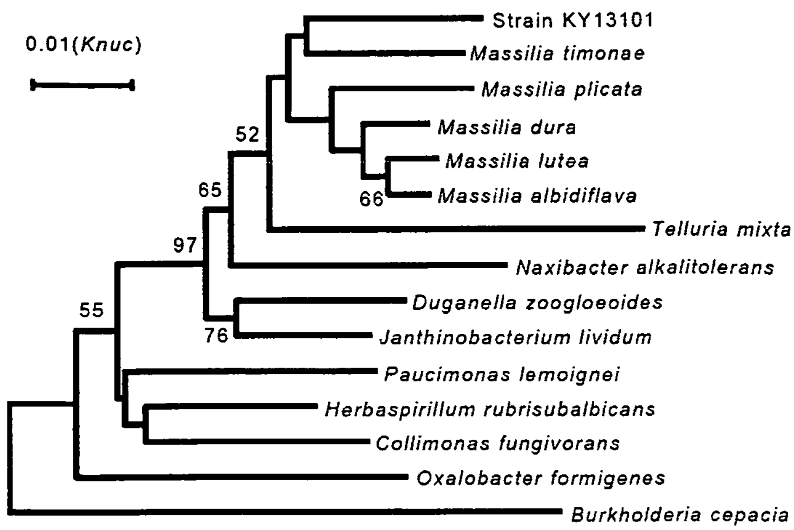


[Fig. 2]

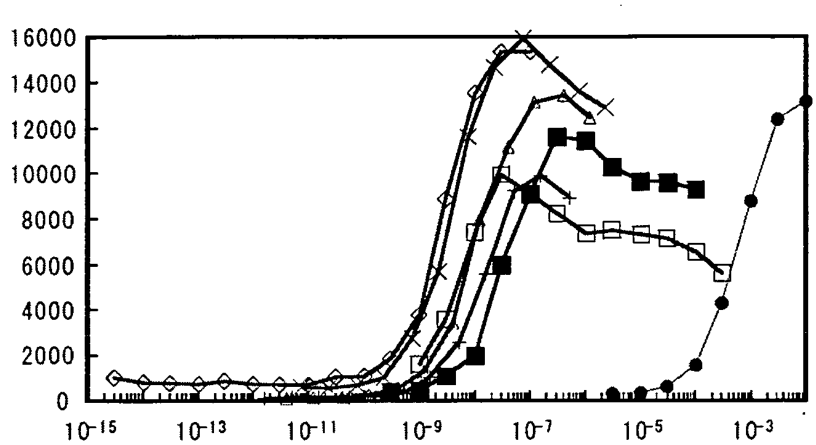


[Fig. 3]

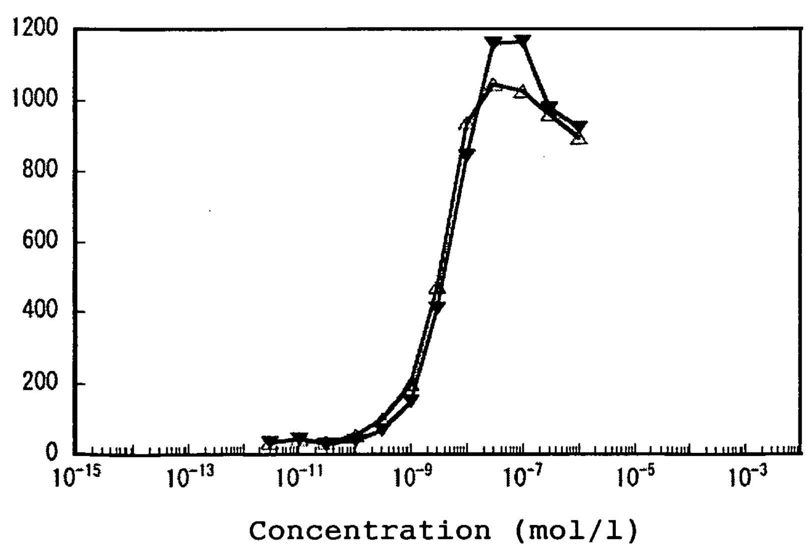

**INTERNATIONAL SEARCH REPORT**

International application No.
PCT/JP2007/058148

A. CLASSIFICATION OF SUBJECT MATTER
*C07H19/20*(2006.01)i, *A61K31/7064*(2006.01)i, *A61P31/00*(2006.01)i, *A61P35/00*(2006.01)i, *A61P37/04*(2006.01)i, *A61P37/08*(2006.01)i, *A61P43/00*(2006.01)i, *C07H19/10*(2006.01)i, *C12N1/20*(2006.01)i, *C12P19/30*(2006.01)i

According to International Patent Classification (IPC) or to both national classification and IPC

B. FIELDS SEARCHED

Minimum documentation searched (classification system followed by classification symbols)
C07H19/10, C07H19/11, C07H19/20-C07H19/213, A61K31/7064-A61K31/708, C12N1/20, C12P19/30-C12P19/36

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
REGISTRY/CAplus/BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
|---|---|---|
| X | Kovensky, J. et. al, D-Galactofuranosylphosphonates. First Synthesis of UDP-C-D-galactofuranose, J. Org. Chem, 1999, Vol.64, No.17, pp.6202-6205. | 1-7,38,41,43 |
| A | WO 98/12343 A1 (Kyowa Hakko Kogyo Co., Ltd.), 26 March, 1998 (26.03.98), & US 2002/0064836 A1 & EP 870841 A1 & CN 1207135 A | 1-25,38-49 |
| A | Zamyatina, A. et. al, Efficient chemical synthesis of both anomers of ADP L-glycero- and D-glycero-manno-heptopyranose, Carbohyde. Res, 2003, Vol.338, pp.2571-2589. | 1-25,38-49 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 June, 2007 (29.06.07) | 10 July, 2007 (10.07.07) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2007/058148

**Box No. II Observations where certain claims were found unsearchable (Continuation of item 2 of first sheet)**

This international search report has not been established in respect of certain claims under Article 17(2)(a) for the following reasons:

1. [×] Claims Nos.: 26-37
because they relate to subject matter not required to be searched by this Authority, namely:
Claims 26 to 37 are relevant to methods for treatment of the human body or animal body by therapy under the provisions of the PCT Rule 39.1(iv).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

(See extra sheet)

1. [×] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
- [ ] The additional search fees were accompanied by the applicant's protest and, where applicable, payment of a protest fee..
- [ ] The additional search fees were accompanied by the applicant's protest but the applicable protest fee was not paid within the time limit specified in the invitation.
- [×] No protest accompanied the payment of additional search fees.

Form PCT/ISA/210 (continuation of first sheet (2)) (April 2005)

**INTERNATIONAL SEARCH REPORT**

International application No.
PCT/JP2007/058148

Continuation of Box No.III of continuation of first sheet(2)

(1) Claims 1-7, 19-23, 38-43 relating to medicines containing as the active ingredient compounds represented by the general formula (I); (2) Claims 8-12 relating to compounds represented by the general formula (IA); (3) Claims 13-18, 44-49 relating to medicines containing as the active ingredient compounds represented by the general formula (IA); (4) Claim 24 relating to Massilia sp. strain KY13101; and (5) Claim 25 relating to a process for the production of compounds represented by the general formula (I) or (IA) by using Massilia sp. strain KY13101.

The compounds represented by the general formula (IA) are included among the compounds represented by the general formula (I) and the compounds of group (2) as represented by the general formula (IA) have medical activities which are special technical features of the compounds of group (1) as represented by the general formula (I). Thus, groups (1) to (3) satisfy the requirement of unity of invention (see PCT Administrative Instructions Annex B, Examples 4 and 16). However, it cannot be said that the compounds represented by the general formula (I) themselves are special technical features (see Zamyatina, A. et. al, Efficient chemical synthesis of both anomers of ADP L-glycero- and D-glycero-manno-heptopyranose, Carbohyde. Res, 2003, 338, pp.2571-2589). Therefore, it cannot be said that there is a technical relationship among groups (1) to (3) of inventions and the inventions of claims 24 and 25 involving one or more of the same or corresponding special technical features.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0468520 A
- WO 9602555 A
- WO 9818810 A
- WO 9837919 A
- WO 9840100 A
- WO 9951259 A
- WO 9956755 A
- JP 16947900 B
- WO 940100 A


### Non-patent literature cited in the description


- *Nature,* 2000, vol. 408, 740
- *Nature Reviews Drug Discovery,* 2002, vol. 1, 797
- *Nature Reviews Immunology,* 2004, vol. 4, 1
- *Clinical Cancer Research,* 2003, vol. 9, 2693
- *Clinical Cancer Research,* 2003, vol. 9, 3105
- *The Journal of Immunology,* 1998, vol. 160, 3627
- *Proceedings of the National Academy of Sciences of the United States of America,* 1999, vol. 96, 6970
- *Journal of Allergy & Clinical Immunology,* 2002, vol. 110, 867
- *American Journal of Respiratory and Critical Care Medicine,* 2004, vol. 170, 1153
- *American Society of Clinical Oncology: ASCO, Annual Meeting,* 2005
- *Journal of Allergy & Clinical Immunology,* 2004, vol. 113, 235
- *Blood,* 1997, vol. 89, 2635
- *Journal of Experimental Medicine,* 1996, vol. 184, 981
- *Leukemia Lymphoma,* 1995, vol. 19, 267
- *Journal of Experimental Medicine,* 1993, vol. 178, 1057
- *Journal of Bacteriology,* 2002, vol. 184, 363
- *Carbohydrate Research,* 2003, vol. 338, 2571
- **T.W. GREENE.** Protective Groups in Organic Synthesis. John Wiley & Sons Inc, 1999
- **P. KOSMA et al.** *Carbohydrate Research,* 2003, vol. 338 (23), 2571-2589
- **C. C. TANG et al.** *Synthetic Communication,* 1998, vol. 28 (15), 2769
- **R. A. FIELD et al.** *Tetrahedron Letters,* 2001, vol. 42, 2231-2234
- **M. J. HADD et al.** *Journal of Organic Chemistry,* 1997, vol. 62, 6961-6967
- **A. MURAI et al.** *Tetrahedron,* 1998, vol. 54, 21-44
- **A. VASELLA et al.** *Helvetica Chimica Acta,* 1986, vol. 69, 25-34
- **R. K. SOOD et al.** *Canadian Journal of Chemistry,* 1994, vol. 72, 247-251
- **S. BERTIL et al.** *Carbohydrate Research,* 1978, vol. 67, 263-236
- **P. MANITTO et al.** *Tetrahedron Letters,* 1987, vol. 28 (42), 5047-5048
- **AKEPILOTTI et al.** *Acta Chemica Scandinavica,* 1972, vol. 26, 4143-4146
- **KISHI et al.** *Journal of American Chemical Society,* 1996, vol. 118 (34), 7946-7968
- **J. H. VAN BOOM et al.** *Organic Letters,* 2000, vol. 2 (9), 1275-1277
- **C. MIOSKOWSKI et al.** *Journal of Organic Chemistry,* 2002, vol. 67 (1), 146-153
- **C. MIOSKOWSKI et al.** *Journal of Organic Chemistry,* 1995, vol. 60, 2946-2947
- *Cytotechnology,* 1988, vol. 1, 151
- *Bio Techniques,* 1997, vol. 22, 130-131134-138

SEQUENCE LISTING

<110> KYOWA HAKKO KOGYO CO., LTD

<120> TOLL-LIKE RECEPTOR 9 AGONISTS

<130> 1863

<150> JP2006-111950
<151> 2006-04-14

<160> 8

<170> PatentIn Ver 3.1

<210> 1
<211> 21
<212> DNA
<213> Artificial

<220>
<223> Forward Primer

<400> 1
accagggctg cttttaactc t 21

<210> 2
<211> 21
<212> DNA
<213> Artificial

<220>
<223> Reverse Primer

<400> 2
tcatgagtcc ttccacgata c 21

<210> 3

<211> 22
<212> DNA
<213> Artificial

<220>
<223> Forward Primer

<400> 3
cctatgatta tgtccggtta tg 22

<210> 4
<211> 22
<212> DNA
<213> Artificial

<220>
<223> Reverse Primer

<400> 4
ggatctctct gatttttctt gc 22

<210> 5
<211> 24
<212> DNA
<213> Artificial

<220>
<223> Forward Primer

<400> 5
ataaagcaat ttagggccac ttac 24

<210> 6
<211> 22
<212> DNA
<213> Artificial

<220>
<223> Reverse Primer

<400> 6
tgacaatttc atgtccttag cc 22

<210> 7
<211> 24
<212> DNA
<213> Artificial

<220>
<223> Forward Primer

<400> 7
tagaaggata atttgctcaa cctc 24

<210> 8
<211> 22
<212> DNA
<213> Artificial

<220>
<223> Reverse Primer

<400> 8
gctaccttca gatcttttca gc 22